# EUROPEAN PATENT SPECIFICATION

(11) **EP 1 802 307 B1**
(45) Date of publication and mention of the grant of the patent: **27.02.2008**
(21) Application number: 05802453.0
(22) Date of filing: 13.10.2005
(51) Int. Cl.: A61K 31/496, A61K 31/4025, C07D 401/04, C07D 403/04, C07D 401/14, C07D 403/10, C07D 207/16, A61P 25/00

(54) **PYRROLIDINE DERIVATIVES AS HISTAMINE RECEPTORS LIGANDS**
PYRROLIDINDERIVATE ALS HISTAMINREZEPTOR-LIGANDEN
LIGANDS DE RECEPTEURS HISTAMINIQUES A BASE DE DERIVES PYRROLIDINIQUES

(30) Priority: 15.10.2004 GB 0423005; 26.04.2005 GB 0508441
(43) Date of publication of application: 04.07.2007
(73) Proprietor: GLAXO GROUP LIMITED, Greenford, Middlesex UB6 ONN (GB)
(72) Inventor: BRUTON, Gordon, Harlow Essex CM19 5AW (GB); COOPER, Ian Ronald, Harlow Essex CM19 5AW (GB); ORLEK, Barry, Sidney, Harlow Essex CM19 5AW (GB)
(74) Representative: Goddard, Carolyn Janice
(86) International application number: PCT/EP2005/011371
(87) International publication number: WO 2006/040192

(56) References cited:
- WO-A-02/07250
- WO-A-20/04037257
- WO-A-20/04101546
- WO-A1-98/06705
- WO-A1-03/062234

## Description

The present invention relates to novel pyrrolidine derivatives having pharmacological activity, processes for their preparation, to compositions containing them and to their use in the treatment of neurological and psychiatric disorders.

WO2004/101546 (Glaxo Group Ltd; published 25 November 2004) describes a series of piperazine derivatives and their use in the treatment of neurological disorders. JP 10130241 (Wakunaga Seiyaku KK) describes a series of pyridine carboxylic acid derivatives which are claimed to be useful as platelet coagulation inhibitors for treating peripheral circulation disorders, as metastasis inhibitors of malignant tumour or as antiinflammatory agents. WO 97/06802 (Zeneca Ltd.) discloses a series of heterocyclic derivatives as oxido-squalene cyclase inhibitors. WO2003062234 (Yamanouchi Pharmaceutical Co. Ltd.) describes quinoxaline derivatives which are stated to be poly(ADP-ribose) polymerase inhibitors, and the use of these compounds in the treatment of arthritis. WO 02/072570 (Schering Corporation and Pharmacopeia, Inc.) discloses a series of compounds that are useful in the treatment of allergies and CNS disturbances. WO 2004/000831 (Schering Corporation) describes a series of indole derivatives which are stated to be H3 antagonists. The use of these compounds in the treatment of CNS disturbances is also described.

The histamine H3 receptor is predominantly expressed in the mammalian central nervous system (CNS), with minimal expression in peripheral tissues except on some sympathetic nerves (Leurs et al., (1998), Trends Pharmacol. Sci. 19, 177-183). Activation of H3 receptors by selective agonists or histamine results in the inhibition of neurotransmitter release from a variety of different nerve populations, including histaminergic and cholinergic neurons (Schlicker et al., (1994), Fundam. Clin. Pharmacol. 8, 128-137). Additionally, *in vitro* and *in vivo* studies have shown that H3 antagonists can facilitate neurotransmitter release in brain areas such as the cerebral cortex and hippocampus, relevant to cognition (Onodera et al., (1998), In: The Histamine H3 receptor, ed Leurs and Timmerman, pp255-267, Elsevier Science B.V.). Moreover, a number of reports in the literature have demonstrated the cognitive enhancing properties of H3 antagonists (e.g. thioperamide, clobenpropit, ciproxifan and GT-2331) in rodent models including the five choice task, object recognition, elevated plus maze, acquisition of novel task and passive avoidance (Giovanni et al., (1999), Behav. Brain Res. 104, 147-155). These data suggest that novel H3 antagonists and/or inverse agonists such as the current series could be useful for the treatment of cognitive impairments in neurological diseases such as Alzheimer's disease and related neurodegenerative disorders.

The present invention provides, in a first aspect, a compound of formula (I) or a pharmaceutically acceptable salt thereof: 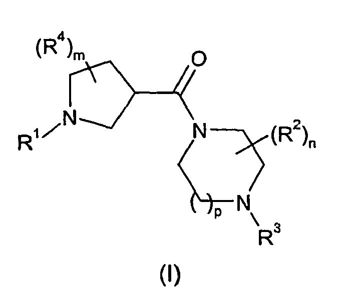 wherein:
R¹ represents aryl, heteroaryl, -aryl-X-C₃₋₇ cycloalkyl, -heteroaryl-X-C₃₋₇ cycloalkyl, -aryl-X-aryl, -aryl-X-heteroaryl, -aryl-X-heterocyclyl, -heteroaryl-X-heteroaryl, -heteroaryl-X-aryl or -heteroaryl-X-heterocyclyl;
wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, haloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, -CO-haloC₁₋₆ alkyl, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -NR¹⁵R¹⁶, -CONR¹⁵R¹⁶, -NR¹⁵COR¹⁶, -C(R¹⁵)=NOR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring;
X represents a bond, O, CO, SO₂, OCH₂ or CH₂O;
each R² and R⁴ independently represents C₁₋₄ alkyl;
R³ represents C₂₋₆ alkyl, C₃₋₆ alkenyl, C₃₋₆ alkynyl, C₃₋₆ cycloalkyl, C₅₋₆ cycloalkenyl or -C₁₋₄alkyl-C₃₋₆ cycloalkyl;
wherein said C₃₋₆ cycloalkyl groups of R³ may be optionally substituted by one or more (e.g. 1, 2 or 3) substituents which may be the same or different, and which are selected from the group consisting of halogen, C₁₋₄ alkyl or trifluoromethyl groups;
m and n independently represent 0, 1 or 2;
p represents 1 or 2;
or a solvate thereof.

In one aspect, the invention provides compounds of formula (I) wherein:
R¹ represents aryl, heteroaryl, -aryl-X-C₃₋₇ cycloalkyl, -heteroaryl-X-C₃₋₇ cycloalkyl, -aryl-X-aryl, -aryl-X-heteroaryl, -aryl-X-heterocyclyl, -heteroaryl-X-heteroaryl, -heteroaryl-X-aryl or -heteroaryl-X-heterocyclyl;
wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, haloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, -COC₁₋₆ alkyl-halogen, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -NR¹⁵R¹⁶, -CONR¹⁵R¹⁶, -NR¹⁵COR¹⁶, -C(R¹⁵)=NOR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring;
X represents a bond, O, CO, SO₂, OCH₂ or CH₂O;
each R² and R⁴ independently represents C₁₋₄ alkyl;
R³ represents C₂₋₆ alkyl, C₃₋₆ alkenyl, C₃₋₆ alkynyl, C₃₋₆ cycloalkyl, C₅₋₆ cycloalkenyl or -C₁₋₄alkyl-C₃-₆ cycloalkyl;
wherein said C₃₋₆ cycloalkyl groups of R³ may be optionally substituted by one or more (e.g. 1, 2 or 3) substituents which may be the same or different, and which are selected from the group consisting of halogen, C₁₋₄ alkyl or trifluoromethyl groups;
m and n independently represent 0, 1 or 2;
p represents 1 or 2;
or a solvate thereof.

In another aspect, the aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, halOC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, -COC₁₋₆ alkyl-halogen, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, phenyl, phenylsulfonyl, phenylsulfonyloxy, phenoxy, phenylsulfonamido, phenylcarboxamido, phenylcarbonyl, or a group -NR¹⁵R¹⁶, -CONR¹⁵R¹⁶, -NR¹⁵COR¹⁶,-C(R¹⁵)=NOR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring.

In a further aspect, in which R¹ is pyridin-4-yl or pyrimidin-4-yl optionally substituted by one or two substituents selected from the group consisting of amino, halogen, cyano, C₁₋₆ alkyl or C₁₋₆ alkoxy group, R³ is other than -C₁₋₄alkyl-C₅₋₆ cycloalkyl.

In another aspect in which R¹ represents heteroaryl, the heteroaryl group is other than a 1,4-dihydro-quinolin-7-yl group or a 1,4-dihydro-1,8-naphthyridin-7-yl group.

In yet another aspect in which R¹ represents heteroaryl, heteroaryl-X-aryl, heteroaryl-X-heteroaryl, heteroaryl-X-heterocyclyl or heteroaryl-X-C₃₋₇ cycloalkyl, and wherein R¹ is further substituted, the heteroaryl group directly attached to the nitrogen atom of the pyrrolidine ring is other than a quinoxalinyl group that is substituted by a carboxyamide group at the 5-position and further optionally substituted by a C₁₋₆ alkyl, C₁₋₆ alkoxy, halo C₁₋₆ alkyl, or halogen atom.

A specific set of compounds of formula (I) which may be mentioned are those wherein R¹ represents heteroaryl, -heteroaryl-X-C₃₋₇ cycloalkyl, -heteroaryl-X-heteroaryl, -heteroaryl-X-aryl or-heteroaryl-X-heterocyclyl optionally substituted by one or more (e.g. 1, 2 or 3) substituents which may be the same or different, and which are selected from the group consisting of halogen, hydroxy, cyano, nitro, haloC₁₋₆ alkyl, haloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, - COC₁₋₆ alkyl-halogen, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -NR¹⁵R¹⁶, -CONR¹⁵R¹⁶, -NR¹⁵COR¹⁶, -C(R¹⁵)=NOR¹⁶, -NR¹⁵SO₂R¹⁶ or -SO₂NR¹⁵R¹⁶, wherein R¹⁵ and R¹⁶ independently represent hydrogen or C₁₋₆ alkyl or together form a heterocyclic ring. A more particular set of compounds of formula (I) which may be mentioned are those wherein R¹ represents heteroaryl, -heteroaryl-X-C₃₋₇ cycloalkyl, -heteroaryl-X-heteroaryl, -heteroaryl-X-aryl or -heteroaryl-X-heterocyclyl optionally substituted by one or more (e.g. 1, 2 or 3) substituents which may be the same or different, and which are selected from the group consisting of halogen, hydroxy, cyano, nitro, haloC₁₋₆ alkyl, haloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, -COC₁₋₆ alkyl-halogen, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, phenyl, phenylsulfonyl, phenylsulfonyloxy, phenyloxy, phenylsulfonamido, phenylcarboxamido, phenylcarbonyl, or a group -NR¹⁵R¹⁶, -CONR¹⁵R¹⁶, -NR¹⁵COR¹⁶, -C(R¹⁵)=NOR¹⁶, -NR¹⁵SO₂R¹⁶ or-SO₂NR¹⁵R¹⁶, wherein R¹⁵ and R¹⁶ independently represent hydrogen or C₁₋₆ alkyl or together form a heterocyclic ring.

The term 'C₁₋₆ alkyl' as used herein as a group or a part of the group refers to a linear or branched saturated hydrocarbon group containing from 1 to 6 carbon atoms. Examples of such groups include methyl, ethyl, n-propyl, isopropyl, n-butyl, isobutyl, sec-butyl, tert butyl, n-pentyl, isopentyl, neopentyl or hexyl and the like.

The term 'C₂₋₆ alkenyl' as used herein refers to a linear or branched hydrocarbon group containing one or more carbon-carbon double bonds and having from 2 to 6 carbon atoms. Examples of such groups include ethenyl, propenyl, butenyl, pentenyl or hexenyl and the like.

The term 'C₁₋₆ alkoxy' as used herein refers to an -O-C₁₋₆ alkyl group wherein C₁₋₆ alkyl is as defined herein. Examples of such groups include methoxy, ethoxy, propoxy, butoxy, pentoxy or hexoxy and the like.

The term 'C₃₋₈ cycloalkyl' as used herein refers to a saturated monocyclic hydrocarbon ring of 3 to 8 carbon atoms. Examples of such groups include cyclopropyl, cyclobutyl, cyclopentyl, cyclohexyl, cycloheptyl or cyclooctyl and the like.

The term 'halogen' as used herein refers to a fluorine, chlorine, bromine or iodine atom.

The term 'haloC₁₋₆ alkyl' as used herein refers to a C₁₋₆ alkyl group as defined herein wherein at least one hydrogen atom is replaced with halogen. Examples of such groups include fluoroethyl, trifluoromethyl or trifluoroethyl and the like.

The term 'halo C₁₋₆ alkoxy' as used herein refers to a C₁₋₆ alkoxy group as herein defined wherein at least one hydrogen atom is replaced with halogen. Examples of such groups include difluoromethoxy or trifluoromethoxy and the like.

The term 'aryl' as used herein refers to a C₆₋₁₂ monocyclic or bicyclic hydrocarbon ring wherein at least one ring is aromatic. More particularly, the term 'aryl' refers to a C₆₋₁₀ monocyclic or bicyclic hydrocarbon ring wherein at least one ring is aromatic. Examples of such groups include phenyl, naphthyl or tetrahydronaphthalenyl and the like.

The term 'aryloxy' as used herein refers to an -O-aryl group wherein aryl is as defined herein. Examples of such groups include phenoxy and the like.

The term 'heteroaryl' as used herein refers to a 5-6 membered monocyclic aromatic or a fused 8-10 membered bicyclic aromatic ring, which monocyclic or bicyclic aromatic ring contains 1 to 4 heteroatoms selected from oxygen, nitrogen and sulphur. Examples of such monocyclic aromatic rings include thienyl, furyl, furazanyl, pyrrolyl, triazolyl, tetrazolyl, imidazolyl, oxazolyl, thiazolyl, oxadiazolyl, isothiazolyl, isoxazolyl, thiadiazolyl, pyranyl, pyrazolyl, pyrimidyl, pyridazinyl, pyrazinyl, pyridyl, triazinyl, tetrazinyl and the like. Examples of such fused aromatic rings include quinolinyl, isoquinolinyl, quinazolinyl, quinoxalinyl, pteridinyl, cinnolinyl, phthalazinyl, naphthyridinyl, indolyl, isoindolyl, azaindolyl, indolizinyl, indazolyl, purinyl, pyrrolopyridinyl, furopyridinyl, benzofuranyl, isobenzofuranyl, benzothienyl, benzoimidazolyl, benzoxazolyl, benzoisoxazolyl, benzothiazolyl, benzoisothiazolyl, benzoxadiazolyl, benzothiadiazolyl and the like. In one aspect, the term 'heteroaryl' refers to a 5-6 membered monocyclic aromatic ring.

The term 'heterocyclyl' refers to a 4-7 membered monocyclic ring or a fused or bridged 8-12 membered bicyclic ring which may be saturated or partially unsaturated, which monocyclic or bicyclic ring contains 1 to 4 heteroatoms selected from oxygen, nitrogen or sulphur. Examples of such monocyclic rings include pyrrolidinyl, azetidinyl, pyrazolidinyl, oxazolidinyl, piperidinyl, piperazinyl, morpholinyl, thiomorpholinyl, thiazolidinyl, hydantoinyl, valerolactamyl, oxiranyl, oxetanyl, dioxolanyl, dioxanyl, oxathiolanyl, oxathianyl, dithianyl, dihydrofuranyl, tetrahydrofuranyl, dihydropyranyl, tetrahydropyranyl, tetrahydropyridinyl, tetrahydropyrimidinyl, tetrahydrothiophenyl, tetrahydrothiopyranyl, diazepanyl, azepanyl and the like. Examples of such bicyclic rings include indolinyl, isoindolinyl, benzopyranyl, quinuclidinyl, 2,3,4,5-tetrahydro-1*H*-3-benzazepine, tetrahydroisoquinolinyl and the like.

In one embodiment, R¹ represents:
- aryl;
- aryl-X-heteroaryl;
- aryl-X-heterocyclyl;
- heteroaryl; or
- heteroaryl-X-heteroaryl.

In one aspect, the aryl, heteroaryl or heterocyclic groups of R¹ may optionally be substituted by one or more (e.g. 1, 2 or 3) substituents which may be the same or different, and which are selected from the group consisting of halogen, cyano, oxo, halOC₁₋₆ alkyl, C₁₋₆ alkyl, C₁₋₆ alkoxy, -COC₁₋₆ alkyl, -COC₁₋₆ alkyl-halogen, C₁₋₆ alkoxycarbonyl, phenyl, phenoyl, or a group -CONR¹⁵R¹⁶, -NR¹⁵COR¹⁶ or - C(R¹⁵)=NOR¹⁶, wherein R¹⁵ and R¹⁶ independently represent hydrogen or C₁₋₆ alkyl or together form a heterocyclic ring.

More particularly, the aryl, heteroaryl or heterocyclic groups of R¹ may optionally be substituted by one or more (e.g. 1, 2 or 3) substituents which may be the same or different, and which are selected from the group consisting of halogen, cyano, haloC₁₋₆ alkyl, C₁₋₆ alkyl, C₁₋₆ alkoxy, -COC₁₋₆ alkyl, -COC₁₋₆ alkyl-halogen, C₁₋₆ alkoxycarbonyl, phenyl, phenoyl, or a group -CONR¹⁵R¹⁶, -NR¹⁵COR¹⁶ or -C(R¹⁵)=NOR¹⁶, wherein R¹⁵ and R¹⁶ independently represent hydrogen or C₁₋₆ alkyl or together form a heterocyclic ring.

Even more particularly, the aryl, heteroaryl or heterocyclic groups of R¹ may optionally be substituted by one or more (e.g. 1, 2 or 3) substituents which may be the same or different, and which are selected from the group consisting of halogen, cyano, oxo, haloC₁₋₆ alkyl, C₁₋₆ alkyl or -COC₁₋₆ alkyl. Most particularly, the aryl, heteroaryl or heterocyclic groups of R¹ may optionally be substituted by one or more (e.g. 1, 2 or 3) substituents which may be the same or different, and which are selected from the group consisting of halogen, cyano, haloC₁₋₆ alkyl, C₁₋₆ alkyl or -COC₁₋₆ alkyl.

In one embodiment in which R¹ represents -aryl-X-heteroaryl; -aryl-X-heterocyclyl or - heteroaryl-X-heteroaryl and the aryl or heteroaryl linked to the nitrogen atom of the pyrrolidine group is a 6 membered ring, the bond to X is in the para position relative to the attachment to the linkage to the nitrogen atom of the pyrrolidine group.

In one embodiment in which R¹ represents -aryl or -heteroaryl, wherein the aryl and heteroaryl groups are six membered rings that are substituted by one substitutent, the substituent is in the para position relative to the attachment to X.

In a more particular embodiment, R¹ represents:
- aryl (e.g. phenyl) optionally substituted by one or more (e.g. 1, 2 or 3) -COC₁₋₆ alkyl (e.g. -COMe or -COEt) or cyano groups;
- aryl-X-heteroaryl (e.g. -phenyl-oxadiazolyl (particularly-phenyl-1,2,4-oxadiazolyl or -phenyl-1,3,4-oxadiazolyl), -phenyl-1,3-oxazolyl, -phenyl-isoxazolyl, -phenyl-pyrrolyl (particularly -phenyl-pyrrol-1-yl) or -phenyl-imidazolyl (particularly -phenyl-imidazol-1-yl)) optionally substituted on the aryl group by a halogen (e.g. fluorine), and/or optionally substituted on the heteroaryl by a C₁₋₆ alkyl (e.g. methyl or ethyl) group;
- aryl-X-heterocyclyl (e.g. -phenyl-pyrrolidinyl) optionally substituted by one or more oxo groups (e.g. -phenyl-pyrrolidin-2-one);
- heteroaryl (e.g. pyridinyl, pyrimidinyl or quinolinyl) optionally substituted by one or more (e.g. 1, 2 or 3) C₁₋₆ alkyl (e.g. methyl) or haloC₁₋₆ alkyl (e.g. CF₃) groups; or
- heteroaryl-X-heteroaryl (e.g. -pyridinyl-oxadiazolyl (particularly -pyridin-3-yl-1,2,4-oxadiazolyl) optionally substituted by one or more (e.g. 1, 2 or 3) C₁₋₆ alkyl (e.g. methyl) groups.

In an even more particular embodiment, R¹ represents
- aryl (e.g. phenyl) optionally substituted by one or more (e.g. 1, 2 or 3) -COC₁₋₆ alkyl (e.g. -COMe) or cyano groups;
- aryl-X-heteroaryl (e.g. -phenyl-oxadiazolyl (particularly -phenyl-1,2,4-oxadiazolyl),-phenyl-oxazolyl (particularly-phenyl-1,3-oxazolyl) or -phenyl-imidazolyl (particularly -phenyl-1H-imidazolyl)) optionally substituted by a halogen (e.g. fluorine) or C₁₋₆ alkyl (e.g. methyl or ethyl) group; or
- heteroaryl (e.g. 3-pyridinyl) optionally substituted by one or more (e.g. 1, 2 or 3) haloC₁₋₆ alkyl (e.g. CF₃) groups.

Most particularly, R¹ represents
- phenyl, optionally substituted by a -COC₁₋₆ alkyl (e.g. -COMe) group;
- phenyl-1,3-oxazol-4-yl, optionally substituted on the oxazolyl group by a C₁₋₆ alkyl (e.g. methyl) group;
- phenyl-1,2,4-oxadiazol-5-yl, optionally substituted on the oxadiazolyl group by a C₁₋₆ alkyl (e.g. methyl) group; or
- phenyl-1H-imidazol-1-yl, optionally substituted on the imidazolyl group by a C₁₋₆ alkyl (e.g. methyl) group.

In a further embodiment, X represents CO or a bond. In a more particular embodiment, X represents a bond.

In yet another embodiment, m represents 0.

in one embodiment, n represents 0, 1 or 2. In a more particular embodiment, n represents 0 or 1, especially 0.

In embodiments in which R² is present, R² may represent methyl.

In one embodiment, p represents 1 or 2. In a more particular embodiment, p represents 1.

In another embodiment, R³ represents C₂₋₆ alkyl (e.g. isopropyl or isopentyl), C₃₋₆ cycloalkyl (e.g. cyclobutyl) or -C₁₋₄alkyl-C₃₋₆ cycloalkyl (e.g. -CH₂-cyclopropyl).

In a more particular embodiment, R³ represents isopropyl, cyclobutyl or -CH₂-cyclopropyl. Most particularly, R³ represents isopropyl or cyclobutyl.

Compounds of formula (I) may exist as stereoisomers. The 3 position of the pyrrolidine ring is a chiral centre and may exist in R and S forms. In addition, where the pyrrolidine and piperazine rings are substituted by R² and R⁴ (i.e. when m and n do not represent 0), the substituted carbon atoms are also chiral centres.

In one embodiment, the stereochemistry of the carbon atom in the pyrrolidine group that is attached to the carbonyl group has the S configuration.

In embodiments in which R² represents methyl, said R² group may be attached to the carbon atom adjacent to the N-R³ group. When R² represents methyl, the stereochemistry of R² may have the S configuration. In one embodiment where R² represents methyl and is attached to the carbon atom adjacent to the N-R³ group, the stereochemistry of R² has the S configuration.

In one aspect of the invention, the invention provides a compound of formula (I) wherein:
R¹ represents aryl, aryl-X-heteroaryl, heteroaryl or heteroaryl-X-heteroaryl;
X represents a bond;
m represents 0;
n represents 0 or 1;
p represents 1 or 2;
R² represents methyl and is attached to the carbon atom adjacent to the N-R³ group;
R³ represents C₂₋₆ alkyl, C₃₋₆ cycloalkyl or -C₁₋₄alkyl-C₃₋₆ cycloalkyl;
wherein said aryl or heteroaryl groups of R¹ may be optionally substituted by one or more (e.g. 1,2 or 3) substitutents which may be the same or different and which are selected from the group consisting of halogen, cyano, oxo, C₁₋₆ alkyl, haloC₁₋₆ alkyl or - COC₁₋₆ alkyl groups;
or a pharmaceutically acceptable salt or solvate thereof.

In a more particular aspect, R¹ may be optionally substituted by one or more (e.g. 1,2 or 3) substitutents which may be the same or different and which are selected from the group consisting of halogen, cyano, C₁₋₆ alkyl, haloC₁₋₆ alkyl or -COC₁₋₆ alkyl groups.

In a further aspect, in which R¹ is pyridin-4-yl or pyrimidin-4-yl optionally substituted by one or two substituents selected from the group consisting of halogen, cyano or C₁₋₆ alkyl, R³ does not represent -C₁₋₄alkyl-C₅₋₆ cycloalkyl.

Compounds according to the invention include examples E1-E60 as shown below, or a pharmaceutically acceptable salt thereof.

In a more particular aspect, compounds according to the invention include:
1-(1-Methylethyl)-4-({(3*S*)-1-[4-(3-methyl-1,2,4-oxadiazol-5-yl)phenyl]-3-pyrrolidinyl}carbonyl)piperazine (E3);
1-[4-((3*S*)-3-{[(3*S*)-3-Methyl-4-(1-methylethyl)-1-piperazinyl]carbonyl}-1-pyrrolidinyl)phenyl]ethanone (E5);
(2*S*)-2-Methyl-1-(1-methylethyl)-4-({(3*S*)-1-[4-(3-methyl-1,2,4-oxadiazol-5-yl)phenyl]-3-pyrrolidinyl}carbonyl)piperazine (E6);
1-Cyclobutyl-4-({(3*S*)-1-[4-(3-methyl-1,2,4-oxadiazol-5-yl)phenyl]-3-pyrrolidinyl}carbonyl)piperazine (E13);
1-Cyclobutyl-4-({(3*S*)-1-[6-(trifluoromethyl)-3-pyridinyl]-3-pyrrolidinyl}carbonyl) hexahydro -1*H*-1,4-diazepine hydrochloride (E17B);
1-({(3*S*)-1-[3-Fluoro-4-(3-methyl-1,2,4-oxadiazol-5-yl)phenyl]-3-pyrrolidinyl}carbonyl)-4-(1-methylethyl)piperazine (E24);
1-[4-((3*S*)-3-{[4-(1-Methylethyl)hexahydro-1*H*-1,4-diazepin-1-yl]carbonyl}-1-pyrrolidinyl)phenyl]ethanone (E27);
1-[1-Methylethyl)-4-({(3*S*)-1-[4-(5-methyl-1,3,4-oxadiazol-2-yl)phenyl]-3-pyrrolidinyl}carbonyl)piperazine (E30);
1-(1-Methylethyl)-4-({(3*S*)-1-[4-(2-methyl-1,3-oxazol-4-yl)phenyl]-3-pyrrolidinyl}carbonyl)piperazine (E32);
4-((3*S*)-3-{[4-(1-Methylethyl)hexahydro-1*H*-1,4-diazepin-1-yl]carbonyl}-1-pyrrolidinyl)benzonitrile hydrochloride (E46);1-Cyclobutyl-4-({(3S)-1-[3-fluoro-4-(3-methyl-1,2,4-oxadiazol-5-yl)phenyl]-3-pyrrolidinyl}carbonyl)piperazine hydrochloride (E52); or
1-(1-Methylethyl)-4-({(3*S*)-1-[4-(4-methyl-1H-imidazol-1-yl)phenyl]-3-pyrrolidinyl}carbonyl)piperazine dihydrochloride (E55).

In a most particular aspect, compounds according to the invention include:
1-[4-((3*S*)-3-{[(3*S*)-3-Methyl-4-(1-methylethyl)-1-piperazinyl]carbonyl}-1-pyrrolidinyl)phenyl]ethanone (E5);
1-(1-Methylethyl)-4-({(3*S*)-1-[4-(2-methyl-1,3-oxazol-4-yl)phenyl]-3-pyrrolidinyl)carbonyl)piperazine (E32);
1-Cyclobutyl-4-({(3*S*)-1-[3-fluoro-4-(3-methyl-1,2,4-oxadiazol-5-yl)phenyl]-3-pyrrolidinyl}carbonyl)piperazine hydrochloride (E52); or
1-(1-Methylethyl)-4-({(3*S*)-1-[4-(4-methy)-1H-imidazol-1-yl)phenyl]-3-pyrrolidinyl}carbonyl)piperazine dihydrochloride (E55).

Because of their potential use in medicine, the salts of the compounds of formula (I) are preferably pharmaceutically acceptable.

A pharmaceutically acceptable acid addition salt can be formed by reaction of a compound of formula (I) with a suitable inorganic or organic acid (such as hydrobromic, hydrochloric, sulfuric, nitric, phosphoric, succinic, maleic, formic, acetic, propionic, fumaric, citric, tartaric, lactic, benzoic, salicylic, glutamaic, aspartic, p-toluenesulfonic, benzenesulfonic, methanesulfonic, ethanesulfonic, naphthalenesulfonic such as 2-naphthalenesulfonic, or hexanoic acid), optionally in a suitable solvent such as an organic solvent, to give the salt which is usually isolated for example by crystallisation and filtration. A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salt.

Free base compounds may be converted into the corresponding hydrochloride salts by treatment in methanol with a solution of hydrogen chloride in diethyl ether followed by evaporation of solvents.

The invention includes within its scope all possible stoichiometric and non-stoichiometric forms of the salts of the compounds of formula (I) including hydrates and solvates.

Compounds of formula (I) are capable of existing in stereoisomeric forms. It will be understood that the invention encompasses all geometric and optical isomers of these compounds and the mixtures thereof including racemates. The different stereoisomeric forms may be separated one from the other by methods known in the art (e.g. separation by chiral HPLC), or any given isomer may be obtained by stereospecific or asymmetric synthesis. The invention also extends to any tautomeric forms and mixtures thereof.

In one aspect, the stereochemistry at the 3 position of the pyrrolidine ring of the compound of formula (I) is in the S configuration. Compounds with this stereochemistry are referred to as compounds of formula (la).

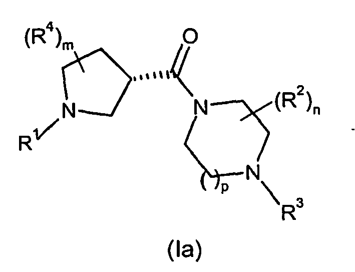

The present invention also provides a process for the preparation of a compound of formula (I) or a pharmaceutically acceptable salt or solvate thereof, which process comprises:
(a) reacting a compound of formula (II) 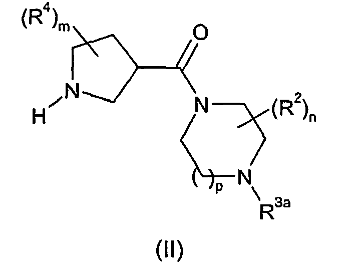 or an optionally activated or protected derivative thereof, wherein R², R⁴, m, n and p are as defined above and R^{3a} is as defined for R³ above or a group convertible to R³, with a compound of formula R¹-L¹, wherein R¹ is as defined above and L¹ represents a suitable leaving group, such as a halogen atom (e.g. fluorine, chlorine, bromine or iodine) or triflate group, followed by a deprotection reaction as necessary; or
(b) reacting a compound of formula (III) 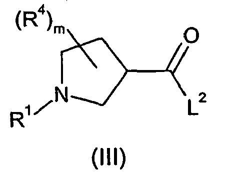 wherein R¹, R⁴ and m are as defined above and L² represents OH or a suitable leaving group, such as a halogen atom (e.g. chlorine), with a compound of formula (IV) 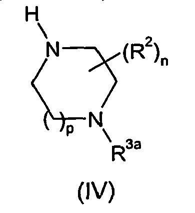 wherein R², n and p are as defined above R^{3a} is as defined for R³ above or a group convertible to R³; or
(c) deprotecting a compound of formula (I) or converting groups which are protected; or
(d) interconversion from one compound of formula (I) to another.

Process (a) typically comprises the use of a suitable base, such as potassium carbonate in a suitable solvent such as dimethylsulfoxide or N,N-dimethylformamide at elevated temperature. Alternatively process (a) may be carried out with a suitable catalyst in the presence of a suitable base such as sodium t-butoxide or potassium phosphate in a solvent such as o-xylene, dioxane, toluene or dimethoxyethane under an inert atmosphere, optionally at an elevated temperature. Suitable catalysts include tris(dibenzylideneacetone)dipalladium(0) and 2-dicyclohexylphosphino-2'-(N,N-dimethylamino)biphenyl, bis(dibenzylideneacetone)palladium and 2-dicyclohexylphosphino-2'-(N,N-dimethylamino)biphenyl, acetato(2'-di-t-butylphosphin-1,1'-biphenyl-2-yl)palladium II ortris(dibenzylideneacetone)dipalladium(0)and 2-(dicyclohexylphosphino)biphenyl.

An R^{3a} group convertible to R³ may for example be a protecting group such as tert-butoxycarbonyl which may be removed under acidic conditions, e.g. trifluoroacetic acid or HCl or a benzyloxycarbonyl or benzyl group which may be removed by hydrogenolysis, to give a compound where R^{3a} represents hydrogen. Subsequent conversion to a compound where R^{3a} represents R³ may be carried out by reductive amination with a compound of formula R3'=O (where R^{3'} may be convertible to a group R³) in the presence of sodium triacetoxyborohydride or alkylation with a compound of formula R³-L³ where L³ is a leaving group such as bromine or iodine.

Process (b) typically comprises activation of the compound of formula (III) wherein L² represents OH with a coupling reagent such as 1-(3-dimethylaminopropyl)-3-ethylcarbodiimide hydrochloride (EDC) in the presence of 1-hydroxybenzotriazole (HOBT) or 1-hydroxy-7-azabenzotriazole (HOAT) in a suitable solvent such as dichloromethane or dimethylformamide and optionally in the presence of a suitable base, followed by reaction with the compound of formula (IV) or a salt of this compound.

When L² represents a halogen (e.g. chlorine) atom, process (b) takes place in the presence of a suitable base such as triethylamine or a solid supported base such as diethylaminomethylpolystyrene in a suitable solvent such as dichloromethane. Compounds of formula (III) in which L² represents a halogen atom may be prepared from compounds of formula (III) wherein L² represents OH by treatment with a suitable halogenating agent (e.g. thionyl chloride or oxalyl chloride).

In process (c), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or tert-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (d) may be performed using conventional interconversion procedures such as epimerisation, oxidation, reduction, alkylation, nucleophilic or electrophilic aromatic substitution, ester hydrolysis or amide bond formation. Examples of transition metal mediated coupling reactions useful as interconversion procedures include the following: Palladium catalysed coupling reactions between organic electrophiles, such as aryl halides, and organometallic reagents, for example boronic acids (Suzuki cross-coupling reactions); Palladium catalysed amination and amidation reactions between organic electrophiles, such as aryl halides, and nucleophiles, such as amines and amides; Copper catalysed amidation reactions between organic electrophiles (such as aryl halides) and nucleophiles such as amides; and Copper mediated coupling reactions between phenols and boronic acids.

Compounds of formula (II) may be prepared in accordance with the following procedure: 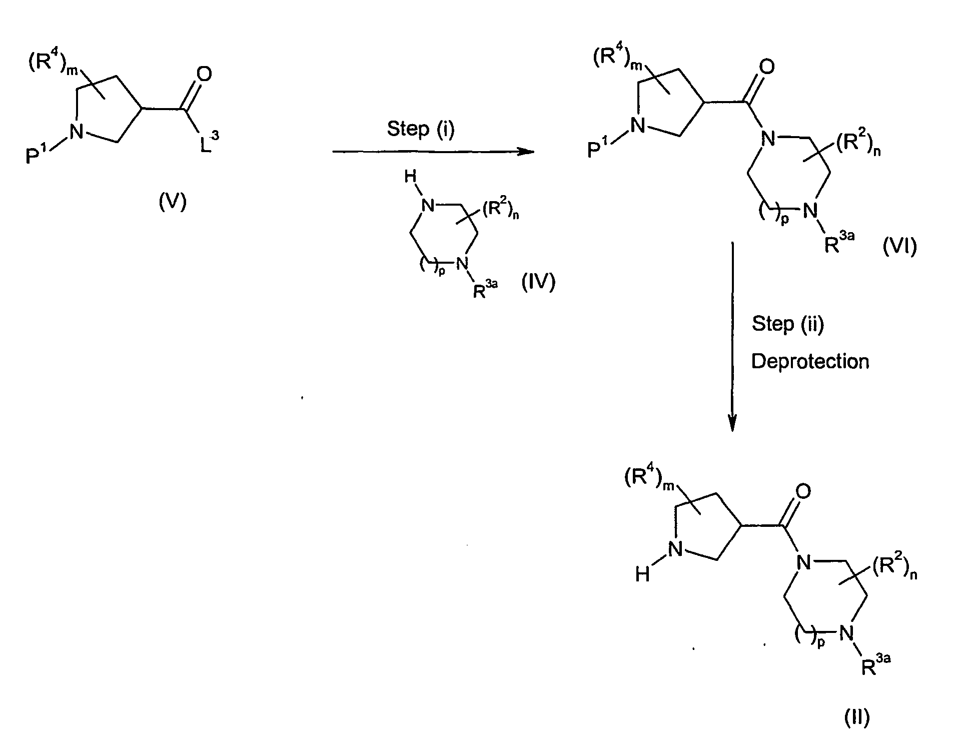 wherein R², R⁴, m, n and p are as defined above, R^{3a} is as defined for R³ above or a group convertible to R³, L³ represents OH or a suitable leaving group such as a halogen atom (e.g. chlorine), and P¹ represents a suitable protecting group such as t-butoxycarbonyl.

When L³ represents OH, step (i) typically comprises the use of suitable coupling conditions e.g. 1-(3-dimethylaminopropyl)-3-ethylcarbodiimide hydrochloride (EDC) in the presence of 1-hydroxybenzotriazole (HOBT) or 1-hydroxy-7-azabenzotriazole (HOAT), optionally in the presence of a suitable base and in a suitable solvent such as dichloromethane or dimethylformamide.

When L³ represents a suitable leaving group such as a halogen atom (e.g. chlorine), step (i) typically comprises the use of a suitable base such as triethylamine or a solid supported base such as diethylaminomethylpolystyrene in a suitable solvent such as dichloromethane.

Step (ii) typically comprises a suitable deprotection reaction using standard conditions such as those described above for process (c). Where P¹ is a tert butoxycarbonyl group this may involve a suitable acid such as HCl or trifluoroacetic acid

Compounds of formula (III) wherein L² represents OH, may be prepared in accordance with the following procedure: 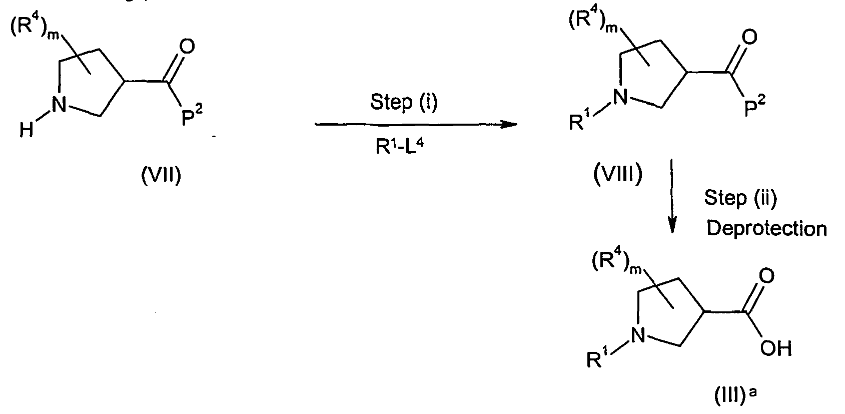 wherein R¹, R⁴ and m are as defined above, L⁴ represents a suitable leaving group such as a halogen atom or triflate group and P² represents a suitable protecting group such as methoxy, ethoxy, t-butoxy or benzyloxy.

Step (i) is typically carried out in a suitable solvent such as N,N-dimethylformamide or dimethylsulfoxide in the presence of a base such as potassium carbonate at elevated temperature. Alternatively step (i) may be carried out with a suitable catalyst in the presence of a suitable base such as sodium t-butoxide or potassium phosphate in a solvent such as o-xylene, dioxane, toluene or dimethoxyethane under an inert atmosphere optionally at an elevated temperature. Suitable catalysts include tris(dibenzylideneacetone)dipalladium(0) and 2-dicyclohexylphosphino-2'-(N,N-dimethylamino)biphenyl, bis(dibenzylideneacetone)palladium and 2-dicyclohexylphosphino-2'-(N,N-dimethylamino)biphenyl, acetato(2'-di-t-butylphosphino-1,1'-biphenyl-2-yl)palladium II, or tris(dibenzylideneacetone)dipalladium(0) and 2-(dicyclohexylphosphino)biphenyl.

Step (ii) typically comprises a suitable deprotection reaction using standard conditions such as those described above for process (c). Where P² is an alkoxy group such as ethoxy this may involve suitable acid or base catalysed hydrolysis e.g. using aqueous hydrochloric acid or a base such as sodium hydroxide or lithium hydroxide.

Compounds of formula (III) wherein L² represents a suitable leaving group, such as a halogen atom (e.g. chlorine) may be prepared by treating a compound of formula (III)^{a} with thionyl chloride or oxalyl chloride.

Stereoisomers of optionally substituted pyrrolidine-3-carboxylic acid in which the stereochemistry at the 3 position of the pyrrolidine ring is in either the R or S configurations may be prepared in accordance with the procedure set forth below wherein R⁴ and m are as described above, P³ is a protecting group, such as benzyloxycarbonyl or *tert*-butoxycarbonyl, OX represents a leaving group such as a mesylate, tosylate or triflate group, cNu represents a carbon nucleophile that can be converted to a carboxylic acid, and L⁵ and L⁶ represent suitable leaving groups such as a halogen atom (e.g. a chlorine atom). This scheme shows the production of the S enantiomer, however, it will be appreciated that the R enantiomer may be produced by an analogous process. 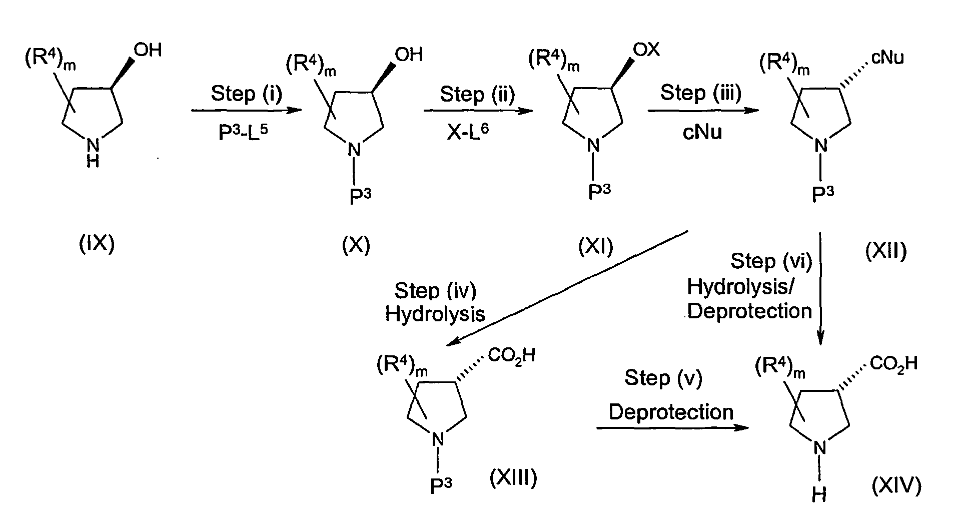
Step (i) is typically carried out in a suitable solvent such as dichloromethane in the presence of a suitable base such as triethylamine at a suitable temperature such as 0°C to room temperature.
Step (ii) is typically carried out in a suitable solvent such as dichloromethane in the presence of a suitable base such as triethylamine at a suitable temperature such as 0°C to room temperature.
Step (iii) is typically carried out by reaction with a carbon nucleophile that can be converted to a carboxylic acid, such as a cyanide salt (eg. KCN), in a suitable solvent such as DMSO at a suitable temperature such as 90°C.
Step (iv) refers to the situation where P³ is not hydrolysed in acidic conditions. This step typically comprises the use of an acid such as concentrated hydrochloric acid at a suitable temperature such as reflux.
Step (v) typically comprises a suitable deprotection reaction using standard conditions such as those described above for process (c).
Step (vi) refers to the situation where P³ can be hydrolysed, for example, where P³ is a acid labile urethane protecting group such as benzyloxycarbonyl or *tert*-butoxycarbonyl. This step typically comprises the use of an acid such as concentrated hydrochloric acid at a suitable temperature such as reflux. The acid hydrolyses both the urethane protecting group and the cyano/nitrile group.

Compounds of formula (IX) wherein m represents 0 are commercially available (e.g. from Lancaster). Compounds of formula (IX) wherein m represents 1 or 2 may be prepared by following procedures described or analogous to those described in the literature. For example, (3R,5R)-5-methyl-3-pyrrolidinol could be prepared following procedures described in WO2005/060665 (Scheme 10, 10-5).

Compounds of formula (XIV) may be used to prepare stereoisomers of the compounds of formula (V) and compounds of formula (VII) wherein the stereochemistry at the 3 position of the pyrrolidine ring is in the S or R configuration. This enables stereoisomers of the compounds of formula (I) to be prepared, wherein the stereochemistry at the 3 position of the pyrrolidine ring is in either the R or S configurations.

Stereoisomers of the compounds of formula (XIV) in which the stereochemistry at the 3 position of the pyrrolidine ring is in the S or R configurations may be used to prepare stereoisomers of the compound of formula (V) in which the stereochemistry at the 3 position of the pyrrolidine ring is in either the R or S configurations, by reaction with a compound of formula P¹-L⁷, wherein P¹ is as described above and L⁷ is a suitable leaving group such as a halogen atom. This reaction typically takes place in a suitable solvent such as dichloromethane in the presence of a suitable base such as triethylamine at a suitable temperature such as 0°C to room temperature. Where P¹ is tert-butoxycarbonyl, the reaction is typically carried out using di-tertbutyldicarbonate in a suitable solvent such as aqueous acetone at a suitable temperature, such as 0°C.

Stereoisomers of the compounds of formula (XIV) in which the sterochemistry at the 3 position of the pyrrolidine ring is in the S or R configurations may be used to prepare stereoisomers of the compound of formula (VII) in which the stereochemistry at the 3 position of the pyrrolidine ring is in either the R or S configurations, by reaction with a compound of formula P²-H, wherein P² is as described above. This reaction typically takes place in acidic conditions at a suitable temperature, such as room temperature.

The process described above is one of a number of possible methods for producing stereoisomers of the compounds of formula (I) in which the stereochemistry at the 3 position of the pyrrolidine ring is in either the R or S configurations. The methods envisaged all share a step of reacting a pyrrolidine derivative with a carbon nucleophile that can be converted to a carboxylic acid as set forth below: 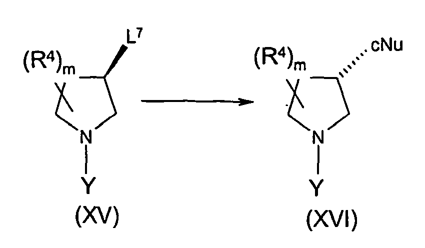 wherein R⁴ and m are as defined above, wherein L⁷ represents a leaving group, such as a halogen atom or a leaving group defined by OX above, wherein Y represents a protecting group or R¹ as defined above, and wherein cNu represents a carbon nucleophile that can be converted to a carboxylic acid.

The step indicated above is typically carried out by reaction with a carbon nucleophile that can be converted to a carboxylic acid, such as a cyanide salt (e.g. KCN), in a suitable solvent such as DMSO at a suitable temperature such as 90°C.

This scheme shows the production of the S enantiomer, however, it will be appreciated that the R enantiomer may be produced by an analogous process.

Compounds of formula (IV), (V), (VII), (IX) and R¹-L⁴ are either known in the literature or can be prepared by analogous methods.

Compounds of formula (I) and their pharmaceutically acceptable salts have affinity for and are antagonists and/or inverse agonists of the histamine H3 receptor and are believed to be of potential use in the treatment of neurological diseases including Alzheimer's disease, dementia (including Lewy body dementia and vascular dementia), age-related memory dysfunction, mild cognitive impairment, cognitive deficit, epilepsy, pain of neuropathic origin including neuralgias, neuritis and back pain, and inflammatory pain including osteoarthritis, rheumatoid arthritis, acute inflammatory pain and back pain, migraine, Parkinson's disease, multiple sclerosis, stroke and sleep disorders (including narcolepsy and sleep deficits associated with Parkinson's disease); psychiatric disorders including schizophrenia (particularly cognitive deficit of schizophrenia), attention deficit hypereactivity disorder, depression, anxiety and addiction; and other diseases including obesity and gastro-intestinal disorders.

It will also be appreciated that compounds of formula (I) are expected to be selective for the histamine H3 receptor over other histamine receptor subtypes, such as the histamine H1 receptor. Generally, compounds of the invention may be at least 10 fold selective for H3 over H1, such as at least 100 fold selective.

Thus the invention also provides a compound of formula (I) or a pharmaceutically acceptable salt thereof, for use as a therapeutic substance in the treatment or prophylaxis of the above disorders, in particular cognitive impairments in diseases such as Alzheimer's disease and related neurodegenerative disorders.

In another aspect, the invention provides the use of a compound of formula (I) or a pharmaceutically acceptable salt thereof in the manufacture of a medicament for use in the treatment of the above disorders.

When used in therapy, the compounds of formula (I) are usually formulated in a standard pharmaceutical composition. Such compositions can be prepared using standard procedures.

Thus, the present invention further provides a pharmaceutical composition for use in the treatment of the above disorders which comprises the compound of formula (I) or a pharmaceutically acceptable salt thereof and a pharmaceutically acceptable carrier.

The present invention further provides a pharmaceutical composition which comprises the compound of formula (I) or a pharmaceutically acceptable salt thereof and a pharmaceutically acceptable carrier.

Compounds of formula (I) may be used in combination with other therapeutic agents, for example medicaments claimed to be useful as either disease modifying or symptomatic treatments of Alzheimer's disease. Suitable examples of such other therapeutic agents may be agents known to modify cholinergic transmission such as 5-HT₆ antagonists, M1 muscarinic agonists, M2 muscarinic antagonists or acetylcholinesterase inhibitors. When the compounds are used in combination with other therapeutic agents, the compounds may be administered either sequentially or simultaneously by any convenient route. The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.1 to 200 mg and even more suitably 1.0 to 200 mg, and such unit doses may be administered more than once a day, for example two or three a day. Such therapy may extend for a number of weeks or months.

The following Descriptions and Examples illustrate the preparation of compounds of the invention. An Emrys^{™} Optimizer microwave reactor was employed for reactions carried out with microwave heating. Where indicated, Varian Mega BE (10g) SCX columns or Isolute Flash SCX-2 (20g) columns were used for the work-up of reactions. Crude mixtures were applied to the column, non-polar materials were washed off with methanol, and the desired amines were eluted with ammonia in methanol. In addition, where indicated, Mass Directed Auto-Purification or MDAP was carried out using a purification system supplied by Waters. The columns used were Waters Atlantis (19mm x 100mm or 30mm x 100mm). The solvent systems used comprised solvent A (water + 0.1 % formic acid) and solvent B (acetonitrile + 0.1 % formic acid) with gradients within the range 5-99% solvent B in solvent A.

### Description 1

### Benzyl (3R)-3-hydroxy-1-pyrrolidinecarboxylate (D1)

### Method A

Benzyl chloroformate (27.4ml) was added to a stirred solution of (*R*)-3-pyrrolidinol (obtainable from Lancaster 19499; 16g) and triethylamine (26.7ml) in dry DCM (250 ml) at 0°C over 10min under an inert atmosphere. The mixture was allowed to warm to room temperature and stirred for a further 2h. The solvent was evaporated and the residue partitioned between EtOAc (250ml) and aqueous 0.5M HCl (80ml). The organic layer was separated, washed with saturated sodium hydrogen carbonate solution (50ml) and brine (50ml), dried (MgSO₄) and evaporated to give the title compound (D1) as a pale orange oil (39g). LCMS electrospray (+ve) 244 (MNa⁺)

### Method B

Benzyl chloroformate (90ml of 95% purity) was dissolved in DCM (100 ml) and added dropwise to a stirred, ice-bath cooled, solution of (R)-3-pyrrolidinol (obtainable commerically from Lancaster; 50g) and triethylamine (84ml) in DCM (700 ml) over 40min under argon. The mixture was allowed to warm to room temperature and stirred for a further 3h. The mixture was transferred to a separating funnel and washed with 0.5M aqueous HCl (100 ml) and saturated sodium hydrogen carbonate solution (100ml). The organic layer was dried (MgSO₄) and evaporated to afford the title compound (D1) as a pale orange oil (126.1g). LCMS electrospray (+ve) 244 (MNa⁺)

### Description 2

### Benzyl (3R)-3-[(methylsulfonyl)oxy]-1-pyrrolidinecarboxylate (D2)

### Method A

Methanesulfonyl chloride (15ml) was added to a stirred solution of benzyl (3*R*)-3-hydroxy-1-pyrrolidinecarboxylate (may be prepared as described in Description 1, method A) (39g) and triethylamine (30ml) in DCM (400ml) at 0°C over 10min under an inert atmosphere. The reaction was allowed to warm to room temperature and stirred for a further 0.5h. The reaction mixture was then washed with saturated sodium hydrogen carbonate solution (80ml), brine (2 x 50ml), dried (MgSO₄) and evaporated to afford the title compound (D2) as a pale orange oil (50.7g). LCMS electrospray (+ve) 322 (MNa⁺)

### Method B

### Benzyl (3R)-3-[(methylsulfonyl)oxy]-1-pyrrolidinecarboxylate

Each of two 63g batches of benzyl (3*R*)-3-hydroxy-1-pyrrolidinecarboxylate (may be prepared as described in Description 1, method B) was separately dissolved in DCM (500ml) and triethylamine (47.5ml). The resultant solutions were stirred and cooled in an ice-bath under argon. Methanesulfonyl chloride (24.5ml) was added dropwise to each solution over 20 min. Each reaction was allowed to warm to room temperature and stirred for a further 1.5h. Both reaction mixtures were then combined in a separating funnel and washed with saturated sodium hydrogen carbonate solution (250ml) and brine (2 x 200ml). The organic layer was dried (Na₂SO₄) and evaporated *in vacuo* to afford the title compound (D2) as a thick orange oil (169g). LCMS electrospray (+ve) 322 (MNa⁺)

### Description 3

### Benzyl (3S)-3-cyano-1-pyrrolidinecarboxylate (D3)

### Method A

Potassium cyanide (21.8g) was added to a stirred solution of benzyl (3*R*)-3-[(methylsulfonyl)oxy]-1-pyrrolidinecarboxylate (may be prepared as described in Description 2, method A) (50g) in DMSO (300ml) and the mixture stirred at 90°C for 4h. The DMSO was evaporated to a minimum and the residue dissolved in EtOAc (500ml), washed with water (2 x 50ml), dried (MgSO₄) and evaporated. The residue was purified by flash chromatography [silica gel, EtOAc:hexane 1:1] and the pure fractions evaporated to give the title compound (D3) as a pale yellow oil (17.5g). LCMS electrospray (+ve) 253 (MNa⁺)

### Method B

Each of two 84.5g batches of benzyl (3*R*)-3-[(methylsulfonyl)oxy]-1-pyrrolidinecarboxylate (may be prepared as described in Description 2, method B) was separately dissolved in DMSO (650ml) and finely ground potassium cyanide (46g) was added to each solution. Both reactions were stirred at 90°C for 9h. The crude reaction mixtures were then combined and the DMSO evaporated *in vacuo.* Brine (600ml) was added to the residue and the mixture extracted with EtOAc (5 x 400ml). The combined organic extracts were then dried (Na₂SO₄) and evaporated *in vacuo.* The residue was purified by chromatography on silica (Flash 75 system), eluting with a gradient (0-50% EtOAc in hexane) and the pure fractions evaporated to afford the title compound (D3) as a pale yellow oil (89.4g). LCMS electrospray (+ve) 253 (MNa⁺)

### Description 4

### (3S)-1-(tert-Butoxycarbonyl)-3-pyrrolidinecarboxylic acid (D4)

### Method A

Benzyl (3*S*)-3-cyano-1-pyrrolidinecarboxylate (may be prepared as described in Description 3, method A) (17.5g) was heated at reflux in a mixture of conc. hydrochloric acid (200ml) and glacial acetic acid (40ml) for 4h. The reaction mixture was evaporated *in vacuo* and re-evaporated from toluene (2 x 100ml). The residue was dissolved in water (50ml) and acetone (30ml), then sodium carbonate (8.5g) and di-*tert-*butyldicarbonate (19.9g) were added sequentially. The mixture was stirred at room temperature for 16h before evaporation of the acetone *in vacuo.* The remaining aqueous solution was washed with diethyl ether (2 x 20ml), cooled in an ice-bath, acidified to pH 3-4 with 2M hydrochloric acid and extracted with EtOAc (3 x 100ml). The combined organic extracts were dried (MgSO₄) and evaporated to afford the title compound (D4) as a colourless solid (10.1g). LCMS electrospray (-ve) 214 (M-H). ¹H NMR (DMSO-d6) δ 1.39 (9H, s), 1.91-2.05 (2H, m), 2.99-3.06 (1H, m), 3.22-3.41 (4H, m), 12.48 (1H, bs)

### Method B

Benzyl (3*S*)-3-cyano-1-pyrrolidinecarboxylate (may be prepared as described in Description 3, method B) (88.5g) was heated at reflux in a mixture of conc. hydrochloric acid (450ml) and glacial acetic acid (100ml) for 3h. The reaction mixture was evaporated to dryness *in vacuo* and the residue dissolved in water (200ml) and acetone (120ml). The solution was cooled in an ice-bath, sodium carbonate (42.8g) was then added portionwise over 10min followed by di-*tert*-butyldicarbonate (101g) and the resultant mixture stirred at room temperature for 16h. Acetone was evaporated from the mixture *in vacuo* and the remaining aqueous solution was washed with diethyl ether (2 x 50ml). The aqueous layer was then cooled in an ice-bath, the pH was adjusted to pH 3-4 with 2M hydrochloric acid (keeping internal temperature below 10°C) and extracted with EtOAc (4 x 400ml). The combined organic extracts were dried (Na₂SO₄) and evaporated. The residue was dissolved in a minimum volume of hot EtOAc and allowed to crystallise overnight. The residue was filtered and dried to afford the title compound (D4) as an off-white crystalline solid (54.6g). A further 4.8g was obtained by evaporating the mother liquors and recrystallising the residue from hot EtOAc. LCMS electrospray (-ve) 214 (M-H).¹H NMR (DMSO-d6) δ 1.39 (9H, s), 1.91-2.08 (2H, m), 2.99-3.07 (1H, m), 3.18-3.44 (4H, m), 12.48 (1H, bs)

### Description 5

### tert-Butyl (3S)-3-{[4-(1-methylethyl)-1-piperazinyl]carbonyl}-1-pyrrolidinecarboxylate (D5)

### Method A

EDC (3.21 g), HOBT (1.13g) and 1-(1-methylethyl)piperazine (obtainable from Chess 1214; 1.07g) were added sequentially to a stirred solution of (3*S*)-1-(*tert-*butoxycarbonyl)-3-pyrrolidinecarboxylic acid (may be prepared as described in Description 4, method A) (1.8g) in DMF (20ml) and the reaction mixture stirred at room temperature under argon for 3h. The DMF was removed by evaporation and the residue partitioned between EtOAc/saturated sodium hydrogen carbonate solution (120:20ml). The organic layer was dried (Na₂SO₄), evaporated and the residue purified by flash chromatography [silica gel, 0-10% MeOH (containing 10% 0.88 ammonia solution)/DCM]. The clean fractions were evaporated to afford the title compound (D5) as a pale yellow oil (2.37g). LCMS electrospray (+ve) 348 (MNa⁺)

### Method B

EDCl (35.6g) and HOBT (12.6g) were added sequentially to a stirred solution of (3S)-1-(*tert*-butoxycarbonyl)-3-pyrrolidinecarboxylic acid (may be prepared as described in Description 4, method B) (20g) in DMF (200ml). 1-(1-Methylethyl)piperazine (obtainable commercially from Chess; 13.3ml) was then added and the reaction mixture stirred at room temperature under argon for 3h. The DMF was evaporated to a minimum and the residue partitioned between EtOAc/water (500:150ml). The layers were separated and the aqueous layer extracted with EtOAc (3 x 400ml). The combined extracts were washed with brine (40ml), dried (Na₂SO₄) and evaporated to afford the title compound (D5) as a pale orange oil (27.1g). LCMS electrospray (+ve) 348 (MNa⁺)

### Method C

EDCI (17.8g), HOBT (6.28g) and 1-(1-Methytethyl)piperazine (obtainable commercially from Chess; 5.96g, 6.65ml) were added to a solution of (3*S*)-1-(*tert*-butoxycarbonyl)-3-pyrrolidinecarboxylic acid (may be prepared as described in Description 4, method B) (10g) in DMF (100ml). The reaction mixture was stirred at room temperature under argon for 3h. The DMF was evaporated, followed by a co-evaporation from ethyl acetate. The residue was partitioned between ethyl acetate (300 ml) and water (60 ml). The aqueous layer was re-extracted with ethyl acetate (240 ml) and combined with the original ethyl acetate layer. The organic extracts were dried (MgSO₄), filtered and evaporated to dryness to afford the title compound (D5) as a brown oil (10.8g).
LCMS electrospray (+ve) 326 (MH+)

### Description 6

### 1-(1-Methylethyl)-4-[(3S)-3-pyrrolidinylcarbonyl]piperazine (D6)

### Method A

4M HCl in dioxane (15ml) was added to a solution of *tert*-Butyl (3*S*)-3-{[4-(1-methylethyl)-1-piperazinyl]carbonyl}-1-pyrrolidinecarboxylate (may be prepared as described in Description 5, method A) (2.37g) in MeOH (15ml) and the mixture stirred for 3h under an argon atmosphere. The reaction mixture was then evaporated to dryness and the residue basified with saturated potassium carbonate solution (10 ml) and extracted with DCM (3 x 50ml). The combined organic extracts were dried (Na₂SO₄) and evaporated to afford the title compound (D6) as a pale yellow thick oil (1.46g). LCMS electrospray (+ve) 226 (MH⁺). ¹H NMR (CDCl₃) δ 1.05 (6H, d, J=6.4Hz), 1.92-2.03 (3H, m), 2.47-2.52 (4H, m), 2.67-2.93 (3H, m), 3.04-3.13 (2H, m), 3.18-3.22 (1H, m), 3.52-3.55 (2H, m), 3.61-3.64 (2H, m).

### Method B

4M HCl in dioxane (60ml) was added to a solution of *tert*-Butyl (3*S*)-3-{[4-(1-methylethyl)-1-piperazinyl]carbonyl}-1-pyrrolidinecarboxylate (may be prepared as described in Description 5, method B) (27g) in MeOH (60ml) and the mixture stirred for 4h under an argon atmosphere. The solvent was removed in vacuo and the residue dissolved in saturated potassium carbonate solution (100ml) and extracted with DCM (4 x 400ml). The combined organic extracts were dried (Na₂SO₄) and evaporated to afford the title compound (D6) as a pale orange oil (12.92g). LCMS electrospray (+ve) 226 (MH⁺). ¹H NMR (CDCl₃) δ 1.05 (6H, d, J=6.4Hz), 1.91-2.04 (3H, m), 2.47-2.53 (4H, m), 2.67-2.93 (3H, m), 3.04-3.13 (2H, m), 3.18-3.22 (1H, m), 3.52-3.55 (2H, m), 3.61-3.64 (2H, m).

### Method C

4M HCl in dioxane (50ml) was added to a solution of *tert*-Butyl (3*S*)-3-{[4-(1-methylethyl)-1-piperazinyl]carbonyl}-1-pyrrolidinecarboxylate (may be prepared as described in Description 5, method C) (10.8g) in MeOH (50ml) and the mixture stirred for 16h under an argon atmosphere. The reaction mixture was evaporated to dryness and the residue dissolved in methanol and loaded onto an SCX cartridge, eluting with methanol, followed by 2M ammonia/methanol. The appropriate fractions were combined and evaporated to dryness, to afford the title compound (D6) as an orange/brown oil (4.66g).
LCMS electrospray (+ve) 226 (MH+)

### Description 7

### 1-tert-Butoxycarbonyl-4-cyclobutyl-piperazine (D7)

1-*tert*-Butoxycarbonyl-piperazine (5.6g) and cyclobutanone (2.10g) were dissolved in DCM (100ml) and the reaction mixture was stirred at room temperature for 30min. Sodium triacetoxyborohydride (6.37g) was added portion-wise over 10min. The mixture was then stirred overnight. The reaction mixture was washed with 1 N NaOH (70ml) and the DCM layer was dried (MgSO₄) and evaporated to give the title compound as an oil (6.5g) ¹H NMR δ [DMSO-d6]: 1.39 (9H, s), 1.68-1.87 (4H, m), 1.9-2.01 (2H, m), 2.15-2.2 (3H, m), 2.5 (1H, m),2.6-2.78 (1H, m), 3.18-3.3 (4H, m).

### Description 8

### 1-Cyclobutyl-piperazine dihydrochloride (D8)

1-*tert*-Butoxycarbonyl-4-cyclobutyl-piperazine (may be prepared as described in Description 7) (6.1g) was dissolved in dry MeOH (70ml) followed by the addition of 4N HCl in dioxane (20ml). The reaction mixture was stirred at room temperature under argon for 4 hours. The solvent was evaporated and the precipitate was filtered off and dried in a vacuum oven to give the title compound (D8) as a white solid (4.05g). ¹H NMR (DMSO): 1.63-1.80 (2H, m), 2.12-2.19 (2H, m), 2.34-2.39 (2H, m), 3.08 (2H, m), 3.43-3.56 (6H, m), 3.71 (1H, m), 9.70 (2H, bs) and 12.40 (1H, bs).

### Description 9

### tert-Butyl (3S)-3-[(4-cyclobutyl-1-piperazinyl)carbonyl]-1-pyrrolidinecarboxylate (D9)

EDC (2.79g), HOBT (0.956g) were added to a solution of (3*S*)-1-(*tert*-butoxycarbonyl)-3-pyrrolidinecarboxylic acid (may be prepared as described in Description 4) (1.522g) in DMF (50ml) and the reaction was stirred for 10 minutes. Triethylamine (2.88 ml) was then syringed into the reaction mixture and this was followed by addition of 1-cyclobutyl-piperazine dihydrochloride (may be prepared as described in Description 8) (1.5g) dissolved in DMF (25ml). The reaction mixture was stirred at room temperature for 3h. 50 ml water was added to the reaction mixture and it was then washed with 3 portions of EtOAc (30 ml). The organic layer was washed twice with brine (30 ml) and seven times with saturated NaHCO₃ (30 ml). Following this, the organic component was dried over MgSO₄ and filtered. The filtrate was evaporated of solvent to give the title compound (D9) as a light brown solid (2.81g). LCMS electrospray (+ve) 360 (MNa⁺)

### Description 10

### 1-Cyclobutyl-4-[(3S)-3-pyrrolidinylcarbonyl]piperazine (D10)

4M HCl in dioxane (5ml) was added to a solution of *tert*-Butyl (3S)-3-[(4-cyclobutyl-1-piperazinyl)carbonyl]-1-pyrrolidinecarboxylate (may be prepared as described in Description 9) (2.81g) in MeOH (75ml) and the mixture was left to stand overnight. The solvent was then evaporated to give a brown oil. The residue was dissolved in MeOH and loaded onto an SCX column. The column was washed with MeOH and eluted with 2M NH₃/MeOH. Ammoniacal fractions were combined and the solvent was evaporated to afford the title compound (D10) as a brown oil (0.8g). LCMS electrospray (+ve) 238 (MH⁺). ¹H NMR (CDCl₃) δ 1.61-2.10 (9H, m), 2.27-2.32 (4H, m), 2.68-3.20 (6H, m), 3.52-3.55 (2H, m), 3.62-3.65 (2H, m).

### Description 11

### tert-Butyl (3S)-3-{[4-(1-ethylpropyl)-1-piperazinyl]carbonyl}-1-pyrrolidinecarboxylate (D11)

EDC (3.21g), HOBT (1.13g) and 1-(1-ethylpropyl)-piperazine (1.31g) were added sequentially to a stirred solution of (3*S*)-1-(*tert*-butoxycarbonyl)-3-pyrrolidinecarboxylic acid (may be prepared as described in Description 4) (1.8g) in DMF (20ml) and the reaction mixture stirred at room temperature under argon for 3h. The DMF was removed by evaporation and the residue partitioned between EtOAc/saturated sodium hydrogen carbonate solution (120:20ml). The organic layer was dried (Na₂SO₄), evaporated and the residue purified by flash chromatography [silica gel, 0-10% MeOH (containing 10% 0.88 ammonia solution)/DCM]. The clean fractions were evaporated to afford the title compound (D11) as a pale yellow oil (2.80g). LCMS electrospray (+ve) 376 (MNa⁺)

### Description 12

### 1-(1-Ethylpropyl)-4-[(3S)-3-pyrrolidinylcarbonyl]piperazine (D12)

4M HCl in dioxane (15ml) was added to a solution of *tert*-Butyl (3*S*)-3-{[4-(1-ethylpropyl)-1-piperazinyl]carbonyl}-1-pyrrolidinecarboxylate (may be prepared as described in Description 11) (2.80g) in MeOH (15ml) and the mixture stirred for 3h under an argon atmosphere. The reaction mixture was then evaporated to dryness and the residue basified with saturated potassium carbonate solution (10 ml) and extracted with DCM (3 x 50ml). The combined organic extracts were dried (Na₂SO₄) and evaporated to afford the title compound (D12) as a pale yellow thick oil (2.0g). LCMS electrospray (+ve) 254 (MH⁺). ¹H NMR (CDCl₃) δ 0.90 (6H, t, J=7.2Hz), 1.22-1.36 (2H, m), 1.37-1.51 (2H, m), 1.88-2.02 (2H, m), 2.14-2.21 (1H, m), 2.46-2.52 (4H, m), 2.78-3.22 (6H, m), 3.47-3.50 (2H, m), 3.56-3.59 (2H, m).

### Description 13

### tert-Butyl 4-(cyclopropy)methyl)-hexahydro-1H-1,4-diazepine-1-carboxylate (D13)

(Bromomethyl)cyclopropane (3.9 ml) was added to a stirred mixture of potassium carbonate (6.9g) and *tert-butyl* hexahydro-1*H*-1,4-diazepine-1-carboxylate (Aldrich 51,138-2; 5.0g) in acetonitrile (70ml) and the mixture heated at reflux for 5h. The solvent was evaporated and the residue partitioned between EtOAc (100ml) and water (30ml). The organic layer was dried, evaporated and the residue purified by chromatography [silica gel, 0-10% MeOH (containing 10% 0.88 ammonia solution)/DCM]. The pure fractions were evaporated to afford the title compound (D13) as a pale yellow solid (3.25g). LCMS electrospray (+ve) 255 (MH⁺).

### Description 14

### 1-(Cyclopropylmethyl)-hexahydro-1H-1,4-diazepine dihydrochloride (D14)

4M HCl in dioxane (10ml) was added to a solution of the *tert*-Butyl 4-(cyclopropylmethyl)-hexahydro-1H-1,4-diazepine-1-carboxylate (may be prepared as described in Description 13) (3.25g) in MeOH (10ml) and the solution was stirred for 2.5h at room temperature. The solvent was evaporated to give the title compound (D14) as a beige solid (2.8g). LCMS electrospray (+ve) 155 (MH⁺).

### Description 15

### tert-Butyl (3S)-3-{[4-(cyclopropylmethyl)-hexahydro-1H-1,4-diazepin-1-yl]carbonyl}pyrrolidine-1-carboxylate (D15)

EDC (2.67g), HOBT (0.94g) and 1-(cyclopropylmethyl)-hexahydro-*1H*-1,4-diazepine dihydrochloride (may be prepared as described in Description 14) (1.6g) were added sequentially to a stirred solution of (3*S*)-1-(*tert*-butoxycarbonyl)-3-pyrrolidinecarboxylic acid (may be prepared as described in Description 4) (1.5g) in DMF (20ml). Diisopropylethylamine (2.67ml) was added and the reaction mixture stirred at room temperature under argon for 3h. The DMF was removed by evaporation and the residue partitioned between EtOAc/saturated sodium hydrogen carbonate solution (100:10ml).

The organic layer was dried (Na₂SO₄) and evaporated to afford the title compound (D15) as a pale yellow oil (1.96g). LCMS electrospray (+ve) 352 (MH⁺)

### Description 16

### 1-(Cyclopropylmethyl)-4-[(3S)-pyrrolidin-3-ylcarbonyl]-hexahydro-1H-1,4-diazepine (D16)

4M HCl in dioxane (10ml) was added to a solution of tert-butyl (3S)-3-{[4-(cyclopropylmethyl)-hexahydro-1*H*-1,4-diazepin-1-yl]carbonyl}pyrrolidine-1-carboxylate (may be prepared as described in Description 15) (1.96g) in MeOH (10ml) and the mixture stirred overnight under an argon atmosphere. The reaction mixture was then evaporated to dryness and the residue dissolved in MeOH, loaded onto SCX (20g) and the cartridge eluted with MeOH followed by 2MNH₃/MeOH (50ml). The ammoniacal fractions were evaporated to afford the title compound (D16) as a pale orange oil (0.795g). LCMS electrospray (+ve) 252 (MH⁺). ¹H NMR (CDCl₃) δ 0.0-0.1 (2H, m), 0.39-0.48 (2H, m), 0.70-0.81 (1H, m), 1.74-1.96 (5H, m), 2.29 (2H, t, J=6.4Hz), 2.57-2.76 (5H, m), 2.85 (1H, dd, J=11.2, 8.4Hz), 2.97-3.13 (3H, m), 3.47-3.59 (4H, m).

### Description 17

### (3S)-1-Benzyloxycarbonyl-3-methylpiperazine (D17)

(S)-2-methylpiperazine (10g) was dissolved in dry DCM (200ml). Triethylamine (14.5ml) was added and the reaction was cooled to 0°C. Benzyl chloroformate (18.66g) in dry DCM (30ml) was added dropwise to the reaction mixture, which was then stirred at room temperature for 3h. The DCM layer was then washed with saturated bicarbonate and water, dried (MgSO₄) and filtered and stripped to give an oil. The oil was absorbed onto silica and then purified by chromatography on a silica column (Flash, size E column) eluting with a gradient of MeOH/NH₄OH in DCM (the MeOH/NH₄OH component being comprised of methanol containing 10% 0.88 ammonia solution). The product was eluted with 6% of the NH₄OH/MeOH component to give the title compound (D17) as an oil (10.18g). LCMS electrospray (+ve) 235 (MH⁺).

### Description 18

### (2S)-1-(1-Methylethyl)-4-(benzyloxycarbonyl)-2-methylpiperazine (D18)

Potassium carbonate (8.8g) was added to a solution of (3S)-1-benzyloxycarbonyl-3-methylpiperazine (may be prepared as described in Description 17) (7.5g) in CH₃CN (100ml), followed by isopropyl iodide (10.0ml) and the mixture was heated at reflux overnight. The reaction mixture was then allowed to cool to room temperature and the potassium carbonate was filtered off. The filtrate was stripped to give an oil which was partitioned between EtOAc and water. The EtOAc layer was dried (MgSO₄), filtered and stripped to give the title compound (D18) as an oil (8.39g). LCMS electrospray (+ve) 277 (MH⁺).

### Description 19

### (2S)-1-(1-Methylethyl)-2-methylpiperazine dihydrochloride (D19)

(2S)-1-(1-methylethyl)-4-(benzyloxycarbonyl)-2-methylpiperazine (may be prepared as described in Description 18) (8.39g) was dissolved in EtOH (150ml) and treated with 10%Pd/C paste (4 heaped spatulas) and stirred under atmospheric hydrogen conditions overnight. The catalyst was filtered off and the solvent was removed by evaporation. 1 N ethereal HCl (100ml) was added and the reaction mixture was stripped, filtered and dried in a dessicator to give the title compound (D18) (4.5g). LCMS electrospray (+ve) 143 (MH⁺).

### Description 20

### tert-Butyl (3S)-3-{[(3S)-4-(1-methytethyl)-3-methyl-1-piperazinyl]carbonyl}-1-pyrrolidinecarboxylate (D20)

EDC (1.73g), HOBT (0.609g) and (2S)-1-(1-methylethyl)-2-methylpiperazine dihydrochloride (may be prepared as described in Description 19) (0.978g) and diisopropylethylamine (1.73ml) were added sequentially to a solution of (3*S*)-1-(*tert-*butoxycarbonyl)-3-pyrrolidinecarboxylic acid (may be prepared as described in Description 4) (0.97g) in DMF (10ml) and the reaction mixture was stirred at room temperature under argon for 3h. Most of the DMF was removed by evaporation and the residue partitioned between EtOAc/saturated sodium hydrogen carbonate solution (150:20ml). The layers were separated and the organic layer was washed with water (5ml), dried (MgSO₄), evaporated and the residue purified by chromatography on a silica column (FM(II) system), eluting with 0-10% 2M NH₃/MeOH in DCM. The clean fractions were evaporated to afford the title compound (D20) as a pale yellow oil (0.86g). LCMS electrospray (+ve) 362 (MNa⁺)

### Description 21

### (2S)-1-(1-Methylethyl)-2-methyl-4-[(3S)-3-pyrrolidinylcarbonyl]piperazine (D21)

4M HCl in dioxane (10 ml) was added to a solution of tert-butyl (3S)-3-{[(3S)-4-(1-methylethyl)-3-methyl-1-piperazinyl]carbonyl}-1-pyrrolidinecarboxylate (may be prepared as described in Description 20) (0.86g) in MeOH (5ml) and the mixture stirred overnight under an argon atmosphere. The solvent was removed *in vacuo* and the residue was dissolved in MeOH (20ml), loaded onto a 20g SCX cartridge and eluted with MeOH (50ml) followed by 2M NH₃/MeOH (50ml). The ammoniacal fractions were evaporated and dried to afford the title compound (D21) as a pale yellow oil (0.58g). LCMS electrospray (+ve) 240 (MH⁺). ¹H NMR (CDCl₃) δ 0.84 (3H, d, J=6.4Hz), 1.06 (3H, t, J=7.2), 1.09 (3H, d, J=6.4Hz), 1.90-2.04 (4H, m), 2.19-2.29 (1H, m), 2.50-3.29 (9H, m), 3.62-3.83 (1H, m), 4.17-4.29 (1H, m).

### Description 22

### 4-Bromo-N-[(1-(dimethylamino)ethylidene]benzamide (D22)

4-Bromobenzamide (51.48g) was heated in N,N-dimethylacetamide dimethylacetal (165ml) at 120°C for 2h. The solution was allowed to cool overnight and the product crystallised as pale yellow needles, which were collected by filtration, washed on the filter with diethyl ether and dried overnight at 40°C *in vacuo* to give the title compound (D22) (57.84g). ¹H NMR δ [DMSO-d6]: 2.26 (3H, s), 3.13 (3H, s), 3.14 (3H, s), 7.61 (2H, d, J = 8.6Hz), 7.94 (2H, d, J = 8.6Hz).

### Description 23

### 5-(4-Bromophenyl)-3-methyl-1,2,4-oxadiazole (D23)

4-Bromo-*N*-[(1-(dimethylamino)ethylidene]benzamide (may be prepared as described in Description 22) (57.8g) was treated with a solution of hydroxylamine hydrochloride (19.6g) in 1 M NaOH solution (350ml). Dioxane (350ml) and glacial acetic acid (450ml) were added, and the resulting solution was stirred at 25°C for 30min and then at 90°C for 3h. After cooling overnight, the crystalline product (colourless needles) was collected by filtration, washed with dilute aqueous acetic acid and water and dried at 50°C *in vacu*o to give the title compound (D23). Concentration of the filtrate yielded a second crop of product, spectroscopically identical to the first, which was collected and dried as before (46.1g total). ¹H NMR δ [CDCl₃]: 2.48 (3H, s), 7.67 (2H, d, J = 8.4Hz), 7.98 (2H, d, J = 8.4Hz); (MH)⁺ = 239, 241.

### Description 24

### tert-Butyl 4-(cyclobutyl)-hexahydro-1H-1,4-diazepine-1-carboxylate (D24)

*tert*-Butyl hexahydro-1*H*-1,4-diazepine-1-carboxylate (Aldrich 51,138-2; 10.0g) was dissolved in DCM (300ml). Cyclobutanone (7.5ml) was added and the reaction was left to stir for 5min. Sodium triacetoxyborohydride (21.1g) was then added and the reaction was stirred at room temperature for 16h. The reaction mixture was washed with saturated potassium carbonate solution (2 x 200ml). The organic layer was dried (MgSO₄) and evaporated to give the title compound (D24) as a clear oil (11.3g).

### Description 25

### 1-(Cyclobutyl)hexahydro-1H-1,4-diazepine dihydrochloride (D25)

tert-Butyl 4-(cyclobutyl)-hexahydro-1*H*-1,4-diazepine-1-carboxylate (may be prepared as described in Description 24) (11.3g) was dissolved in methanol (200ml) and 4N HCl in dioxan (100ml) was added. The reaction was stirred at room temperature for 3h and then co-evaporated from toluene (3 x 50ml) to give the title compound (D25) as a white solid (9.8g). ¹H NMR δ (DMSO-d6): 11.95 (1H, s), 9.55 (1H, s), 9.64 (1H, s), 3.78-3.08 (9H, m), 2.51-2.07 (6H, m), 1.80-1.51 (2H, m).

### Description 26 1-(Cyclopropylmethyl)piperazine dihydrochloride (D26)

(Bromomethyl)cyclopropane (2.86 ml) was added to a stirred mixture of potassium carbonate (7.4g) and *tert*-butyl 1-piperazinecarboxylate (5.0g) in acetonitrile (70ml) and the mixture heated at reflux for 5h. The mixture was cooled, filtered and the solvent evaporated. The residue was dissolved in MeOH (20ml), added to 4M HCl in dioxane (20 ml) and the solution stirred for 2.5h at room temperature. The precipitate was filtered off and dried to give the title compound (D26) as a white solid (4.4g). LCMS electrospray (+ve) 141 (MH⁺).

### Description 27

### tert-Butyl (3S)-3-{[4-(cyclopropylmethyl)-1-piperazinyl]carbonyl}-1-pyrrolidinecarboxylate (D27)

EDC (2.67g), HOBT (0.94g) and 1-(cyclopropylmethyl)piperazine dihydrochloride (1.5g) (may be prepared as described in Description 26) were added sequentially to a stirred solution of (3*S*)-1-(*tert*-butoxycarbonyl)-3-pyrrolidinecarboxylic acid (may be prepared as described in Description 4) (1.5g) in DMF (20ml). Diisopropylethylamine (2.67ml) was added and the reaction mixture stirred at room temperature under argon for 3h. The DMF was removed by evaporation and the residue partitioned between EtOAc/saturated sodium hydrogen carbonate solution (100:10ml). The organic layer was dried (MgSO₄) and evaporated to afford the title compound (D27) as a pale yellow oil (1.83g). LCMS electrospray (+ve) 338 (MH⁺)

### Description 28

### 1-(Cyclopropylmethyl)-4-[(3S)-3-pyrrolidinylcarbonyl] piperazine (D28)

4M HCl in dioxane (20ml) was added to a solution of *tert-*butyl (3S)-3-{[4-(cyclopropylmethyl)-1-piperazinyl]carbonyl}-1-pyrrolidinecarboxylate (may be prepared as described in Description 27) (1.83g) in MeOH (10ml) and the mixture stirred for 16h under an argon atmosphere and then evaporated to dryness. The residue was dissolved in MeOH (50ml), loaded onto a 20g SCX cartridge and eluted with MeOH (50ml) followed by 2M NH₃/MeOH (50ml). The ammoniacal fractions were evaporated to afford the title compound (D28) as a pale yellow thick oil (1.16g). LCMS electrospray (+ve) 238 (MH⁺). ¹H NMR (CDCl₃) δ: 0.0-0.1 (2H, m), 0.39-.0.48 (2H, m), 0.70-0.81 (1H, m), 1.80-1.92 (2H, m), 2.10-2.20 (3H, m), 2.36-2.42 (4H, m), 2.69-3.11 (5H, m), 3.43-3.57 (4H, m).

### Description 29

### (R,S)-1-tert-Butyl 3-[(4-cyclobutylhexahydro-1H-1,4-diazepin-1-yl)carbonyl]-1-pyrrolidinecarboxylate (D29)

1-(*tert*-Butoxycarbonyl)-3-pyrrolidinecarboxylic acid (6.6g) in dry DMF (150ml) was treated with EDC (12.17g) and HOBT (4.17g). The reaction mixture was then stirred at room temperature for 20min. 1-(Cyclobutyl)hexahydro-1*H*-1,4-diazepine dihydrochloride (may be prepared as described in Description 25) (6.66g) was added to the stirred solution followed by triethylamine (12.8ml) and the reaction mixture was stirred at room temperature overnight. The mixture was then poured into water (500ml) and extracted with EtOAc (2x100ml). The combined organic layers were washed with water (5x100ml), dried (MgSO₄) and concentrated to give a brown oil (8.8g). LCMS electrospray (+ve) 352 (MH⁺).

### Description 30

### (R,S)-1-Cyclobutyl-4-(3-pyrrolidinylcarbonyl)hexahydro-1H-1,4-diazepine (D30)

### Method A

(R,S)-1-*tert*-Butyl 3-[(4-cyclobutylhexahydro-1*H*-1,4-diazepin-1-yl)carbonyl]-1-pyrrolidinecarboxylate (may be prepared as described in Description 29) (1.7g) in dry MeOH (30ml) was treated with 4N dioxane HCl (10ml) and stirred at room temperature overnight. The reaction mixture was concentrated to dryness to give a brown oil (1.7g) which was dissolved in water (5ml) and treated with excess solid K₂CO₃ to liberate the free base which was extracted into EtOAc. The organic extracts were dried (MgSO₄) and concentrated to give the title compound (D30) as an oily residue (1.08g). LCMS electrospray (+ve) 252 (MH⁺).

### Method B

A solution of (R,S)-1-*tert*-Butyl *3*-[(4-cyclobutylhexahydro-1*H-1*,4-diazepin-1-yl)carbonyl]-1-pyrrolidinecarboxylate (may be prepared as described in Description 29) (8.8g) in MeOH (200ml), was treated with 4N dioxane HCl (40ml) and then stirred at room temperature overnight. The solvent was evaporated to give a brown gum which was dissolved in MeOH (60ml) and divided into 3 aliquots. Each aliquot (20ml) was poured on to a 50g SCX column which was washed with MeOH (50ml) and then eluted with 2N MeOH ammonia solution (40ml). The ammonia solutions were combined and evaporated to dryness to the give the title compound (D30) (5.5g) LCMS electrospray (+ve) (252) (MH+).

### Description 31

### 5-(4-Bromo-3-fluorophenyl)-3-methyl-1,2,4-oxadiazole (D31)

4-Bromo-3-fluorobenzoic acid (10.09g) was heated at reflux in thionyl chloride (100ml) for 4h and then allowed to cool. The mixture was evaporated *in vacuo* and the residue re-evaporated with DCM (2x) to give the acid chloride as a light brown oil. This was added dropwise to vigorously stirred, ice-cooled concentrated aqueous ammonia (100ml) and the precipitated product was collected by filtration, washed on the filter with water and dried at 40°C *in vacuo* to give 4-bromo-3-fluorobenzamide as a white solid (9.13g). This material and *N,N*-dimethylacetamide dimethyl acetal (27ml) were heated together at 120°C for 2h. The reaction was allowed to cool to room temperature and the liquid evaporated *in vacuo* to give a brown gum which was partitioned between saturated aqueous sodium hydrogen carbonate and EtOAc. The organic extract was washed with water and brine, dried and evaporated to give the acylamidine intermediate as a gum which solidified *in vacuo,* overnight (12.3 g). This intermediate was treated with a solution of hydroxylamine hydrochloride (4.16g) in 1M aqueous NaOH (74.2ml), dioxane (75ml) and glacial acetic acid (95ml). The reaction mixture was first stirred at room temperature for 30min then heated at 90°C for 3h. On cooling a first crop of crystals was filtered off and dried *in vacuo* at 50°C to give the title compound (D31) (5.5g). The filtrate afforded a second crop of crystals (2.1g). LCMS electrospray (+ve) 257 and 259 (MH⁺).

### Description 32

### 5-(4-Bromo-2-fluorophenyl)-3-methyl-1,2,4-oxadiazole (D32)

4-Bromo-2-fluorobenzoic acid (5.27g) was heated at reflux in thionyl chloride (50ml) for 4h and then allowed to cool. The mixture was evaporated *in vacuo* and the residue re-evaporated with DCM (2x) to give the acid chloride as a light brown oil. This was added dropwise to vigorously stirred, ice-cooled concentrated aqueous ammonia (50ml) and when addition was complete the mixture was stirred for 5min and then extracted (3x) with EtOAc. The combined organic extracts were washed with water and brine, dried (Na₂SO₄) and evaporated to give 4-bromo-2-fluorobenzamide as a white solid (4.72g). This material and *N,N*-dimethylacetamide dimethyl acetal (17ml) were heated together at 120°C for 2h. The reaction was allowed to cool to rt and the liquid evaporated *in vacuo* to give a brown gum which was partitioned between saturated aqueous sodium hydrogen carbonate and EtOAc. The organic extract was washed with water and brine, dried (Na₂SO₄) and evaporated to a gum. This was purified by chromatography (silica gel, eluant hexane/EtOAc) to give the acylamidine intermediate as a gum which solidified *in vacuo* (4.15g). Hydroxylamine hydrochloride (1.32g) in 1 N NaOH solution (23.5ml) was added, followed by dioxane (23.5ml) then AcOH (30ml). The reaction mixture was stirred at rt for 30min then heated at 90°C for 3h. The reaction was allowed to cool to rt and poured into water. The pH was adjusted to -9 by addition of solid NaHCO₃ and the precipitated product was collected by filtration, washed on the filter with water and dried at 40°C *in vacuo* to give the title compound (D32) as a greyish-brown solid (2.82g). LCMS electrospray (+ve) 257 and 259 (MH⁺).

### Description 33

### 1-tert-Butyl 4-(1-methylethyl)-hexahydro-1H-1,4-diazepine-1-carboxylate (D33)

### Method A

*tert*-Butyl hexahydro-1*H*-1,4-diazepine-1-carboxylate (obtainable from Aldrich 51,138-2; 10.0g) was dissolved in DCM (200ml). Acetone (7.33ml) was added and the reaction was left to stir for 5min. Sodium triacetoxyborohydride (21.0g) was then added and the reaction was stirred at room temperature for 16h. The reaction mixture was washed with saturated potassium carbonate solution (2 x 200ml). The organic layer was dried (magnesium sulphate) and evaporated to give the title compound (D33) as a clear oil (11.0g).

### Method B

*tert-*Butyl hexahydro-1*H*-1,4-diazepine-1-carboxylate (obtainable from Aldrich 51,138-2; 25.06g) was dissolved in acetonitrile (250ml). Anhydrous potassium carbonate (34.5g) and 2-iodopropane (63g, 37ml) were added and the mixture was heated at reflux for 18h. The cooled mixture was filtered and the solids were washed with acetonitrile. The combined filtrates were evaporated and the residual oil was dissolved in diethyl ether, washed with water, sodium thiosulphate solution and brine, dried (Na₂SO₄) and evaporated to give the title compound (D33) as a light brown oil (29.8g).

### Description 34

### 1-(1-Methylethyl)-hexahydro-1H-1,4-diazepine dihydrochloride (D34)

1-*tert*-Butyl 4-(1-methylethyl)-hexahydro-1*H*-1,4-diazepine-1-carboxylate (may be prepared as described in Description 33) (11.0g) was dissolved in methanol (200ml) and 4N HCl in dioxan (100ml) was added. The reaction was stirred at room temperature for 2h and then evaporated to give the title compound (D34) as a white solid (9.6g). ¹H NMR δ (CDCl₃): 11.35 (1H, s), 10.22 (1H, s), 9.72 (1H, s), 4.15-3.52 (9H, m), 2.83-2.40 (2H, m), 1.47 (6H, d, J=6.24Hz).

### Description 35

### (R,S)-tert-Butyl 3-{[4-(1-methylethyl)-hexahydro-1H-1,4-diazepin-1-yl]carbonyl}-1-pyrrolidinecarboxylate (D35)

EDC (3.57g), HOAT (0.1g) and 1-(1-methylethyl)-hexahydro-1*H*-1,4-diazepine dihydrochloride (may be prepared as described in Description 34) (2.0g) were added sequentially to a stirred solution of 1-(*tert*-butoxycarbonyl)-3-pyrrolidine carboxylic acid (2.0g) in DMF (80ml). Diisopropylethylamine (4.06ml) was added and the reaction mixture stirred at room temperature under argon for 3h. Saturated sodium hydrogen carbonate solution (100ml) was added and the mixture extracted with EtOAc (100ml x 3) The combined organic extracts were dried (Na₂SO₄), evaporated and the residue purified by flash chromatography [silica gel, 0-10% MeOH (containing 10% 0.88 ammonia solution)/DCM]. The pure fractions were evaporated to afford the title compound (D35) as a white solid (1.55g). LCMS electrospray (+ve) 362 (MNa⁺)

### Description 36

### (R,S)-1-(1-Methylethyl)-4-(3-pyrrolidinylcarbonyl)hexahydro-1H-1,4-diazepine (D36)

4M HCl in dioxane (10ml) was added to a solution of (R,*S*)-*tert*-butyl 3-{[4-(1-methylethyl)-hexahydro-1*H*-1,4-diazepin-1-yl]carbonyl}-1-pyrrolidinecarboxylate (may be prepared as described in Description 35) (1.52g) in MeOH (10ml) and the mixture stirred for 2h under an argon atmosphere. The reaction mixture was then evaporated to dryness and the residue basified with saturated potassium carbonate solution (10 ml) and extracted with DCM (3 x 50ml). The combined organic extracts were dried (Na₂SO₄) and evaporated to afford the title compound (D36) as a colourless oil (0.78g). ¹H NMR (CDCl₃) δ 0.99 (6H,d, J=6.5Hz), 1.77-2.05 (4H, m), 2.52-3.12 (11H, m), 3.52-3.62 (4H, m).

### Description 37

### (3R)-1-(tert-Butoxycarbonyl)-3-pyrrolidinecarboxylic acid (D37)

The title compound (D37) was prepared using an analogous process to that described in Descriptions 1 to 4 from (S)-3-pyrrolidinol (Lancaster 19498). LCMS electrospray (-ve) 214 (M-H). ¹H NMR (DMSO-d6) δ 1.39 (9H, s), 1.91-2.05 (2H, m), 2.99-3.06 (1H, m), 3.22-3.41 (4H, m), 12.48 (1H, bs).

### Description 38

### 1-Cyclobutyl-4-[(3R)-3-pyrrolidinylcarbonyl]hexahydro-1H-1,4-diazepine

The title compound (D38) was prepared using an analogous process to that described in Descriptions 5 and 6 from (3*R*)-1-(*tert*-butoxycarbonyl)-3-pyrrolidinecarboxylic acid (may be prepared as described in Description 37) and 1-cyclobutylhexahydro-1*H*-1,4-diazepine dihydrochloride (may be prepared as described in Description 25). LCMS electrospray (+ve) 252 (MH⁺).

### Description 39

### (3S)-1,1-Dimethylethyl 3-{[4-(1-methylethyl)hexahydro-1H-1,4-diazepin-1-yl]carbonyl}-1-pyrrolidinecarboxylate (D39)

### Method A

EDC (4.5g), HOBT (1.6g) and 1-(1-methylethyl)hexahydro-1H-1,4-diazepine dihydrochloride (may be prepared as described in Description 34) (2.5g) were added sequentially to a stirred solution of (3*S*)-1-(tert-butoxycarbonyl)-3-pyrrolidine carboxylic acid (may be prepared as described in Description 4) (2.5g) in DMF (25ml). Diisopropylethylamine (4.45ml) was added and the reaction mixture stirred at room temperature under argon for 2h. The DMF was removed by evaporation and the residue partitioned between EtOAc/saturated sodium hydrogen carbonate solution (100:50ml). The layers were separated and the aqueous layer extracted with EtOAc (100ml). The combined organic layers were dried (Na₂SO₄), evaporated and the residue purified by flash chromatography [silica gel, 0-10% MeOH (containing 10% 0.88 ammonia solution)/DCM]. The clean fractions were evaporated to afford the title compound (D39) as a pale yellow oil (2.2g). LCMS electrospray (+ve) 362 (MNa+).

### Method B

1-(1-Methylethyl)hexahydro-1H-1,4-diazepine dihydrochloride (may be prepared as described in Description 34) (15g) and diisopropylethylamine (26.8ml) were added sequentially to a mixture of (3S)-1-(tert-butoxycarbonyl)-3-pyrrolidine carboxylic acid (may be prepared as described in Description 4) (15g), EDC (26.7g) and HOBT (9.4g) in DMF (150ml) and the reaction stirred for 16h. The DMF was evaporated and the residue partitioned between EtOAc/saturated sodium hydrogen carbonate solution (500:150ml). The layers were separated and the aqueous layer extracted with EtOAc (4 x 350ml). The combined organic layers were dried (MgSO₄) and evaporated to afford the title compound (D39) as an orange oil (21.5g). LCMS electrospray (+ve) 362 (MNa+).

### Description 40

### 1-(1-Methylethyl)-4-[(3S)-3-pyrrolidinylcarbonyl]hexahydro-1H-1,4-diazepine (D40)

### Method A

4M HCl in dioxane (10ml) was added to a solution of (3S)-1,1-dimethylethyl 3-{[4-(1-methylethyl)hexahydro-1H-1,4-diazepin-1-yl]carbonyl}-1-pyrrolidinecarboxylate (may be prepared as described in Description 39) (2.2g) in MeOH (10ml) and the mixture stirred for 2h under an argon atmosphere. The solvent was evaporated and the residue dissolved in MeOH (30ml) and divided into 3 aliquots. Each aliquot was loaded onto a 10g SCX cartridge which was eluted with MeOH (50ml) followed by 2M NH₃/MeOH (50ml). The ammoniacal fractions were evaporated to afford the title compound (D40) as a pale orange oil (1.33g). LCMS electrospray (+ve) 240 (MH⁺).

### Method B

4M HCl in dioxane (60ml) was added to a solution of (3S)-1,1-dimethylethyl 3-{[4-(1-methylethyl)hexahydro-1H-1,4-diazepin-1-yl]carbonyl}-1-pyrrolidinecarboxylate (may be prepared as described in Description 39) (21.5g) in MeOH (60ml) and the mixture stirred for 1 h under an argon atmosphere. The solvent was evaporated and the residue partitioned between DCM/saturated potassium carbonate solution (400:100ml). The layers were separated and the aqueous layer extracted with DCM (5 x 200ml). The combined organic layers were dried (MgSO₄) and evaporated to afford the title compound (D40) as an orange oil (9.97g). LCMS electrospray (+ve) 240 (MH⁺).

### Description 41

### 1-(1-Methylethyl)-4-[(3R)-3-pyrrolidinylcarbonyl]piperazine (D41)

The title compound was prepared from (3*R*)-1-(*tert*-butoxycarbonyl)-3-pyrrolidinecarboxylic acid (may be prepared as described in Description 37) and 1-(1-methylethyl)piperazine (obtainable from Chess 1214) using an analogous process to that described in Descriptions 5 and 6 LCMS electrospray (+ve) 226 (MH+)¹H NMR (CDCl₃) δ 1.05 (6H,d, J=6.4 Hz), 1.92-2.03 (3H, m), 2.47-2.52 (4H, m), 2.67-2.93 (3H, m), 3.04-3.13 (2H, m), 3.18-3.22 (1H, m), 3.52-3.55 (2H, m), 3.61-3.64 (2H, m).

### Description 42

### 1-Cyclobutyl-4-[(3R)-3-pyrrolidinylcarbonyl]piperazine (D42)

The title compound was prepared from (3*R*)-1-(*tert*-butoxycarbonyl)-3-pyrrolidinecarboxylic acid (may be prepared as described in Description 37) and 1-cyclobutylpiperazine dihydrochloride (may be prepared as described in Description 8) using an analogous process to that described in Descriptions 9 and 10. LCMS electrospray (+ve) 238 (MH⁺). ¹H NMR (CDCl₃) δ 1.61-2.10 (9H, m), 2.27-2.32 (4H, m), 2.68-3.20 (6H, m), 3.52-3.55 (2H, m), 3.62-3.65 (2H, m).

### Description 43

### 4-(4-Bromophenyl)-2-methyl-1,3-oxazole (D43)

### Method A

4-Bromophenacyl bromide (21.3g) and acetamide (11.31g) were heated together in an oil bath at 130°C under argon. After 2.5h the reaction mixture was allowed to cool, and partitioned between water and diethyl ether. The organic phase was washed with aqueous NaOH (0.5M), aqueous HCl (0.5M) and brine (100ml), dried (Na₂SO₄) and evaporated to give a brown solid which was recrystallised from hexanes and dried in a vacuum oven overnight at 60°C to give the title compound (D43) as an orange solid (4.1g). LCMS electrospray (+ve) 238 and 240 (MH⁺).

### Method B

A solution of 4-Bromophenacyl bromide (12.7g) and acetonitrile (6.8g) in NMP (40 ml) was heated at reflux for 2.5 h. The reaction was cooled to room temperature, diluted with 300 ml ether, washed with 1 M NaOH (50 ml), 2M HCl (50 ml) and brine (50 ml), dried (MgSO₄) and evaporated. The residue was recrystallised from hexane to afford an orange solid which was further purified by silica chromatography (FM(II) system, 100g), eluting with a gradient of 0-35% ethyl acetate in pentane. The clean fractions were evaporated to afford the title compound (D43) as a pale yellow solid (4.1g). LCMS electrospray (+ve) 238 and 240 (MH+).

### Description 44

### 4-Bromo-N-hydroxy-benzenecarboximidamide (D44)

4-Bromophenylcarbonitrile (10.2g), hydroxylamine hydrochloride (7.8g) and triethylamine (11.3g) were dissolved in EtOH (250ml) and the reaction mixture was heated at reflux for 3h, after which it was evaporated to form a white precipitate of the desired amidoxime, which was filtered and washed with water (25ml). The filtrate was extracted into EtOAc (2x25ml), and the combined organic extracts were dried (Na₂SO₄) and evaporated to give a second crop of the title compound (D44) (combined yield = 11.1g). LCMS electrospray (+ve) 215 and 217 (MH⁺).

### Description 45

### 3-(4-Bromophenyl)-5-methyl-1,2,4-oxadiazole (D45)

4-Bromo-N-hydroxy-benzenecarboximidamide (D44) was suspended in acetic anhydride and heated to 100°C for 4h, then 120°C for 3h. After cooling the reaction mixture was evaporated to give a brown solid. This was partitioned between saturated aqueous sodium hydrogen carbonate and EtOAc. The organic phase was washed with saturated brine, dried (Na₂SO₄) and evaporated to give a yellow solid. The crude product was purified by column chromatography (silica gel, 10-100% gradient of EtOAc in hexanes) to give the title compound (D45) as a white solid (6.2g). LCMS electrospray (+ve) 239 and 241 (MH⁺).

### Description 46

### 5-(4-Bromophenyl)-2-methyl-1,3-oxazole (D46)

Trifluoromethanesulfonic acid (6.6ml) was added to a flask containing iodobenzene diacetate (12.2g) and MeCN (200ml) at rt. After 25min a solution of 4'-bromoacetophenone (5g) in MeCN (50ml) was added and the resultant mixture heated at reflux for 6h. The reaction was allowed to cool to rt before the solvent was evaporated and the residue partitioned between saturated aqueous sodium hydrogen carbonate (150ml) and EtOAc (150ml). The organic phase was washed with saturated brine (150ml), dried (MgSO₄) and evaporated to give an orange solid. The crude product was purified by column chromatography (silica gel, 50% EtOAc in hexanes) to give the title compound (D46) as a pale yellow solid (3.5g). LCMS electrospray (+ve) 238 and 240 (MH⁺).

### Description 47

### 5-Bromo-2-(trifluoromethyl)pyrimidine (D47)

A mixture of potassium fluoride (1.77g) and cuprous iodide (5.79g) was stirred and heated together using a heat gun under vacuum (∼1 mm) for 20min. After cooling, dimethylformamide (20ml) and N-methyl pyrrolidinone (20ml) were added followed by (trifluoromethyl)trimethylsilane (4.1ml) and 5-bromo-2-iodopyrimidine (6.5g). The mixture was stirred at rt for 5h and then the brown solution was poured into 6N ammonia solution. The product was extracted into ethyl acetate and the extracts were washed with saturated aqueous sodium hydrogen carbonate solution and brine and then dried (Na₂SO₄) and evaporated. Chromatography on silica gel (elution with 20-50% dichloromethane in pentane) gave the title compound (D47) as a white solid (2.4g). ¹H NMR (CDCl₃): 8.97 (2H, s).

### Description 48

### 5-(3-Bromophenyl)-3-methyl-1,2,4-oxadiazole (D48)

N-Hydroxyethanimidamide (2.58g) was added to a suspension of NaH (60% dispersion in mineral oil, 1.44g) in THF (100ml) and the mixture stirred for 10 min at room temperature followed by 50 min at 50°C. Methyl 3-bromobenzoate (5g) in THF (100ml) was added and the mixture heated to reflux for 1 h. The reaction was allowed to cool, poured into water (100ml) and extracted with EtOAc (2 x 1 00ml). The combined extracts were washed with water (2 x 20ml) and brine (20ml), dried (Na₂SO₄) and evaporated. The solid residue was recrystallised from hexane to afford the title compound (D48) as white needles (3.08g).LCMS electrospray (+ve) 239, 241 (MH⁺) ¹H NMR (CDCl₃) δ 2.47 (3H,s), 7.41 (1H, t, J=7.6Hz), 7.72 (1H, m), 8.05 (1H, m) and 8.28 (1H, t, J=1.6Hz).

### Description 49

### 5-(4-Bromophenyl)-3-ethyl-1,2,4-oxadiazole (D49)

Propionitrile (7.7g) and hydroxylamine (50% aq., 4.3ml) were heated to reflux in EtOH (10ml) for 18.5h The reaction mixture was allowed to cool to room temperature before extracting into DCM (25ml). The organic phase was washed with saturated brine (50ml), water (25ml), dried (MgSO₄) and evaporated. Analysis of the crude mixture indicated approximately 30% conversion. More hydroxylamine (10ml) and EtOH (5ml) were added and the reaction mixture was heated for a further 7h at 90°C. After cooling and extraction into DCM (25ml) as before, the organic phase was again washed with saturated brine (50ml), water (25ml), dried (MgSO₄) and evaporated to give the crude amidoxime as an oil. This was combined with 4-bromobenzoic acid (2.2g), EDC (2.2g) and HOBt (1.47g) in DMF (25ml), and Hunig's base (7ml) was added. The reaction mixture was stirred for 4h. The reaction mixture was then divided into two aliquots which were heated in the microwave reactor at 180°C for 20 min. each. The two crude mixtures were combined and the DMF evaporated. The crude oxadiazole was purified by flash chromatography [silica gel, 0-20% EtOAc/pentane]. The clean fractions were evaporated to afford the title compound (D49) as a yellow solid (1.5g). LCMS electrospray (+ve) 253/255 (MH⁺). ¹H NMR (CDCl₃) δ 1.39 (3H, t, J=7.6Hz), 2.83 (2H, q, J=7.6Hz), 7.65 (2H, d, J=8.0Hz) and 7.97 (2H, d, J=8.0Hz).

### Description 50

### 5-Bromo-2-pyridinecarboxylic acid (D50)

5-Bromo-2-pyridinecarbonitrile (15g) was heated at reflux in concentrated hydrochloric acid (200ml) for 6 hours. On cooling in an ice bath the product crystallised out as white needles which were filtered and washed with iced water and dried in an oven under vacuum to give the title compound (D50) (11g). LCMS electrospray (+ve) 202 and 204 (MH+).

### Description 51

### 5-Bromo-2-(3-methyl-1,2,4-oxadiazol-5-yl)pyridine (D51)

5-Bromo-2-pyridinecarboxylic acid (may be prepared as described in Description 50; 4.5g) and carbonyldiimidazole (3.97g) in THF (40ml) were heated at reflux temperature for 90 minutes followed by the addition of acetamide oxime (4.95g). The reaction mixture was allowed to continue refluxing overnight. After cooling to room temperature the reaction mixture was diluted with EtOAc and washed with water (2x), 2M NaOH (2x), water (2x) followed by brine (2x). The organic layer was dried (MgSO₄) and concentrated to give a pale yellow solid which was recrystallised from a mixture of hot ethanol and methanol to give the title compound (D51) as colourless crystals (4.4g). LCMS electrospray (+ve) 240 and 242 (MH+).

### Description 52

### N-[1-(Dimethylamino)ethylidene]-2,4-difluorobenzamide (D52)

2,4-Difluorobenzamide(19.98g) in N,N-dimethylacetamide dimethylacetal (82ml) was heated at 120°C for 2 hours and allowed to cool overnight. The crude reaction mixture was diluted with water (500ml) and extracted with EtOAc (2x200ml). The EtOAc layer was dried (MgSO₄), filtered and evaporated (stripped) to give an oil. The residue was absorbed onto silica (100g) and purified by silica chromatography (on a size E column), eluting with a gradient of from 10-100% EtOAc in hexane. The pure fractions were combined and stripped to give the title compound (D52)(24g). LCMS (+ve) electrospray 227 (MH+).

### Description 53

### 5-(2,4-Difluorophenyl)-3-methyl-1,2,4-oxadiazole (D53)

To a stirred mixture of hydroxylamine hydrochloride (9.9g) and 1 N NaOH (140ml) was added *N*-[1-(dimethylamino)ethylidene]-2,4-difluorobenzamide (may be prepared as described in Description 52; 23g), acetic acid (170ml) and dioxane (140ml) sequentially.

The mixture was stirred for 20 minutes at room temperature and was then heated at reflux for 3 hours. After cooling the crude reaction mixture was poured into water (1litre) and adjusted to pH9 with NaHCO₃. The mixture was filtered and the residue was diluted with EtOAc (1 litre). The EtOAc layer was filtered and the filtrate was evaporated to dryness to give the title compound as a light brown solid (11.2g). LCMS electrospray (+ve) 197 (MH+).

### Description 54

### 4-(4-MethyHmidazol-1-yl)iodobenzene and 4-(4-methylimidazol-1-yl)bromobenzene (D54)

A mixture of 4-methylimidazole (1.642g), 4-bromoiodobenzene (5.658g), caesium carbonate (13.70g), cuprous iodide (0.192g) and *trans*-N,N-dimethyl-1,2-cyclohexane diamine (0.579g) in DMF (10 ml) was added to a Buchi miniclave (25 ml) according to the method of Buchwald et al. (Journal of Organic Chemistry, 2004, 69: 5578). The reactants were added under a gentle stream of argon. The tube was then sealed and the mixture was heated at 110°C for 18-20h. After cooling, the reaction mixture was poured into water and extracted (3x) with ethyl acetate. The combined extracts were washed with water (2x) and brine, dried (MgSO₄) and evaporated to give a light brown semi-solid. This was filtered through SiO₂, eluting with 0-80-100% EtOAc in hexane. The resulting light brown solid was recrystallised from hexane/toluene. The product, which was obtained as light yellow needles (2.086g), consisted of a mixture of 4-(4-methylimidazol-1-yl)iodobenzene and 4-(4-methylimidazol-1-yl)bromobenzene (D54) as evidenced by the spectroscopic data. ¹H NMR δ (CDCl₃): 2.30 (3H, s), 6.97 (1H, s), 7.12 (2H, d, J = 8.8Hz, iodo compound), 7.24 (2H, d, J = 8.4Hz, bromo compound), 7.59 (2H, d, J = 8.8Hz, bromo compound), 7.73 (1H, s), 7.78 (2H, d, J = 8.8Hz, iodo compound); LCMS electrospray (+ve) (MH⁺) 237/239 (Rₜ = 1.47 min., 34%, bromo compound), 285 (Rt = 1.57 min., 66%, iodo compound)

### Description 55

### 5-(4-Fluorophenyl)-3-methyl-1,2,4-oxadiazole (D55)

To an ice-cooled, stirred solution of acetamidoxime (28.0g; only partially soluble initially) and triethylamine (42.1g) in THF (950mL) under an atmosphere of argon, was added a solution of 4-fluorobenzoyl chloride (60.0g) in THF (350ml), dropwise, over 56min. The cooling bath was then removed and the resulting slurry was stirred for 57min. DBU (115.1g) was added fairly rapidly from an addition funnel, which was then washed through with THF (50ml). The stirred mixture was heated at 63°C for 16h and then allowed to cool to room temperature. The solid precipitate was filtered off, washed on the filter with THF and discarded. The combined filtrate was concentrated *in vacuo* and the resulting material was partitioned between 2M HCl (1.01) and Et₂O (0.51). The layers were separated and the aqueous acidic layer was re-extracted twice with diethyl ether (0.5l). The combined ethereal extracts were washed sequentially with water (0.5l), sat. NaHCO₃, water (0.51) and brine; dried (MgSO₄) and evaporated to give a white solid (51.7g). This was combined with another batch prepared similarly from identical quantities of the starting materials (crude weight 56g) and the combined batches were recrystallised from a 50:50 mixture of water/glacial acetic acid (700ml), affording the title product (D55) as white needles which were collected by filtration, washed on the filter with 50:50 water/glacial acetic acid (220ml) and dried at 39°C *in vacuo,* over KOH pellets for 48h to give the dry product (D55) (81.65g) ¹H NMR (CDCl₃) 2.47 (3H, s), 7.22 (2H, m), 8.13 (2H, m); LCMS electrospray (+ve) 179 (MH⁺).

### Description 56

### 1,1-Dimethylethyl (3S)-3-[(4-ethyl-1-piperazinyl)carbonyl]-1-pyrrolidinecarboxylate (D56)

EDC (0.696g), HOBT (0.23g), 1-ethylpiperazine (0.2g) and triethylamine (0.25ml) were added sequentially to a stirred solution of (3*S*)-1-(*tert*-butoxycarbonyl-3-pyrrolidinecarboxylic acid (0.38g) (may be prepared as described in Description 4) in DMF (20ml) and the reaction mixture was stirred at room temperature overnight. The reaction mixture was diluted with water (30ml) and extracted with EtOAc (2x30ml). The combined EtOAc layers were washed with water (8x30ml) and then dried (MgSO4) and evaporated to give the title compound (D56) as an oil (0.11g).

### Description 57

### 1-Ethyl-4-[(3S)-3-pyrrolidinylcarbonyl]piperazine (D57)

4N HCl in dioxane (3ml) was added to a solution of 1,1-dimethylethyl (3S)-3-[(4-ethyl-1-piperazinyl)carbonyl]-1-pyrrolidinecarboxylate (may be prepared as described in D56) (0.11g) in MeOH (3ml). The reaction mixture was left standing at room temperature overnight. The solvent was removed to give an oil which was basified in water (3ml) and solid potassium carbonate. The oil obtained was then extracted into DCM (3x15ml). The combined organic extracts were dried (MgSO₄) and concentrated to give the title compound (D57) as an oil (52mg). ¹H NMR (methanol-d4) δ:1.33-1.55 (3H, t), 2.0-2.49 (2H, m), 2.97-3.27 (4H, m), 3.3-3.45 (4H, m), 3.54-3.78 (5H, m), 4.25-4.37 (1H, m), 4.65-4.75 (1H, m)

### Example 1

### 1-(1-Methylethyl)-4-({(3S)-1-[6-(trifluoromethyl)-3-pyridinyl]-3-pyrrolidinyl} carbonyl)piperazine (E1)

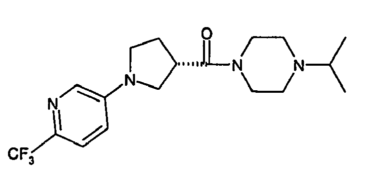

5-Bromo-2-(trifluoromethyl)pyridine (may be prepared as described in Cottet and Schlosser, Eur. J. Org. Chem., 2002, 327) (0.242g) was added to a stirred solution of tris(dibenzylideneacetone) dipalladium(0) (0.055g) and 2-dicyclohexylphosphino-2'-(N,N dimethylamino)-biphenyl (0.07g) in DME under argon at room temperature. Potassium phosphate (0.377g) and 1-(1-methylethyl)-4-[(3S)-3-pyrrolidinylcarbonyl]piperazine (may be prepared as described in Description 6) (0.2g in DME) were added sequentially to bring the total volume of DME to 5 ml and the mixture heated to 75°C for 4h under argon. The mixture was then diluted with MeOH, loaded onto a 10g SCX cartridge and eluted with MeOH followed by 2M NH₃/MeOH. The ammoniacal fractions were collected and evaporated and the residue purified on a silica chromatography column eluting with 0-10% MeOH (containing 10% 0.88 ammonia solution)/DCM. The pure fractions were crystallised from EtOAc to afford the title compound (E1) as pale yellow crystals (0.109g). LCMS electrospray (+ve) 371 (MH⁺). ¹H NMR (CDCl₃) δ 1.05 (6H, d, J=6.8Hz), 2.23-2.37 (2H, m), 2.50-2.57 (4H, m), 2.71-2.78 (1H, m), 3.38-3.68 (9H, m), 6.82 (1H, dd, J=8.8, 2.8Hz), 7.47 (1H, d, J=8.8Hz), 8.01 (1H, d, J=2.8Hz).

### Example 2

### 1-[4-((3S)-3-{[4-(1-Methylethyl)-1-piperazinyl]carbonyl}-1-pyrrolidinyl) phenyl]ethanone (E2)

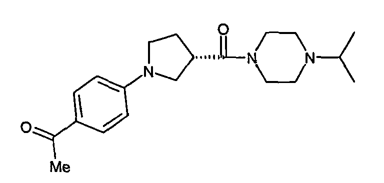

4-Bromoacetophenone (0.213g) was added to a stirred solution of tris(dibenzylideneacetone) dipalladium(0) (0.055g) and 2-dicyclohexylphosphino-2'-(N,N-dimethylamino)-biphenyl (0.07g) in DME under argon at room temperature. Potassium phosphate (0.377g) and 1-(1-methylethyl)-4-[(3S)-3-pyrrolidinylcarbonyl]piperazine (may be prepared as described in Description 6) (0.2g in DME) were added sequentially to bring the total volume of DME to 5 ml and the mixture heated to 75°C for 4h under argon. The mixture was then diluted with MeOH, loaded onto a 10g SCX cartridge and eluted with MeOH followed by 2M NH₃/MeOH. The ammoniacal fractions were collected and evaporated and the residue purified on a silica chromatography column (FM(II) system) eluting with 0-10% MeOH (containing 10% 0.88 ammonia solution)/DCM.. The residue was triturated with diethyl ether and crystallised from ethyl acetate to afford the title compound (E2) as pale yellow crystals (0.109g). LCMS electrospray (+ve) 344 (MH⁺). ¹H NMR (CDCl₃) δ 1.05 (6H, d, J=6.8Hz), 2.21-2.38 (2H, m), 2.49 (3H, s), 2.50-2.57 (4H, m), 2.70-2.76 (1H, m), 3.37-3.68 (9H, m), 6.52 (2H, d, J=8.8Hz), 7.87 (2H, d, J=8.8Hz).

### Example 3

### 1-(1-Methylethyl)-4-({(3S)-1-[4-(3-methyl-1,2,4-oxadiazol-5-yl)phenyl]-3-pyrrolidinyl}carbonyl)piperazine (E3)

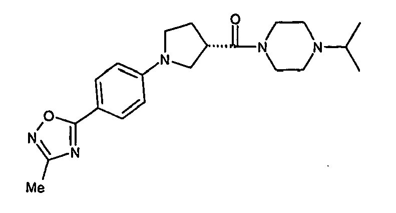

5-(4-Bromophenyl)-3-methyl-1,2,4-oxadiazole (may be prepared as described in Description 23) (0.255g) was added to a stirred solution of tris(dibenzylideneacetone)dipalladium(0) (0.055g) and 2-dicyclohexylphosphino-2'-(*N,N-*dimethylamino)-biphenyl (0.07g) in DME (4ml) under argon at room temperature. Potassium phosphate (0.377g) and 1-(1-methylethyl)-4-[(3S)-3-pyrrolidinylcarbonyl]piperazine (may be prepared as described in Description 6) (0.2g in 1ml DME) were added sequentially and the mixture heated to 75°C for 4h. The mixture was then diluted with MeOH, loaded onto a 10g SCX cartridge and eluted with MeOH (70ml) followed by 2M NH₃/MeOH (70ml). The ammoniacal fractions were collected and evaporated and the residue purified by flash chromatography [silica gel, 0-10% MeOH (containing 10% 0.88 ammonia solution)/DCM]. The pure fractions were evaporated and the residue triturated with diethyl ether to afford the title compound (E3) as a white solid (0.085g). LCMS electrospray (+ve) 384 (MH⁺). ¹H NMR (CDCl₃) δ 1.06 (6H, d, J=6.8Hz), 2.21-2.39 (2H, m), 2.42 (3H, s), 2.50-2.56 (4H, m), 2.70-2.77 (1H, m), 3.38-3.68 (9H, m), 6.59 (2H, d, J=8.8Hz), 7.95 (2H, d, J=8.8Hz).

### Example 3A

### 1-(1-Methylethyl)-4-({(3S)-1-[4-(3-methyl-1,2,4-oxadiazol-5-yl)phenyl]-3-pyrrolidinyl}carbonyl)piperazine hydrochloride (E3A)

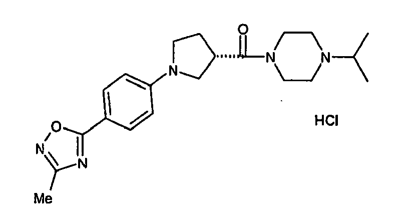

5-(4-Bromophenyl)-3-methyl-1,2,4-oxadiazole (may be prepared as described in Description 23) (1.55g) was added to a stirred solution of tris(dibenzylideneacetone)dipalladium(0) (0.124g) and 2-dicyclohexylphosphino-2'-(*N,N-*dimethylamino)-biphenyl (0.16g) in DME (10ml) under argon at room temperature. Potassium phosphate (2.3g) and 1-(1-methylethyl)-4-[(3*S*)-3-pyrrolidinylcarbonyl]piperazine (may be prepared as described in Description 6) (1.22g in 10ml DME) were added sequentially and the mixture heated to 75°C. Further portions of tris(dibenzylideneacetone)dipalladium(0) (0.124g) and 2-dicyclohexylphosphino-2'-(N,N-dimethylamino)-biphenyl (0.16g) were added to the reaction after both 1.5h and 4.5h of heating. After 16h the mixture was allowed to cool, filtered through a pad of celite and the pad washed with MeOH. The solvent was evaporated and the residue purified by flash chromatography [silica gel, gradient elution: DCM/0-5% 2M ammonia in MeOH]. The pure fractions were evaporated and the residue triturated with diethyl ether. The free base was then dissolved in a minimum volume of EtOAc and 1 M HCl/diethyl ether (4ml) added. The solvents were removed and the residue re-evaporated from acetone (x 3). The residue was then dissolved in a minimum volume of hot ethanol and the hot solution rapidly filtered. The product was allowed to crystallise from solution, filtered off and washed with cold ethanol. The product was then re-crystallised from hot ethanol following the same procedure to afford the title compound (E3A) as a colourless, crystalline solid. LCMS electrospray (+ve) 384 (MH⁺). ¹H NMR (DMSO-d6) δ 1.29 (6H, d, J=6.4Hz), 2.10-2.25 (2H, m), 2.34 (3H, s), 2.93 (1H, m), 3.05-3.16 (2H, m), 3.33-3.67 (9H, m), 4.25 (1H, m), 4.52 (1H, m), 6.70 (2H, d, J=8.8Hz), 7.86 (2H, d, J=8.8Hz) and 10.75 (1H, bs). Enantiomeric excess = 99.8% (Chiralcel OJ (250 x 4.6 mm, 10 micron particle size); Heptane:Ethanol 50:50 v/v; isocratic for 25 min at 1 ml/min). Retention time of major component = 19.4 min, minor component = 12.0 min.

### Example 4

### (2S)-2-Methyt-1-(1-methytethyl)-4-({(3S)-1-[6-(trifluoromethyl)-3-pyridinyl]-3-pyrrolidinyl}carbonyl)piperazine (E4)

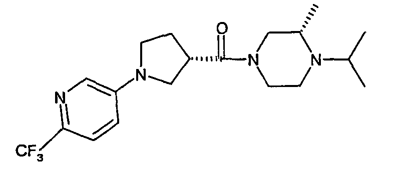

5-Bromo-2-(trifluoromethyl)pyridine (may be prepared as described in Cottet and Schlosser, Eur. J. Org. Chem., 2002, 327) (0.171g) was added to a stirred solution of tris(dibenzylideneacetone) dipalladium(0) (0.055g) and 2-dicyclohexylphosphino-2'-(N,N-dimethylamino)-biphenyl (0.07g) in DME under argon at room temperature. Potassium phosphate (0.267g) and (2S)-1-(1-methylethyl)-2-methyl-4-[(3S)-3-pyrrolidinylcarbonyl]piperazine (may be prepared as described in Description 21) (0.15g in DME) were added sequentially to bring the total volume of DME to 4 ml and the mixture heated to 75°C for 4h under argon. The mixture was then diluted with MeOH, loaded onto a 10g SCX cartridge and eluted with MeOH followed by 2M NH₃/MeOH. The ammoniacal fractions were collected and evaporated and the residue purified on silica chromatography column (FM(II) system) eluting with 0-10% MeOH (containing 10% 0.88 ammonia solution)/DCM]. The residue was triturated with ether and dried to afford the title compound (E4) as a white solid (0.035g). LCMS electrospray (+ve) 385 (MH⁺). ¹H NMR (CDCl₃) δ 0.89 (3H, d, J=6.8Hz), 1.08 (3H, dd, J=14.4, 6.4Hz), 1.13 (3H, d, J=6.8Hz), 2.22-2.36 (3H, m), 2.54-3.08 (3H, m), 3.20-3.81 (8H, m), 4.21-4.31 (1H, m), 6.82 (1H, dd, J=8.8, 2.4Hz), 7.47 (1H, d, J=8.8Hz), 8.02 (1H, d, J=2.4Hz).

### Example 5

### 1-[4-((3S)-3-{[(3S)-3-Methyl-4-(1-methylethyl)-1-piperazinyl]carbonyl}-1-pyrrolidinyl)phenyl]ethanone (E5)

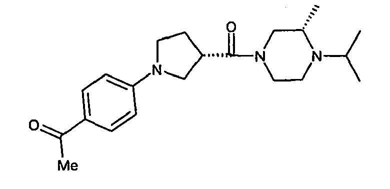

### Method A

4-Bromoacetophenone (0.151 g) was added to a stirred solution of tris(dibenzylideneacetone) dipalladium(0) (0.055g) and 2-dicyclohexylphosphino-2'-(N,N-dimethylamino)-biphenyl (0.07g) in DME under argon at room temperature. Potassium phosphate (0.267g) and (2*S*)-1-(1-methylethyl)-2-methyl-4-[(3*S*)-3-pyrrolidinylcarbonyl]piperazine (may be prepared as described in Description 21) (0.15g in DME) were added sequentially to bring the total volume of DME to 4 ml and the mixture heated to 75°C for 4h under argon. The mixture was then diluted with MeOH, loaded onto a 10g SCX cartridge and eluted with MeOH followed by 2M NH₃/MeOH. The ammoniacal fractions were collected and evaporated and the residue purified on a silica chromatography column (FM(II) system) eluting with 0-10% MeOH (containing 10% 0.88 ammonia solution)/DCM.. The pure fractions were triturated with ether and dried to afford the title compound (E5) as a pale yellow solid (0.055g). LCMS electrospray (+ve) 358 (MH⁺). ¹H NMR (CDCl₃) δ 0.89 (3H, dd, J=6.4, 3.2Hz), 1.08 (3H, dd, J=14.0, 6.0Hz), 1.12 (3H, d, J=6.8Hz), 2.21-2.38 (3H, m), 2.50 (3H, s), 2.52-3.08 (3H, m), 3.21-3.81 (8H, m), 4.22-4.31 (1H, m), 6.52 (2H, d, J=8.0Hz), 7.86 (2H, d, J=8.0Hz).

### Method B

4-Bromoacetophenone (0.167g) was dissolved in DME (2ml).
Tris(dibenzylideneacetone) dipalladium(0) (0.055g) and 2-dicyclohexylphosphino-2'-(N,N-dimethylamino)-biphenyl (0.072g) were added and the mixture was stirred briefly before addition of (2*S*)-1-(1-methylethyl)-2-methyl-4-[(3*S*)-3-pyrrolidinylcarbonyl]piperazine (may be prepared as described in Description 21) (0.2g) in DME (2ml). Potassium phosphate (0.365g) was then added and the reaction was heated to 80°C for 6h under argon. After cooling, the reaction mixture was filtered and diluted with MeOH (20ml). The solution was loaded onto a 10g SCX cartridge and eluted with MeOH (50ml) followed by 2M NH₃/MeOH (50ml). The ammoniacal fractions were collected and stripped. The residue was divided into two portions and purified on two 12M silica columns. The columns were run on Biotage SP1 eluting with a gradient of 0-50% MeOH in ethyl acetate over 20 column volumes. The pure fractions were combined and evaporated. The resulting solid was dissolved in MeOH (4ml) and treated with ethereal HCl (2ml). The solvent was blown down to give the title product (E5) (0.018g) as a light brown solid. LCMS electrospray (+ve) 358 (MH⁺). ¹H NMR (CDCl₃) δ 0.89 (3H, dd, J=6.4, 3.2Hz), 1.08 (3H, dd, J=14.0, 6.0Hz), 1.12 (3H, d, J=6.8Hz), 2.21-2.38 (3H, m), 2.50 (3H, s), 2.52-3.08 (3H, m), 3.21-3.81 (8H, m), 4.22-4.31 (1H, m), 6.52 (2H, d, J=8.0Hz), 7.86 (2H, d, J=8.0Hz).

### Example 5A

### 1-[4-((3S)-3-{[(3S)-3-Methyl-4-(1-methylethyl)-1-piperazinyl]carbonyl}-1-pyrrolidinyl)phenyl]ethanone hydrochloride (E5A)

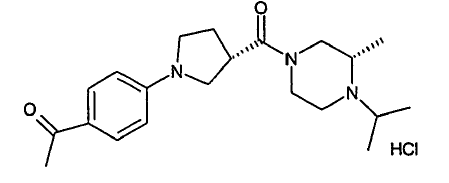

Tris(dibenzylideneacetone)dipalladium (0) (0.055g) and 2-dicyclohexylphosphino-2'-(N,N-dimethylamino)-biphenyl (0.072g) were added to a stirred solution of 4-bromoacetophenone (0.167g) in DME (2ml) under argon followed by the sequential addition of (2*S*)-1(1-methylethyl)-2-methyl-4-[(3*S*)-3-pyrrolidinylcarbonyl]piperazine (may be prepared as described in Description 21) (0.2g) in DME (2ml) and potassium phosphate (0.365g). The reaction mixture was heated to 80°C for 6h. After cooling, the mixture was diluted with MeOH, loaded onto a 10g SCX cartridge and eluted with MeOH (50ml) followed by 2M NH₃/MeOH (50ml). The ammoniacal fractions were collected and evaporated to give a crude product (0.295g) which was purified by chromatography (silica-gel; 0-50% MeOH/ EtOAc). The pure fractions were collected and evaporated to give the free base which was dissolved in dry MeOH (4ml) and treated with 1 N ethereal HCl (2ml). Evaporation of solvents gave the title compound (E5A) as a light brown solid (18mg). LCMS electrospray (+ve) 358 (MH⁺).¹H NMR (methanol-d4) δ: 1.25 (3H, m), 1.37-1.59 (6H, m), 2.25-2.5 (4H, m), 2.88-3.20 (3H, m), 3.45-3.71 (8H, m), 3.95-4.05 (1H, m), 4.33-4.43 (1H, m), 4.61-4.8 (1H, m), 6.58 (2H, d, J=8.0Hz), 7.88 (2H, d, J=8.0Hz).

### Example 6

### (2S)-2-Methyl-1-(1-methylethyl)-4-({(3S)-1-[4-(3-methyl-1,2,4-oxadiazol-5-yl)phenyl]-3-pyrrolidinyl}carbonyl)piperazine (E6)

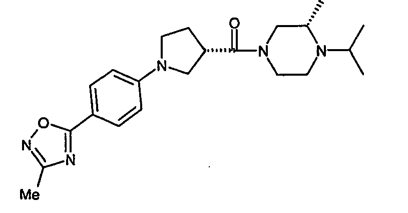

The title compound (E6) was prepared from 5-(4-bromophenyl)-3-methyl-1,2,4-oxadiazole (may be prepared as described in Description 23) and (2*S*)-1-(1-methylethyl)-2-methyl-4-[(3*S*)-3-pyrrolidinylcarbonyl] piperazine (may be prepared as described in Description 21) using an analogous process to that described in Example 5. LCMS electrospray (+ve) 398 (MH⁺). ¹H NMR (CDCl₃) δ 0.89 (3H, dd, J=6.4, 3.6Hz), 1.08 (3H, dd, J=15.2, 6.4Hz), 1.13 (3H, d, J=6.8Hz), 2.23-2.38 (3H, m), 2.43 (3H, s), 2.52-3.08 (3H, m), 3.21-3.81 (8H, m), 4.22-4.31 (1H, m), 6.59 (2H, d, J=8.4Hz), 7.94 (2H, d, J=8.4Hz).

### Example 6A

### (2S)-2-Methyl-1-(1-methylethyl)-4-({(3S)-1-[4-(3-methyl-1,2,4-oxadiazol-5-yl)phenyl]-3-pyrrolidinyl}carbonyl)piperazine hydrochloride (E6A)

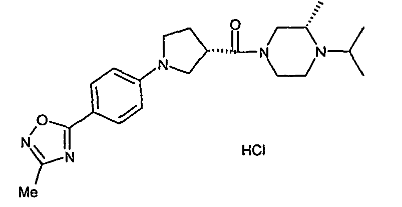

5-(4-Fluorophenyl)-3-methyl-1,2,4-oxadiazole (may be prepared as described in Description 55) (0.158g) and potassium carbonate (0.204g) were added to a mixture of (2S)-1-(1-methylethyl)-2-methyl-4-[(3S)-3-pyrrolidinylcarbonyl] piperazine (may be prepared as described in Description 21) (0.177g) in DMSO (4ml) and the mixture heated to 130°C under Argon for 2h. The mixture was allowed to cool, diluted with MeOH, loaded onto a 10g SCX cartridge and eluted with MeOH (50ml) followed by 2M NH₃/MeOH (50ml). The ammoniacal fractions were evaporated and the residue purified by mass directed auto-purification. The purified product was dissolved in a minimum volume of MeOH and converted to the hydrochloride salt by addition of 1 M HCl/Et₂O. The solvents were evaporated and the residue crystallised from a minimum volume of hot ethanol to afford the title compound (6A) as a white crystalline solid (0.04g). LCMS electrospray (+ve) 398 (MH⁴). ¹H NMR (DMSO-d6) δ: 1.15 (3H, d, J=6.0Hz), 1.35 (6H, bs), 2.06-2.32 (3H, m), 2.34 (3H, s), 2.85-3.15 (2H, m), 3.21-3.71 (7H, m), 3.85 (1H, m), 4.22 (1H, m), 4.50 (1H, m), 6.70 (2H, d, J=8.4Hz), 7.86 (2H, d, J=8.4Hz), 10.05 (1H. bs).

### Example 7

### 1-(1-Ethylpropyl)-4-({(3S)-1-[6-{trifluoromethyl)-3-pyridinyl]-3-pyrrolidinyl} carbonyl)piperazine (E7)

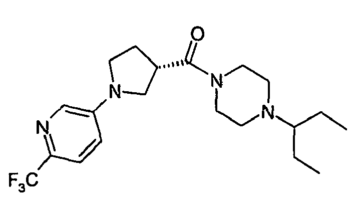

The title compound (E7) was prepared from 5-bromo-2-(trifluoromethyl)pyridine (may be prepared as described in Cottet and Schlosser, Eur. J. Org. Chem., 2002, 327) and 1-(1-ethylpropyl)-4-[(3*S*)-3-pyrrolidinylcarbonyl]piperazine (may be prepared as described in Description 12) using an analogous process to that described in Example 5. LCMS electrospray (+ve) 399 (MH⁺). ¹H NMR (CDCl₃) δ 0.91 (6H, t, J=7.6), 1.25-1.39 (2H, m), 1.40-1.51 (2H, m), 2.17-2.37 (3H, m), 2.50-2.56 (4H, m), 3.37-3.68 (9H, m), 6.82 (1H, dd, J=8.8, 2.8Hz), 7.46 (1H, d, J=8.8Hz), 8.01 (1H, d, J=2.8Hz).

### Example 8

### 1-[4-((3S)-3-{[4-(1-Ethylpropyl)-1-piperazinyl]carbonyl)-1-pyrrolidinyl) phenyl]ethanone (E8)

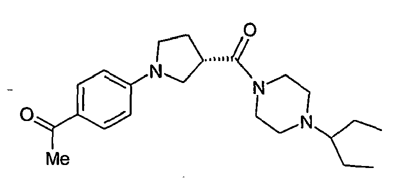

4-Bromoacetophone (0.213g) was added to a stirred solution of tris(dibenzylideneacetone) dipalladium(0) (0.055g) and 2-dicyclohexylphosphino-2'-(N,N-dimethylamino)-biphenyl (0.07g) in DME under argon at room temperature. Potassium phosphate (0.335g) and 1-(1-ethylpropyl)-4-[(3*S*)-3-pyrrolidinylcarbonyl]piperazine (may be prepared as described in Description 12) (0.2g in DME) were added sequentially to bring the total volume of DME to 5 ml and the mixture heated to 75°C for 4h under argon. The mixture was then diluted with MeOH, loaded onto a 10g SCX cartridge and eluted with MeOH followed by 2M NH₃/MeOH. The ammoniacal fractions were collected and evaporated and the residue purified on a silica chromatography column (FM(II) system) eluting with 0-10% MeOH (containing 10% 0.88 ammonia solution)/DCM.. The residue was precipitated from ethyl acetate to afford the title compound (E8) (0.08g) as a pale yellow powder. LCMS electrospray (+ve) 372 (MH⁺). ¹H NMR (CDCl₃) δ 0.91 (6H, t, J=7.6Hz), 1.28-1.35 (2H, m), 1.44-1.51 (2H, m), 2.19-2.37 (3H, m), 2.50 (3H, s), 2.51-2.57 (4H, m), 3.37-3.66 (9H, m), 6.51 (2H, d, J=9.2Hz), 7.86 (2H, d, J=9.2Hz).

### Example 9

### 1-(1-Ethytpropyl)-4-({(3S)-1-[4-(3-methyl-1,2,4-oxadiazol-5-yl)phenyl]-3-pyrrolidinyl}carbonyl)piperazine (E9)

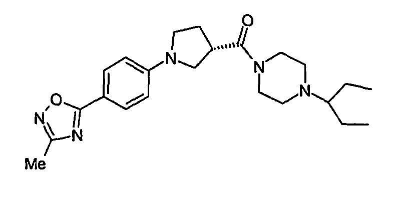

The title compound (E9) was prepared from 5-(4-bromophenyl)-3-methyl-1,2,4-oxadiazole (may be prepared as described in Description 23) and 1-(1-ethylpropyl)-4-[(3*S*)-3-pyrrolidinylcarbonyl]piperazine(may be prepared as described in Description 12) using an analogous process to that described in Example 5. LCMS electrospray (+ve) 412 (MH⁺). ¹H NMR (CDCl₃) δ 0.92 (6H, t, J=7.6Hz), 1.30-1.36 (2H, m), 1.41-1.49 (2H, m), 2.17-2.39 (3H, m), 2.42 (3H, s), 2.50-2.58 (4H, m), 3.38-3.66 (9H, m), 6.60 (2H, d, J=8.8Hz), 7.94 (2H, d, J=8.8Hz).

### Example 10

### 1-[4-((3S)-3-{[4-(Cyclopropylmethyl)hexahydro-1H-1,4-diazepin-1-yl]carbonyl}-1- pyrrolidinyl)phenyl]ethanone (E10)

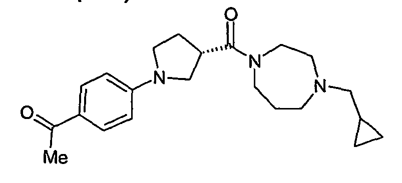

The title compound (E10) was prepared from 4-bromoacetophenone and 1-(cyclopropylmethyl)-4-[(3*S*)-pyrrolidin-3-ylcarbonyl]-hexahydro-1*H*-1,4-diazepine (may be prepared as described in Description 16) using an analogous process to that described in Example 5. LCMS electrospray (+ve) 370 (MH⁺). ¹H NMR (CDCl₃) δ 0.05-0.12 (2H, m), 0.46-0.52 (2H, m), 0.80-0.91 (1H, m), 1.88-2.00 (2H, m), 2.21-2.42 (4H, m), 2.50 (3H, s), 2.69-2.85 (4H, m), 3.37-3.46 (2H, m), 3.55-3.71 (7H, m), 6.51 (2H, d, J=8.8Hz), 7.86 (2H, d, J=8.8Hz).

### Example 11

### 1-(Cyctopropytmethyl)-4-({(3S)-1-[4-(3-methyl-1,2,4-oxadiazol-5-yl)phenyl]-3-pyrrolidinyl}carbonyl)hexahydro-1H-1,4-diazepine (E11)

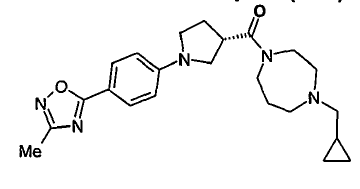

The title compound (E11) was prepared from 5-(4-bromophenyl)-3-methyl-1,2,4-oxadiazole (may be prepared as described in Description 23) and 1-(cyclopropylmethyl)-4-[(3*S*)-pyrrolidin-3-ylcarbonyl]-hexahydro-1*H*-1,4-diazepine (may be prepared as described in Description 16) using an analogous process to that described in Example 5. LCMS electrospray (+ve) 410 (MH⁺). ¹H NMR (CDCl₃) δ 0.07-0.12 (2H, m), 0.49-0.54 (2H, m), 0.82-0.91 (1H, m), 1.88-2.00 (2H, m), 2.22-2.42 (4H, m), 2.42 (3H, s), 2.70-2.86 (4H, m), 3.38-3.47 (2H, m), 3.56-3.72 (7H, m), 6.58 (2H, d, J=8.8Hz), 7.94 (2H, d, J=8.8Hz).

### Example 12

### 1-Cyclobutyl-4-({(3S)-1-[6-(trifluoromethyl)-3-pyridinyl]-3-pyrrolidinyl}carbonyl)piperazine (E12)

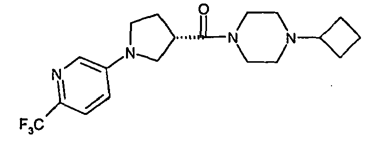

The title compound (E12) was prepared from 5-bromo-2-(trifluoromethyl)pyridine (may be prepared as described in F. Cottet and M. Schlosser, Eur. J. Org. Chem., 2002, 327) and 1-cyclobutyl-4-[(3*S*)-3-pyrrolidinylcarbonyl]piperazine (may be prepared as described in Description 10) using an analogous process to that described in Example 5. LCMS electrospray (+ve) 383 (MH⁺). ¹H NMR (CDCl₃) δ 1.68-1.78 (2H, m), 1.83-1.93 (2H, m), 2.02-2.09 (2H, m), 2.22-2.39 (6H, m), 2.73 (1H, m), 3.38-3.68 (9H, m), 6.82 (1H, dd, J=8.8, 2.8Hz), 7.47 (1H, d, J=8.8Hz), 8.01 (1H, d, J=2.8Hz).

### Example 13

### 1-Cyclobutyl-4-({(3S)-1-[4-(3-methyl-1,2,4-oxadiazol-5-yl)phenyl]-3-pyrrolidinyl}carbonyl)piperazine (E13)

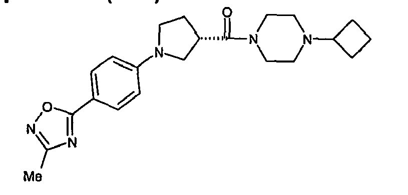

The title compound (E13) was prepared from 5-(4-bromophenyl)-3-methyl-1,2,4-oxadiazole (may be prepared as described in Description 23) and 1-cyclobutyl-4-[(3*S*)-3-pyrrolidinylcarbonyl]piperazine (may be prepared as described in Description 10) using an analogous process to that described in Example 5. LCMS electrospray (+ve) 396 (MH⁺). ¹H NMR (CDCl₃) δ 1.69-1.78 (2H, m), 1.83-1.94 (2H, m), 2.02-2.09 (2H, m), 2.21-2.42 (6H, m), 2.61 (3H, s), 2.73 (1H, m), 3.38-3.67 (9H, m), 6.58 (2H, d, J=8:8Hz), 7.94 (2H, d, J=8.8Hz).

### Example 14

### 1-(Cyclopropylmethyl)-4-({(3S)-1-[6-(trifluoromethyl)-3-pyridinyl]-3-pyrrolidinyl}carbonyl)piperazine (E14)

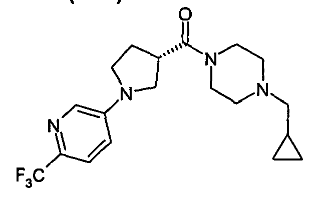

The title compound (E14) was prepared from 5-bromo-2-(trifluoromethyl)pyridine (may be prepared as described in Cottet and Schlosser, Eur. J. Org. Chem., 2002, 327) and 1-(cyclopropylmethyl)-4-[(3*S*)-3-pyrrolidinylcarbonyl]piperazine (may be prepared as described in Description 28) using an analogous process to that described in Example 5. LCMS electrospray (+ve) 383 (MH⁺). ¹H NMR (CDCl₃) δ 0.05-0.12 (2H, m), 0.51-0.57 (2H, m), 0.80-0.91 (1H, m), 2.23-2.39 (4H, m), 2.51-2.59 (4H, m), 3.39-3.75 (9H, m), 6.82 (1H, dd, J=8.8, 2.8Hz), 7.47 (1H, d, J=8.8Hz), 8.01 (1H, d, J=2.8Hz).

### Example 15

### 1-[4-((3S)-3-{[4-(Cyclopropylmethyl)-1-piperazinyl]carbonyl)-1-pyrrolidinyl)phenyl]ethanone (E15)

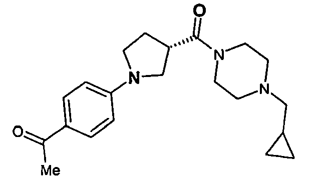

The title compound (E15) was prepared from 4-bromoacetophenone and 1-(cyclopropylmethyl)-4-[(3S)-3-pyrrolidinylcarbonyl]piperazine (may be prepared as described in Description 28) using an analogous process to that described in Example 5. LCMS electrospray (+ve) 356 (MH⁺). ¹H NMR (CDCl₃) δ 0.05-0.12 (2H, m), 0.51-0.57 (2H, m), 0.80-0.91 (1H, m), 2.23-2.39 (4H, m), 2.50 (3H, s), 2.51-2.59 (4H, m), 3.36-3.71 (9H, m), 6.51 (2H, d, J=8.8Hz), 7.86 (2H, d, J=8.8Hz).

### Example 16

### 1-(Cyclopropytmethyl)-4-({(3S)-1-[4-(3-methyl-1,2,4-oxadiazol-5-yl)phenyl]-3-pyrrolidinyl}carbonyl)piperazine (E16)

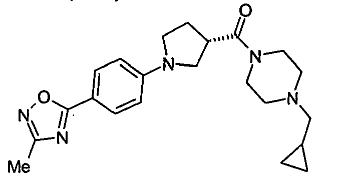

The title compound (E16) was prepared from 5-(4-bromophenyl)-3-methyl-1,2,4-oxadiazole (may be prepared as described in Description 23) and 1-(cyclopropylmethyl)-4-[(3S)-3-pyrrolidinylcarbonyl] piperazine (may be prepared as described in Description 28) using an analogous process to that described in Example 5. LCMS electrospray (+ve) 396 (MH⁺). ¹H NMR (CDCl₃) δ 0.05-0.12 (2H, m), 0.51-0.57 (2H, m), 0.80-0.91 (1H, m), 2.22-2.38 (4H, m), 2.42 (3H, s), 2.52-2.59 (4H, m), 3.37-3.71 (9H, m), 6.59 (2H, d, J=8.8Hz), 7.94 (2H, d, J=8.8Hz).

### Example 17

### (R,S)-1-Cyclobutyl-4-({1-[6-(trifluoromethyl)-3-pyridinyl]-3-pyrrolidinyl}carbonyl)hexahydro-1H-1,4-diazepine (E17)

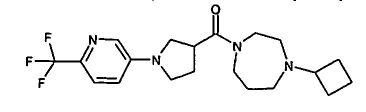

5-Bromo-2-(trifluoromethyl)pyridine (may be prepared as described in Cottet and Schlosser, Eur. J. Org. Chem., 2002, 327) (0.95g) in dry and degassed dioxan (40ml) was treated with bis(dibenzylideneacetone)palladium (0.64g) and 2-dicyclohexylphosphine-2'-(N,N-dimethylamino)biphenyl (0.71g) and stirred at room temperature for 20min followed by the addition of (R,S)-1-cyclobutyl-4-(3-pyrrolidinylcarbonyl)hexahydro-1*H*-1,4-diazepine (may be prepared as described in Description 30) (1.08g) in dioxane (10ml) and sodium t-butoxide (0.81g). The reaction mixture was heated at 95°C for 2h, then allowed to cool and diluted with MeOH (40ml). This solution was poured on to an SCX column which was washed with MeOH (40ml) and then eluted with ammonia in MeOH (2N, 30ml). Concentration of the ammonia fractions afforded a crude product which was further purified by chromatography [silica gel, eluting with 0-10% MeOH (containing 10% 0.880 ammonia solution)/DCM]. Pure fractions were combined and concentrated to give the title compound (E17) as a brown oil (0.350g). LCMS electrospray (+ve) 397 (MH⁺). Two enantiomers: Retention times = 5.3min; 6.6min [Chiralcel OJ column (250mm x 4.6mm, 10micron particle size); hexane ethanol 60 : 40 v/v; isocratic for 10min at 1 ml/min).

### Example 17A and Example 17B

### 1-Cyclobutyl-4-({(3R)-1-[6-(trifluoromethyl)-3-pyridinyl]-3-pyrrolidinyl}carbonyl)hexahydro-1H-1,4-diazepine hydrochloride (E17A) and 1-Cyclobutyl-4-({(3S)-1-[6-(trifluoromethyl)-3-pyridinyl]-3-pyrrolidinyl}carbonyl) hexahydro-1H-1,4-diazepine hydrochloride (E17B)

(R,S)-1-Cyclobutyl-4-({1-[6-(trifluoromethyl)-3-pyridinyl]-3-pyrrolidinyl}carbonyl) hexahydro-1*H*-1,4-diazepine (may be prepared as described in Example 17) (0.35g) was separated by chiral HPLC [Stationary phase: Chiralcel OJ column (250mm x 50mm i.d.; 20micron particle size); Mobile phase: hexane fraction:absolute ethanol 90:10 v/v; isocratic at a flow rate of 50ml/min; detection by UV absorbance at 215nm; sample injected as a solution in absolute ethanol]. Fractions containing the faster running enantiomer (retention time=31.1 min) were combined and concentrated to give the free base which was dissolved in dry DCM (5ml) and treated with 1N ethereal HCl (1ml). Evaporation of solvents afforded the title compound (E17A) as a cream solid (0.077g). ¹H NMR δ (methanol-d4): 1.7-1.95 (2H, m), 2.2-2.45 (8H, m), 2.9-3.2 (2H, m), 3.47-3.68 (8H, m), 3.75-3.9 (3H, m), 4.1-4.25 (1H, m), 7.07 (1H, dd, J=8Hz), 7.57 (1H, d, J=8Hz), 7.97 (1H, d, J=2.8Hz). LCMS electrospray 397 (MH⁺).
The slower running enantiomer (retention time=41.6min) was isolated and converted into the HCI salt as described above to give the title compound (E17B) as a cream coloured solid (0.1g). ¹H NMR δ (methanol-d4): 1.7-1.95 (2H, m), 2.2-2.45 (8H, m), 2.9-3.2 (2H, m), 3.47-3.68 (8H, m), 3.75-3.9 (3H, m), 4.1-4.25 (1H, m), 7.25 (1H, m), 7.70 (1H, m), 8.03 (1H, d, J=2.8Hz). LCMS electrospray 397 (MH⁺).

### Alternative Method

The title compound (E17A) was prepared in an analogous manner to Example 1 from 5-bromo-2-(trifluoromethyl)pyridine (may be prepared as described in F Cottet and M Schlosser, Eur. J. Org. Chem., 2002, 327) and 1-cyclobutyl-4-[(3R)-3-pyrrolidinylcarbonyl]hexahydro-1*H*-1,4-diazepine (may be prepared as described in Description 38). LCMS electrospray (+ve) 397 (MH⁺). ¹H NMR (CDCl₃) δ 1.56-2.07 (8H, m), 2.25-2.52 (6H, m), 2.81-2.99 (1H, m), 3.35-3.69 (9H, m), 6.82 (1H, dd, J=8.7 and 2.8Hz), 7.46 (1H, d, J=8.7Hz), 8.02 (1H, d, J=2.8Hz). Enantiomeric excess = 90% (Chiralcel OJ (250 x 4.6 mm, 10 micron particle size); Hexane:Ethanol 60:40 v/v; isocratic for 10 min at 1 ml/min). Retention time of major component = 5.3 min, minor component = 6.6 min.

### Example 18

### 1-Cyclobutyl-4-({(3R)-1-[4-(3-methyl-1,2,4-oxadiazol-5-yl)phenyl]-3-pyrrolidinyl}carbonyl)hexahydro-1H-1,4-diazepine hydrochloride and 1-Cyclobutyl-4-({(3S)-1-[4-(3-methyl-1,2,4-oxadiazol-5-yl)phenyl]-3-pyrrolidinyl}carbonyl) hexahydro-1H-1,4-diazepine hydrochloride

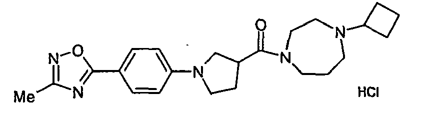

(R,S)-1-Cyclobutyl-4-({1-[4-(3-methyl-1,2,4-oxadiazol-5-yl)phenyl]-3-pyrrolidinyl}carbonyl)hexahydro-1*H*-1,4-diazepine was prepared using an analogous process to that described in Example 17 from (R,S)-1-cyclobutyl-4-(3-pyrrolidinylcarbonyl)hexahydro-1*H*-1,4-diazepine (may be prepared as described in Description 30) and 5-(4-bromophenyl)-3-methyl-1,2,4-oxadiazole (may be prepared as described in Description 23). The racemic product (300mg) was separated by chiral HPLC [Stationary phase: Chiralcel OJ column [250mm x 50 mm i.d., 20micron particle size; mobile phase: 100% ethanol, isocratic at a flow rate of 50.0mL/min; detection by UV absorbance at 215nm; sample injected as a solution in ethanol. Fractions containing the faster running enantiomer (E18A) (retention time = 8.03min) were combined and concentrated to give the free base which was treated with ethereal HCl to give the hydrochloride salt as a cream solid (104mg) ¹H NMR δ (methanol-d4): 1.77-1.95 (2H, m), 2.2-2.35 (7H, m), 2.4 (3H, s), 2.9-3.15, (2H, m), 3.47-3.90 (12H, m), 4.1-4.25 (1H, m), 6.7 (2H, d, J=8.8Hz), 7.9 (2H, d, J=8.8Hz). LCMS electrospray (+ve) 410 (MH⁺). Fractions containing the slower running enantiomer (E18B) (retention time = 13.0min) were combined and concentrated to give the free base which was treated with ethereal HCl to give the hydrochloride salt as a cream solid (81 mg). ¹H NMR δ (methanol-d4): 1.77-1.95 (2H, m), 2.2-2.35 (7H, m), 2.4 (3H, s), 2.9-3.15 (2H, m), 3.47-3.90 (12H, m), 4.1-4.25 (1H, m), 6.7 (2H, d, J=8.8Hz), 7.9 (2H, d, J=8.8Hz). LCMS electrospray (+ve) 410 (MH⁺).

### Example 19

### 6-{(3R)-3-[(4-Cyclobutylhexahydro-1H-1,4-diazepin-1-yl)carbonyl]-1-pyrrolidinyl}-2-methylquinoline hydrochloride and 6-{(3S)-3-[(4-Cyclobutylhexahydro-1H-1,4-diazepin-1-yl)carbonyl]-1-pyrrolidinyl}-2-methylquinoline hydrochloride

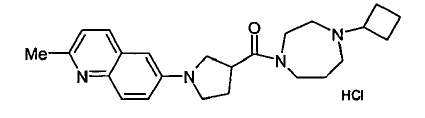

(R,S)-6-{3-[(4-Cyclobutylhexahydro-1*H*-1,4-diazepin-1-yl)carbonyl]-1-pyrrolidinyl}-2-methylquinoline was prepared in using an analogous process to that described in Example 17 from (R,S)-1-cyclobutyl-4-(3-pyrrolidinylcarbonyl)hexahydro-1*H*-1,4-diazepine (may be prepared as described in Description 30) and 6-bromo-2-methylquinoline. The racemic product (0.5g) was separated by chiral HPLC [Stationary phase: Chiralcel OJ column (250mm x 20 mm i.d., 10micron particle size); mobile phase: 100% ethanol; isocratic flow rate of 17ml/min; detection by UV absorbance at 215nm; sample injected as a solution in ethanol]. Fractions containing the faster running enantiomer (E19A) (retention time = 8.03min) were combined and concentrated to give the free base which was treated with ethereal HCl to give the hydrochloride salt as a yellow solid (186mg). ¹H NMR δ (methanol-d4) 1.78-1.95 (2H, m), 2.35-2.5 (7H, m), 2.87 (3H, s), 2.9-3.15 (2H, m), 3.45-3.90 (12, m), 4.10-4.25 (1H, m), 7.05 (1H, s), 7.6 (1H, d, J=8.8Hz), 7.69 (1H, d, J=8.8Hz), 7.94 (1H, d, J=8.8Hz), 8.67 (1H, d, J=8.8Hz). LCMS AP⁺ (+ve) 393 (MH⁺). Fractions containing the slower running enantiomer (E19B) (retention time = 13.0min) were combined and concentrated to give the free base which was treated with ethereal HCl to give the hydrochloride salt as a yellow solid (197mg). ¹H NMR δ (methanol-d4): 1.78-1.95 (2H, m), 2.25-2.5 (7H, m), 2.87 (3H, s), 2.9-3.15 (2H, m), 3.45-3.90 (12, m), 4.10-4.25 (1H, m), 7.05 (1H, s), 7.6 (1H, d, J=8.8Hz),7.69 (1H, d, J=8.8Hz), 7.94 (1H, d, J=8.8Hz), 8.67 (1H, d, J=8.8Hz). LCMS AP⁺ (+ve) 393 (MH⁺).

### Example 20

### 1-Cyclobutyl-4-({(3R)-1-[2-fluoro-4-(3-methyl-1,2,4-oxadiazol-5-yl)phenyl]-3-pyrrolidinyl}carbonyl)hexahydro-1H-1,4-diazepine hydrochloride and 1-Cyclobutyl-4-({(3S)-1-[2-fluoro-4-(3-methyl-1,2,4-oxadiazol-5-yl)phenyl]-3-pyrrolidinyl}carbonyl)hexahydro-1H-1,4-diazepine hydrochloride

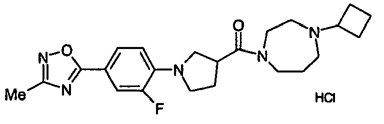

(R,S)-1-Cyclobutyl-4-({1-[2-fluoro-4-(3-methyl-1,2,4-oxadiazol-5-yl)phenyl]-3-pyrrolidinyl}carbonyl)hexahydro-1*H*-1,4-diazepine was prepared using an analogous process to that described in Example 17 from (R,S)-1-cyclobutyl-4-(3-pyrrolidinylcarbonyl)hexahydro-1*H*-1,4-diazepine (may be prepared as described in Description 30) and 5-(4-bromo-3-fluorophenyl)-3-methyl-1,2,4-oxadiazole (may be prepared as described in Description 31). The racemic product (0.09g) was separated by chiral HPLC [Stationary phase: Chiral OJ column (250mm x 50 mm i.d., 20micron particle size); mobile phase: 100% ethanol at a flow rate of 50mUmin; detection by UV absorbance at 215nm; sample injected as a solution in ethanol]. Fractions containing the faster running enantiomer (E20A) (retention time = 6.6min) were combined and concentrated to give the free base which was treated with 1 M ethereal HCl to give a cream solid (17.8mg). ¹H NMR δ (methanol-d4): 1.78-1.95 (2H, m), 2.25-2.5 (7H, m), 2.87 (3H, s), 2.9-3.15 (2H, m), 3.45-3.90 (12H, m), 4.10-4.25 (1H, m), 6.8 (1H, t, J=8.8Hz), 7.65 (1H, d, J=14.8Hz), 7.75 (1H, d, J=8.8Hz). LCMS electrospray (+ve) 428 (MH⁺). Fractions containing the slower running enantiomer (E20B) (retention time = 9.4min) were combined and concentrated to give the free base which was treated with 1M ethereal HCl to give a cream solid (17mg). ¹H NMR δ (methanol-d4): 1.78-1.95 (2H, m), 2.25-2.5 (7H, m), 2.87 (3H, s), 2.9-3.15 (2H, m), 3.45-3.90 (12, m), 4.10-4.25 (1H, m), 6.8 (1H, t, J=8.8Hz), 7.65 (1H, d, J=14.8Hz), 7.75 (1H, d, J=8.8Hz). LCMS electrospray (+ve) 428 (MH⁺).

### Example 21

### 1-(4-{(3R)-3-[(4-Cyclobutylhexahydro-1H-1,4-diazepin-1-yl)carbonyl]-1-pyrrolidinyl}phenyl)ethanone hydrochloride and 1-(4-{(3S)-3-[(4-Cyclobutylhexahydro-1H-1,4-diazepin-1-yl)carbonyl]-1-pyrrolidinyl}phenyl)ethanone hydrochloride

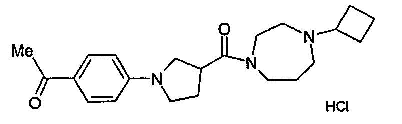

(R,S)-1-(4-{3-[(4-Cyclobutylhexahydro-1*H*-1,4-diazepin-1-yl)carbonyl]-1-pyrrolidinyl}phenyl)ethanone was prepared using an analogous process to that described in Example 17 from (R,S)-1-cyclobutyl-4-(3-pyrrolidinylcarbonyl)hexahydro-1*H*-1,4-diazepine (may be prepared as described in Description 30) and 4-fluoroacetophenone. The racemic product (0.39g) was separated by chiral HPLC [Stationary phase: Chiralcel OJ column (250mm x 50 mm i.d., 20micron particle size); mobile phase: 100% ethanol at a flow rate of 50ml/min; detection by UV absorbance at 215nm; sample injected as a solution in ethanol]. Fractions containing the faster running enantiomer (E21A) (retention time = 6.7min) were combined and concentrated to give the free base which was treated with 1M ethereal HCl to give a cream solid (25mg). ¹H NMR δ (methanol-d4): 1.78-1.95 (2H, m), 2.15-2.45 (7H, m), 2.5 (3H, s), 2.9-3.15 (2H, m), 3.45-3.90 (12H, m), 4.10-4.25 (1H, m), 6.6 (2H, d, J=9.2Hz), 7.85 (2H, d, J=9.2Hz).

LCMS electrospray (+ve) 370 (MH⁺). Fractions containing the slower running enantiomer (E21 B) (retention time = 8.7min) were combined and concentrated to give the free base which was treated with 1M ethereal HCl to give a cream solid (20mg). ¹H NMR δ (methanol-d4): 1.78-1.95 (2H, m), 2.15-2.45 (7H, m), 2.5 (3H, s), 2.9-3.15 (2H, m), 3.45-3.90 (12, m), 4.10-4.25 (1H, m), 6.6 (2H, d, J=9.2Hz), 7.85 (2H, d, J=9.2Hz). LCMS electrospray (+ve) 370 (MH⁺).

### Example 22

### (R,S)-1-(1-Methylethyl)-4-({1-[6-(trifluoromethyl)-3-pyridinyl]-3-pyrrolidinyl}carbonyl)hexahydro-1H-1,4-diazepine hydrochloride (E22)

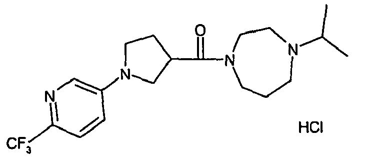

5-Bromo-2-(trifluoromethyl)pyridine (may be prepared as described in F. Cottet and M. Schlosser, Eur. J. Org. Chem., 2002, 327) (0.156g) was added to a solution of tris(dibenzylideneacetone)dipalladium(0) (0.03g) and 2-dicyclohexylphosphino-2'-(N,N-dimethylamino)-biphenyl (0.06g) in dry, degassed 1,4-dioxane (2.5ml) and the mixture stirred for 40 min. at room temperature under argon. (R,S)-1-(1-methylethyl)-4-(3-pyrrolidinylcarbonyl)hexahydro-1*H*-1,4-diazepine (may be prepared as described in Description 36) (0.15g in 2.5ml DME) and sodium *tert*-butoxide (0.12g) were added sequentially and the mixture heated to 90°C for 2h. The mixture was then diluted with MeOH, loaded onto a 10g SCX cartridge and eluted with MeOH (70ml) followed by 2M NH₃/MeOH (70ml). The ammoniacal fractions were collected and evaporated and the residue purified by flash chromatography [silica gel, 0-10% MeOH (containing 10% 0.88 ammonia solution)/DCM]. The pure fractions were evaporated and the free base converted into the title hydrochloride salt (E22) with 1 M HCl in diethyl ether. LCMS electrospray (+ve) 385 (MH⁺). ¹H NMR (DMSO-d6) δ: 1.27 (6H, d, J=6.5Hz), 1.99-2.42 (5H, m), 2.98-3.28 (2H, m), 3.35-3.76 (10H, m), 3.95-4.22 (1H, m), 7.00 (1H, dd, J=8.6 and 2.0Hz), 7.59 (1H, d, J=8.6Hz), 8.05 (1H, d, J=2.0Hz), 10.20-10.40 (1H, bs).

### Example 23

### (R,S)-1-Cyctobutyl-4-({1-[2-(trifluoromethyl)-4-pyridiny)]-3-pyrrolidinyl}carbonyl)hexahydro-1H-1,4-diazepine hydrochloride (E23)

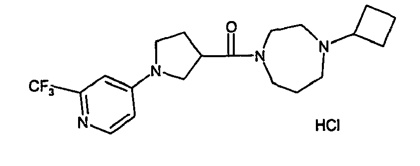

The title compound (E23) was prepared using an analogous process to that described in Example 22 from 4-iodo-2-(trifluoromethyl)pyridine (may be prepared as described in F Cottet et al., Eur. J. Org. Chem., 2003, 1559) and (R,S) 1-cyclobutyl-4-(3-pyrrolidinylcarbonyl)hexahydro-1*H*-1,4-diazepine (may be prepared as described in Description 30). LCMS electrospray (+ve) 397 (MH⁺). ¹H NMR (methanol-d4) δ: 1.72-1.99 (2H, m), 2.15-2.53 (8H, m), 2.84-3.20 (2H, m), 3.51-3.90 (10H, m), 3.99-4.24 (2H, m), 6.99 (1H, dd, J=7.1 and 2.6Hz), 7.25 (1H, d, J=2.6Hz), 8.19 (1H, d, J=7.1Hz).

### Example 24

### 1-({(3S)-1-[3-Fluoro-4-(3-methyl-1,2,4-oxadiazol-5-yl)phenyl]-3-pyrrolidinyl}carbonyl)-4-(1-methylethyl)piperazine (E24)

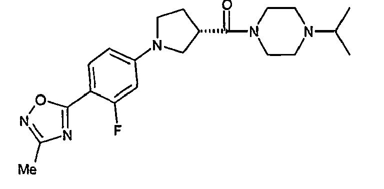

5-(4-Bromo-2-fluorophenyl)-3-methyl-1,2,4-oxadiazole (may be prepared as described in Description 32) (0.275g) was added to a stirred solution of tris(dibenzylideneacetone)dipalladium(0) (0.055g) and 2-dicyclohexylphosphino-2'-(N,N-dimethylamino)-biphenyl (0.07g) in DME (4ml) under argon at room temperature. Potassium phosphate (0.377g) and 1-(1-methylethyl)-4-[(3*S*)-3-pyrrolidinylcarbonyl]piperazine (may be prepared as described in Description 6) (0.2g in 1ml DME) were added sequentially and the mixture heated to 75°C for 4h. The mixture was then diluted with MeOH, loaded onto a 10g SCX cartridge and eluted with MeOH (70ml) followed by 2M NH₃/MeOH (70ml). The ammoniacal fractions were collected and evaporated and the residue purified by flash chromatography [silica gel, 0-10% MeOH (containing 10% 0.88 ammonia solution)/DCM]. The pure fractions were evaporated and the residue triturated with diethyl ether to afford the title compound (E24) as a white solid (0.055g). LCMS electrospray (+ve) 402 (MH⁺). ¹H NMR (CDCl₃) δ 1.05 (6H, d, J=6.8Hz), 2.21-2.39 (2H, m), 2.45 (3H, s), 2.50-2.57 (4H, m), 2.70-2.77 (1H, m), 3.38-3.67 (9H, m), 6.31 (1H, dd, J=14.0 and 2.4Hz), 6.40 (1H, dd, J=8.8 and 2.4Hz), 7.89 (1H, t, J=8.8Hz).

### Example 25

### 1-({(3S)-1-[2-Fluoro-4-(3-methyl-1,2,4-oxadiazol-5-yl)phenyl]-3-pyrrolidinyl}carbonyl)-4-(1-methylethyl)piperazine (E25)

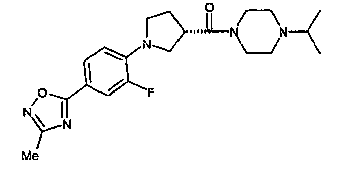

5-(4-Bromo-3-fluorophenyl)-3-methyl-1,2,4-oxadiazole (may be prepared as described in Description 31) (0.275g) was added to a stirred solution of tris(dibenzylideneacetone)dipalladium(0) (0.055g) and 2-dicyclohexylphosphino-2'-(N,N-dimethylamino)-biphenyl (0.07g) in DME (4ml) under argon at room temperature. Potassium phosphate (0.377g) and 1-(1-methylethyl)-4-[(3S)-3-pyrrolidinylcarbonyl]piperazine (may be prepared as described in Description 6) (0.2g in 1 ml DME) were added sequentially and the mixture heated to 75°C for 4h. The mixture was then diluted with MeOH, loaded onto a 10g SCX cartridge and eluted with MeOH (70ml) followed by 2M NH₃/MeOH (70ml). The ammoniacal fractions were collected and evaporated and the residue purified by flash chromatography [silica gel, 0-10% MeOH (containing 10% 0.88 ammonia solution)/DCM]. The pure fractions were evaporated and the residue triturated with diethyl ether (x 2) to afford the title compound (E25) as a white solid (0.050g). LCMS electrospray (+ve) 402 (MH⁺). ¹H NMR (CDCl₃) δ 1.05 (6H, d, J=6.8Hz), 2.17-2.32 (2H, m), 2.42 (3H, s), 2.49-2.56 (4H, m), 2.70-2.77 (1H, m), 3.32-3.67 (9H, m), 6.67 (1H, t, J=8.4Hz), 7.68 (1H, dd, J=14.4 and 2.0Hz), 7.73 (1H, dd, J=8.4 and 2.0Hz).

### Example 26

### 1-(1-Methylethyl)-4-({(3S)-1-[6-(trifluoromethyl)-3-pyridinyl]-3-pyrrolidinyl}carbonyl)hexahydro-1H-1,4-diazepine hydrochloride (E26)

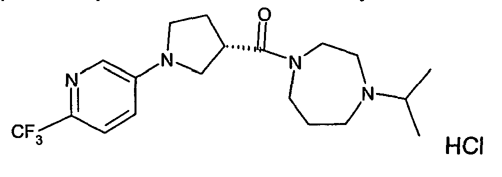

5-Bromo-2-(trifluoromethyl)pyridine (may be prepared as described in Cottet and Schlosser, Eur. J. Org. Chem., 2002, 327) (0.226g) was added to a stirred solution of tris(dibenzylideneacetone)dipalladium(0) (0.055g) and 2-dicyclohexylphosphino-2'-(*N,N-*dimethylamino)-biphenyl (0.07g) in DME (4ml) under argon at room temperature. Potassium phosphate (0.354g) and 1-(1-methylethyl)-4-[(3S)-3-pyrrolidinylcarbonyl]hexahydro-1H-1,4-diazepine (may be prepared as described in Description 40) (0.2g) in DME (1ml) were added sequentially and the mixture heated to 75°C for 4h. The mixture was then diluted with MeOH, loaded onto a 10g SCX cartridge and eluted with MeOH (70ml) followed by 2M NH₃/MeOH (70ml). The ammoniacal fractions were combined, evaporated and the residue purified by flash chromatography [silica gel, 0-10% MeOH (containing 10% 0.88 ammonia solution)/DCM]. The clean fractions were evaporated and the residue dissolved in a minimum of EtOAc. 1 M HCl/diethylether (0.3 ml) was added and evaporation of the solvents afforded the title compound (E26) as a pale yellow solid (0.06g). LCMS electrospray (+ve) 385 (MH⁺). ¹H NMR (CDCl₃) δ: 1.44 (6H, d, J=6.0Hz), 2.01-2.52 (5H, m), 2.80-3.08 (2H, m), 3.24 (1H, m), 3.45-3.99 (9H, m), 4.25-4.41 (1H, m), 6.89 (1H, m), 7.51 (1H, d, J=8.4Hz), 8.04 (1H, dd, J=9.2 and 2.0Hz), 12.35-12.6 (1H, bm).

### Example 27

### 1-[4-((3S)-3-{[4-(1-Methylethyl)hexahydro-1H-1,4-diazepin-1-yl]carbonyl}-1-pyrrolidinyl)phenyl]ethanone (E27)

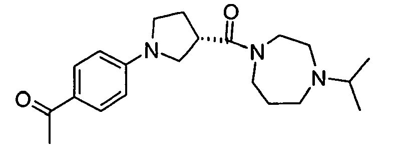

The title compound (E27) was prepared from 4-bromoacetophenone and 1-(1-methylethyl)-4-[(3S)-3-pyrrolidinylcarbonyl]hexahydro-1H-1,4-diazepine (may be prepared as described in Description 40) using an analogous process to that described in Example 5. LCMS electospray (+ve) 358 (MH⁺). ¹H NMR (CDCl₃) δ 1.06 (6H, m), 1.76-1.91 (2H, m), 2.21-2.28 (1H, m), 2.31-2.41 (1H, m), 2.50 (3H, s), 2.59-2.75 (4H, m), 2.89-2.96 (1H, m), 3.35-3.46 (2H, m), 3.54-3.68 (7H, m), 6.51 (2H, d, J=8.8Hz) and 7.86 (2H, d, J=8.8Hz).

### Example 28

### 1-(1-Methylethyl)-4-{{(3S)-1-[4-(3-methyl-1,2,4-oxadiazol-5-yl)phenyl]-3-pyrrolidinyl}carbonyl)hexahydro-1H-1,4-diazepine (E28)

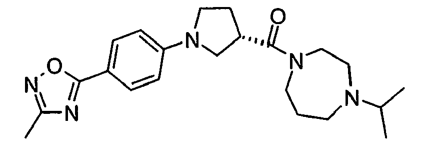

5-(4-Bromophenyl)-3-methyl-1,2,4-oxadiazole (may be prepared as described in Description 23) (0.240g), potassium phosphate (0.354g) and 1-(1-methylethyl)-4-[(3S)-3-pyrrolidinylcarbonyl]hexahydro-1H-1,4-diazepine (may be prepared as described in Description 40) (0.2g in 1 ml DME) was added to a stirred solution of tris(dibenzylideneacetone) dipalladium(0) (0.055g) and 2-dicyclohexylphosphino-2'-(*N,N-*dimethylamino)-biphenyl (0.07g) in dry DME (4 ml). The mixture was heated to 70°C for 3.5 hours under argon. The crude mixture was diluted with MeOH, loaded onto a 10g SCX cartridge and eluted with MeOH followed by 2M NH₃/MeOH. The residue was purified on a silica chromatography column (FM(II) system) eluting with a gradient of 0-30% MeOH in DCM. The pure fractions were collected and evaporated to afford the title compound
(E28) as a pale yellow solid (0.085g). LCMS electrospray (+ve) 398 (MH⁺). ¹H NMR (CDCl₃) δ 1.05 (6H, m), 1.76-1.91 (2H, m), 2.21-2.29 (1H, m), 2.31-2.42 (1H, m), 2.42 (3H, s), 2.58-2.75 (4H, m), 2.89-2.96 (1H, m), 3.36-3.48 (2H, m), 3.55-3.67 (7H, m), 6.58 (2H, d, J=8.8Hz) and 7.94 (2H, d, J=8.8Hz).

### Example 29

### 1-({(3S)-1-[3-Fluoro-4-(3-methyl-1,2,4-oxadiazol-5-yl)phenyl]-3-pyrrolidinyl}carbonyl)-4-(1-methylethyl)hexahydro-1H-1,4-diazepine hydrochloride (E29)

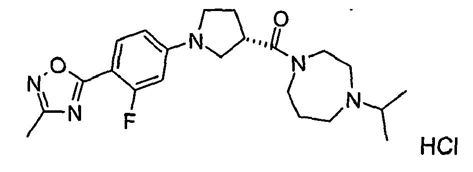

The title compound (E29) was prepared from 5-(4-bromo-2-fluorophenyl)-3-methyl-1,2,4-oxadiazole (may be prepared as described in Description 32) and 1-(1-methylethyl)-4-[(3S)-3-pyrrolidinylcarbonyl]hexahydro-1H-1,4-diazepine (may be prepared as described in Description 40) using an analogous process to that described in Example 26. LCMS electrospray (+ve) 416 (MH⁺). ¹H NMR (DMSO-d6) δ 1.05 (6H, m), 1.07-2.38 (4H, m), 2.38 (3H, s), 2.99-3.23 (2H, m), 3.40-3.80 (11H, m), 3.93-4.05 (1H, m), 6.50-6.57 (2H, m), 7.85 (1H, t, J=8.8Hz) and 10.1 (1H, m).

### Example 30

### 1-(1-Methylethyl)-4-({(3S)-1-[4-(5-methyl-1,3,4-oxadiazol-2-yl)phenyl]-3-pyrrolidinyl}carbonyl)piperazine (E30)

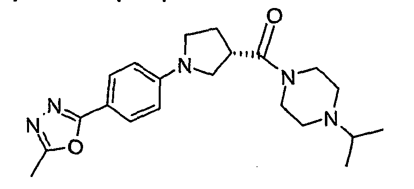

2-(4-Bromophenyl)-5-methyl-1,3,4-oxadiazole, (may be prepared as described in Description 14 of WO9743262) (0.33g) was added to a stirred solution of tris(dibenzylideneacetone) dipalladium(0) (0.076g) and 2-dicyclohexylphosphino-2'-(*N*,*N* dimethylamino)-biphenyl (0.098g) in DME (4ml). Potassium phosphate (0.470g) and 1-(1-methylethyl)-4-[(3S)-3-pyrrolidinylcarbonyl]piperazine (may be prepared as described in Description 6) (0.25g in 1 ml DME) were added sequentially and the mixture was heated to 75°C for 6 hours under argon. After cooling, the reaction mixture was diluted with MeOH, loaded onto a 10g SCX cartridge and eluted with MeOH (30ml) followed by 2M NH₃/MeOH (30ml). The ammoniacal fractions were evaporated and the residue co-evaporated from methanol (x2). The residue was then triturated with ether (x3) to afford the title compound (E30) as a beige solid (0.171g). LCMS electrospray (+ve) 384 (MH⁺). ¹H NMR (CDCl₃) δ 1.06 (6H, d, J=6.4Hz), 2.21-2.39 (2H, m), 2.50-2.57 (7H, m), 2.71-2.77 (1H, m), 3.36-3.48 (2H, m), 3.52-3.68 (7H, m), 6.59 (2H, d, J=8.8Hz), 7.85 (2H, d, J=8.8Hz).

### Example 31

### 1-(1-Methylethyl)-4-({(3S)-1-[3-(3-methyl-1,2,4-oxadiazol-5-yl)phenyl]-3-pyrrolidinyl}carbonyl)piperazine hydrochloride (E31)

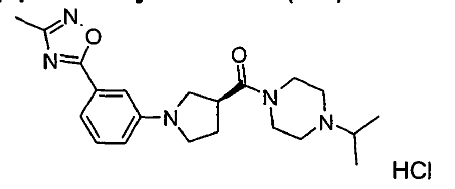

The title compound (E31) was prepared from 5-(3-bromophenyl)-3-methyl-1,2,4-oxadiazole (may be prepared as described in Description 48) and 1-(1-methylethyl)-4-[(3*S*)-3-pyrrolidinylcarbonyl]piperazine (may be prepared as described in Description 6) using an analogous process to that described in Example 26. LCMS electrospray (+ve) 384 (MH⁺). ¹H NMR (Methanol-d4) δ 1.41 (6H, d, J=6.8Hz), 2.26-2.36 (2H, m), 2.42 (3H, s), 3.09-3.25 (3H, m), 3.43-3.69 (9H, m), 4.41 (1H, m), 4.76 (1H, m), 6.91 (1H, m), 7.37 (1H, s) and 7.39 (2H, m).

### Example 32

### (R, S)-1-(1-Methylethyl)-4-({1-[4-(2-methyl-1,3-oxazol-4-yl)phenyl]-3-pyrrolidinyl}carbonyl)piperazine (E32)

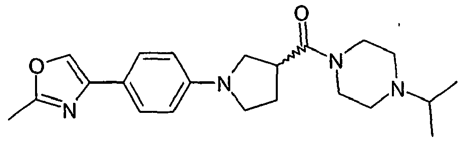

4-(4-Bromophenyl)-2-methyl-1,3-oxazole (may be prepared as described in Description 43) (0.476g), 1-(1-methylethyl)-4-[(3-pyrrolidinylcarbonyl]piperazine (0.45g) (may be prepared in an analogous manner to that described in Description 5 and Description 6 from 1-(*tert*-butoxycarbonyl)-pyrrolidine-3-carboxylic acid, tris(dibenzylideneacetone)dipalladium(0) (0.137g), 2-(dicyclohexylphosphino)biphenyl (0.158g) and potassium phosphate (0.848g) were heated together in dioxane (8ml) under an argon atmosphere at 90°C for 16h. The mixture was cooled and diluted with MeOH, loaded onto a 10g SCX cartridge and eluted with MeOH (100ml) followed by 2M NH₃/MeOH (50ml). The ammoniacal fractions were collected and evaporated. The residue was purified by chromatography on silica (Biotage, KP-NH cartridge) eluting with 0-100% EtOAc in hexane. The clean fractions were evaporated and the residue recrystallised from EtOH to afford the title compound (E32) as colourless needles (0.162g). LCMS electrospray (+ve) 383 (MH⁺). ¹H NMR (CDCl₃) δ 1.06 (6H, d, J=6.8Hz), 2.18-2.24 (1H, m), 2.34-2.39 (1H, m), 2.49 (3H, s), 2.49-2.56 (4H, m), 2.70-2.76 (1H, m), 3.35-3.42 (2H, m), 3.47-3.68 (7H, m), 6.58 (2H, d, J=8.8Hz), 7.56 (2H, d, J=8.8Hz) and 7.66 (1H, s).

### Example 32A

### 1-(1-Methylethyl)-4-({(3S)-1-[4-(2-methyl-1,3-oxazol-4-yl)phenyl]-3-pyrrolidinyl}carbonyl)piperazine (E32A)

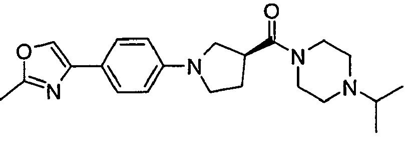

### Method A

4-(4-Bromophenyl)-2-methyl-1,3-oxazole (may be prepared as described in Description 43, method A) (0.329g) was added to a stirred solution of tris(dibenzylideneacetone) dipalladium(0) (0.076g) and 2-dicyclohexylphosphino-2'-(N,N-dimethylamino)-biphenyl (0.098g) in 4ml DME. Potassium phosphate (0.47g) and 1-(1-methylethyl)-4-[(3S)-3-pyrrolidinylcarbonyl]piperazine (may be prepared as described in Description 6, method B) (0.25g in 1ml DME) were added sequentially and the mixture heated to 75°C for 6h under an argon atmosphere. The mixture was cooled and diluted with MeOH, loaded onto a 10g SCX cartridge and eluted with MeOH (30ml) followed by 2M NH₃/MeOH (30ml). The ammoniacal fractions were collected and evaporated and the residue co-evaporated from methanol (x2). The residue was purified on a silica column (FM(II) system) eluting with 0-20% MeOH in DCM. The clean fractions were evaporated and the residue was triturated with diethyl ether (x3) to afford the title compound (E32A) as a pale yellow solid (0.07g) LCMS electrospray (+ve) 383 (MH⁺). ¹H NMR (CDCl₃) δ 1.06 (6H, d, J=6.8Hz), 2.18-2.23 (1H, m), 2.32-2.38 (1H, m), 2.50 (3H, s), 2.50-2.56 (4H, m), 2.70-2.76 (1H, m), 3.35-3.42 (2H, m), 3.46-3.68 (7H, m), 6.58 (2H, d, J=8.8Hz), 7.56 (2H, d, J=8.8Hz) and 7.66 (1H, s).

### Method B

4-(4-Bromophenyl)-2-methyl-1,3-oxazole (may be prepared as described in Description 43, method B) (0.238g) was added to a stirred solution of tris(dibenzylideneacetone) dipalladium(0) (0.068g) and 2-(dicyclohexylphosphino)biphenyl (0.088g) in dry, degassed DME (2ml). Potassium phosphate (0.424g) and 1-(1-methylethyl)-4-[(3S)-3-pyrrolidinylcarbonyl]piperazine (may be prepared as described in Description 6, method C) (0.225g in 2ml dry, degassed DME) were added sequentially and the mixture heated to 60°C for 4.5h followed by 3h at 90°C under an argon atmosphere. The mixture was cooled, dissolved in MeOH, loaded onto a 10g SCX cartridge and eluted with MeOH (60ml) followed by 2M NH₃/MeOH (60ml). The ammoniacal fractions were collected and evaporated. The residue was purified by chromatography (KP-NH cartridge) eluting with 0-100% EtOAc in hexane. The clean fractions were evaporated and the residue recrystallised in a minimum volume of hot EtOH to afford the title compound (E32A) as colourless needles (0.1g) LCMS electrospray (+ve) 383 (MH⁺). ¹H NMR (CDCl₃) δ 1.06 (6H, d, J=6.8Hz), 2.17-2.24 (1H, m), 2.31-2.39 (1H, m), 2.49 (3H, s), 2.49-2.56 (4H, m), 2.70-2.76 (1H, m), 3.35-3.42 (2H, m), 3.46-3.68 (7H, m), 6.58 (2H, d, J=8.8Hz), 7.56 (2H, d, J=8.8Hz) and 7.66 (1H, s). Enantiomeric excess = 95.2% (Chiralcel OD (250 x 4.6 mm, 10 micron particle size); Heptane:Ethanol 50:50 v/v; isocratic for 20 min at 1 ml/min). Retention time of major component = 9.28 min, minor component = 15.1 min.

### Method C

4-(4-Bromophenyl)-2-methyl-1,3-oxazole (may be prepared as described in Description 43, method B) (0.238g) was added to a stirred solution of tris(dibenzylideneacetone) dipalladium(0) (0.068g) and 2-(dicyclohexylphosphino)biphenyl (0.088g) in dry, degassed dioxane (2ml). Caesium carbonate (0.650g) and 1-(1-methylethyl)-4-[(3S)-3-pyrrolidinylcarbonyl]piperazine (may be prepared as described in Description 6, method C) (0.225g in 2ml dry, degassed dioxane) were added sequentially and the mixture heated to 60°C for 4.5h followed by 3h at 90°C under an argon atmosphere. The mixture was loaded onto a 10g SCX cartridge and eluted with MeOH (50ml) followed by 2M NH₃/MeOH (50ml). The ammoniacal fractions were combined and evaporated. 100mg residue was purified by chromatography (12 M amino, KP-NH cartridge). The clean fractions were evaporated to afford the title compound (E32A) as a white solid (0.04g). (LCMS electrospray (+ve) 383 (MH+))

### Method D

4-(4-Bromophenyl)-2-methyl-1,3-oxazole (may be prepared as described in Description 43, method B) (0.238g) was added to a stirred solution of tris(dibenzylideneacetone) dipalladium(0) (0.068g) and 2-(dicyclohexylphosphino)biphenyl (0.088g) in dry, degassed DME (2ml). Caesium carbonate (0.650g) and 1-(1-methylethyl)-4-[(3*S*)-3-pyrrolidinylcarbonyl]piperazine (may be prepared as described in Description 6, method C) (0.225g in 2ml dry, degassed DME) were added sequentially and the mixture heated to 60°C for 4.5h followed by 3h at 90°C under an argon atmosphere to afford the title compound (E32A).

### Method E

4-(4-Bromophenyl)-2-methyl-1,3-oxazole (may be prepared as described in Description 43, method B) (0.238g) was added to a stirred solution of tris(dibenzylideneacetone) dipalladium(0) (0.068g) and 2-(dicyclohexylphosphino)biphenyl (0.088g) in dry, degassed dioxane (2ml). Potassium phosphate (0.424g) and 1-(1-methylethyl)-4-[(3S)-3-pyrrolidinylcarbonyl]piperazine (may be prepared as described in Description 6, method C) (0.225g in 2ml dry, degassed dioxane) were added sequentially and the mixture heated to 60°C for 4.5h followed by 3h at 90°C under an argon atmosphere to afford the title compound (E32A).

### Example 33

### 1-(1-Methylethyl)-4-({(3S)-1-[4-(5-methyl-1,2,4-oxadiazol-3-yl)phenyl]-3-pyrrolidinyl}carbonyl)piperazine (E33)

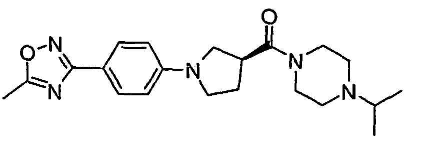

The title compound (E33) was prepared from 3-(4-bromophenyl)-5-methyl-1,2,4-oxadiazole (may be prepared as described in Description 45) and 1-(1-methylethyl)-4-[(3*S*)-3-pyrrolidinylcarbonyl]piperazine (may be prepared as described in Description 6) using an analogous process to that described in Example 30. LCMS electrospray (+ve) 384 (MH⁺). ¹H NMR (CDCl₃) δ 1.06 (6H, d, J=6.4Hz), 2.18-2.26 (1H, m), 2.32-2.39 (1H, m), 2.50-2.57 (4H, m), 2.57 (3H, s), 2.71-2.77 (1H, m), 3.35-3.45 (2H, m), 3.49-3.68 (7H, m), 6.59 (2H, d, J=8.8Hz), 7.90 (2H, d, J=8.8Hz).

### Example 34

### 1-(1-Methylethyl)-4-({(3S)-1-[4-(2-methyl-1,3-oxazol-5-yl)phenyl]-3-pyrrolidinyl}carbonyl)piperazine (E34)

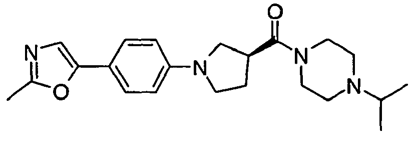

The title compound (E34) was prepared from 5-(4-bromophenyl)-2-methyl-1,3-oxazole (may be prepared as described in Description 46) and 1-(1-methylethyl)-4-[(3*S*)-3-pyrrolidinylcarbonyl]piperazine (may be prepared as described in Description 6) using an analogous process to that described in Example 30. LCMS electrospray (+ve) 383 (MH⁺). ¹H NMR (CDCl₃) δ 1.06 (6H, d, J=6.8Hz), 2.18-2.25 (1H, m), 2.31-2.38 (1H, m), 2.49 (3H, s), 2.49-2.56 (4H, m), 2.70-2.77 (1H, m), 3.35-3.42 (2H, m), 3.46-3.68 (7H, m), 6.57 (2H, d, J=8.8Hz), 6.98 (1H, s) and 7.45 (2H, d, J=8.8Hz).

### Example 35

### 3-((3S)-3-{[4-(1-Methylethyl)-1-piperazinyl]carbonyl}-1-pyrrolidinyl)benzonitrile hydrochloride (E35)

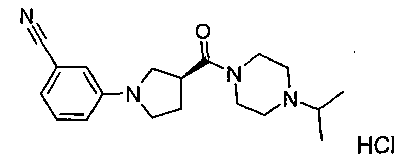

The title compound (E35) was prepared from 3-bromobenzonitrile and 1-(1-methylethyl)-4-[(3*S*)-3-pyrrolidinylcarbonyllpiperazine (may be prepared as described in Description 6) using an analogous process to that described in Example 26. LCMS electrospray (+ve) 327 (MH⁺). ¹H NMR (DMSO-d6) δ 1.28 (6H, d, J=6.0Hz), 2.08-2.19 (2H, m), 2.89-3.12 (3H, m), 3.28-3.59 (9H, m), 4.23 (1H, m), 4.52 (1H, m), 6.85-6.90 (2H, m), 6.98 (1H, d, J=7.6Hz) 7.34 (1H, t, J=7.6Hz). and 10.45 (1H, bs).

### Example 36

### 4-((3S)-3-{[4-(1-Methylethyl)-1-piperazinyl]carbonyl}-1-pyrrolidinyl)benzonitrile hydrochloride (E36)

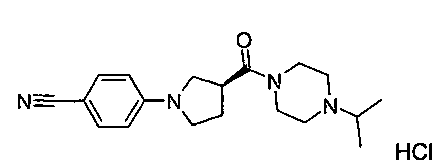

A mixture of 4-fluorobenzonitrile (0.129g), 1-(1-methylethyl)-4-[(3S)-3-pyrrolidinylcarbonyl]piperazine (may be prepared as described in Description 6) (0.2g) and potassium carbonate (0.245g) in DMSO (4ml) was heated to 130°C for 2h under argon. The mixture was allowed to cool, diluted with MeOH, loaded onto a 10g SCX cartridge which was eluted with MeOH (50ml) followed by 2M NH₃/MeOH (50ml). The ammoniacal fractions were evaporated and the residue purified by mass directed autopreparative HPLC . The clean fractions were evaporated, the residue taken up in a minimum of EtOAc and 1 M HCl/diethyl ether (0.5ml) added. The solvents were evaporated to afford the title compound (E36) as a white solid (0.107g). LCMS electrospray (+ve) 327 (MH⁺). ¹H NMR (DMSO-d6) δ 1.27 (6H, d, J=6.0Hz), 2.10-2.23 (2H, m), 2.86-3.12 (3H, m), 3.28-3.62 (9H, m), 4.23 (1H, m), 4.51 (1H, m), 6.62 (2H, d, J=8.4Hz), 7.54 (2H, d, J=8.4Hz) and 10.52 (1H, bs).

### Example 37

### 5-((3S)-3-{[4-(1-Methylethyl)-1-piperazinyl]carbonyl}-1-pyrrolidinyl)-2-(trifluoromethyl)pyrimidine (E37)

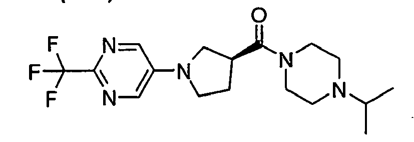

### 5-Bromo-2-(trifluoromethyl)pyrimidine (may be prepared as described in Description 47) (0.314g) was added to a stirred solution of tris(dibenzylideneacetone) dipalladium(0) (0.076g) and 2-dicyclohexylphosphino-2'-(N,N-dimethylamino)-biphenyl (0.98g in 4ml DME) under argon at room temperature in a microwave vial. Potassium phosphate (0.470g) and 1-(1-methylethyl)-4-[(3S)3-pyrrolidinylcarbonyl]piperazine (may be prepared as described in Description 6) (0.25g in 1ml DME) were added sequentially and the vial capped and crimped. The reaction was heated in a microwave oven to 120°C for 8 minutes, cooled and vented. The reaction mixture was diluted with MeOH, loaded onto a 10g SCX cartridge and eluted with MeOH (70ml) followed by 2M NH₃/MeOH (70ml). The ammoniacal fractions were evaporated and the residue triturated with diethyl ether (x 3) to afford the title compound (E37) as a white solid (0.077g). LCMS electrospray (+ve) 372 (MH⁺). ¹H NMR (CDCl₃) δ 1.06 (6H, d, J=6.4Hz), 2.29-2.36 (2H, m), 2.50-2.57 (4H, m), 2.71-2.78 (1H, m), 3.44-3.75 (9H, m) and 8.10 (2H, s).

### Example 38

### 1-(1-Methylethyl)-4-({(3R)-1-[4-(3-methyl-1,2,4-oxadiazol-5-yl)phenyl]-3-pyrrolidinyl}carbonyl)piperazine (E38)

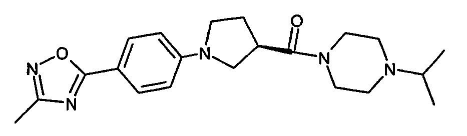

The title compound (E38) was prepared from 1-(1-methylethyl)-4-[(3*R*)-3-pyrrolidinylcarbonyl]piperazine (may be prepared as described in Description 41) and 5-(4-bromophenyl)-3-methyl-1,2,4-oxadiazole (may be prepared as described in Description 23) using an analogous process to that described in Example 3. LCMS electrospray (+ve) 384 (MH⁺). ¹H NMR (CDCl₃) δ 1.06 (6H, d, J=6.8Hz), 2.21-2.39 (2H, m), 2.42 (3H, s), 2.50-2.56 (4H, m), 2.70-2.77 (1H, m), 3.38-3.68 (9H, m), 6.59 (2H, d, J=8.8Hz), 7.95 (2H, d, J=8.8Hz). Enantiomeric excess = 98.8% (Chiralcel OJ (250 x 4.6 mm, 10 micron particle size; Heptane:Ethanol 50:50 v/v; isocratic for 25 min at 1 ml/min). Retention time of major component = 12.0 min, minor component = 19.4 min.

### Example 39

### 1-Cyclobutyl-4-({(3R)-1-[4-(3-methyl-1,2,4-oxadiazol-5-yl)phenyl]-3-pyrrolidinyl}carbonyl)piperazine (E39)

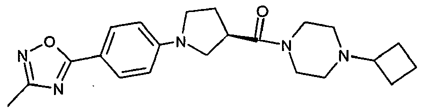

The title compound (E39) was prepared from 1-cyclobutyl-4-[(3R)-3-pyrrolidinylcarbonyl]piperazine (may be prepared as described in Description 42) and 5-(4-bromophenyl)-3-methyl-1,2,4-oxadiazole (may be prepared as described in Description 23) using an analogous process to that described in Example 5. LCMS electrospray (+ve) 396 (MH⁺). ¹H NMR (CDCl₃) δ 1.69-1.78 (2H, m), 1.83-1.94 (2H, m), 2.02-2.09 (2H, m), 2.21-2.42 (6H, m), 2.61 (3H, s), 2.73 (1H, m), 3.38-3.67 (9H, m), 6.58 (2H, d, J=8.8Hz), 7.94 (2H, d, J=8.8Hz).

### Example 40

### 1-(1-Methylethyl)-4-({(3S)-1-[4-(5-phenyl-1,3,4-oxadiazo)-2-yl)phenyl]-3-pyrrolidinyl}carbonyl)piperazine (E40)

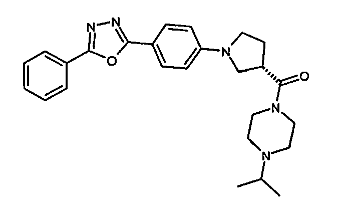

2-(4-Bromophenyl)-5-phenyl-1,3,4-oxadiazole (obtainable from Lancaster 8701; 0.301g), 2-dicyclohexylphosphino-2'-(N,N-dimethylamino)biphenyl (0.076g) and tris(dibenzylidene acetone)dipalladium(0) (0.060g) were introduced into a dry carousel tube under argon. Acetonitrile (3ml) was added, followed by a solution of 1-(1-methylethyl)-4-[(3S)-3-pyrrolidinylcarbonyl]piperazine (may be prepared as described in Description 6) (0.185g) in acetonitrile (4.1ml) and tripotassium phosphate (0.425g). The stirred mixture was heated under argon, at reflux for 3h and allowed to cool to rt. The reaction mixture was applied to an SCX column (50g) and eluted, first with MeOH and then with 2M NH₃ in MeOH. The crude product was obtained by evaporation of appropriate fractions and the title compound (E40) (0.202g) was obtained by trituration of the crude material with Et₂O.¹H NMR δ (CDCl₃): 1.07 (6H, d, J = 6.8Hz), 2.20-2.29 (1H, m), 2.33-2.42 (1H, m), 2.51-2.58 (4H, m), 2.75 (1H, septet, J = 6.8Hz), 3.38-3.51 (2H, m), 3.55-3.71 (7H, m), 6.63 (2H, d, J = 9Hz), 7.52 (3H, m), 7.98 (2H, d, J = 9Hz), 8.12 (2H, m); LCMS electrospray (+ve) 446 (MH⁺).

### Examples 41-45 (E41-E45)

Examples 41-45 were prepared using an analogous process to that described in Example 40 from 1-(1-methylethyl)-4-[(3S)-3-pyrrolidinylcarbonyl]piperazine (may be prepared as described in Description 6) and the appropriate aryl bromide or iodide, and displayed ¹H NMR and mass spectral data that were consistent with structure.

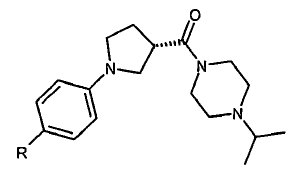

| **Example No** | **Name** | **R** | **Reagent** | **Mass Spectrum (ES⁺)** |
|---|---|---|---|---|
| **E41** | 1-({(3S)-1-[4-(5-isoxazolyl)phenyl]-3-pyrrolidinyl}carbonyl)-4-(1-methylethyl)piperazine | 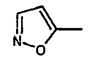 | 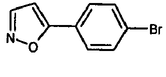 | 369 (MH⁺) |
| **E42** | 1-(1-methylethyl)-4-({(3S)-1-[4-(1H-pyrrol-1-yl)phenyl]-3-pyrrolidinyl}carbonyl)piperazine | 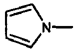 | 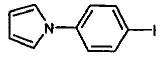 | 367 (MH⁺) |
| **E43** | 1-({(3S)-1-[4-(1H-imidazol-1-yl)phenyl]-3-pyrrolidinyl}carbonyl)-4-(1-methylethyl)piperazine | 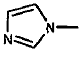 | 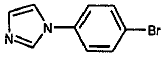 | 390 (MNa⁺) |
| **E44** | 1-(1-methylethyl)-4-({(3S)-1-[4-(1,3-oxazol-5-yl)phenyl]-3-pyrrolidinyl}carbonyl)piperazine | 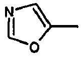 | 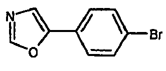 | 369 (MH⁺) |
| **E45** | 1-[4-((3S)-3-{[4-(1-methylethyl)-1-piperazinyl]carbonyl}-1-pyrrolidinyl)phenyl]-2-pyrrolidinone | 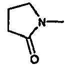 | 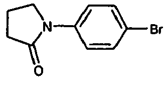 | 385 (MH⁺) |

### Example 46

### 4-((3S)-3-{[4-(1-Methylethyl)hexahydro-1H-1,4-diazepin-1-yl]carbonyl}-1-pyrrolidinyl)benzonitrile hydrochloride (E46)

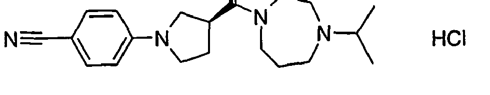

A mixture of 4-fluorobenzonitrile (0.160g), 1-(1-methylethyl)-4-[(3*S*)-3-pyrrolidinylcarbonyl]hexahydro-1*H*-1,4-diazepine (may be prepared as described in Description 40) (0.3g) and potassium carbonate (0.348g) in DMSO (4ml) was heated to 130°C for 2h under Argon. The mixture was allowed to cool, diluted with MeOH and loaded onto a 10g SCX cartridge which was eluted with MeOH (40ml) followed by 2M NH₃/MeOH (40ml). The ammoniacal fractions were evaporated and the residue purified by flash chromatography [silica gel, 0-15% MeOH/DCM]. The clean fractions were evaporated and the residue taken up in a minimum volume of DCM. A solution of 1 M HCl/Et₂O (0.3ml) was added and the solvents evaporated. The residue was then recrystallised from ethanol to afford the title compound (E46) as a white crystalline solid (0.07g). LCMS electrospray (+ve) 341 (MH⁺). ¹H NMR (DMSO-d6) δ 1.26 (6H, d, J=6.3Hz), 2.01-2.39 (4H, m), 2.95-3.30 (2H, m), 3.30-3.80 (11H, m), 4.03 (1H, m), 6.61 (2H, d, J=8.8Hz), 7.54 (2H, d, J=8.8Hz) and 10.15 (1H, bm).

### Example 47

### 1-(1-Methylethyl)-4-({(3S)-1-[4-(5-methyl-1,3,4-oxadiazol-2-yl)phenyl]-3-pyrrolidinyl}carbonyl)hexahydro-1H-1,4-diazepine hydrochloride (E47)

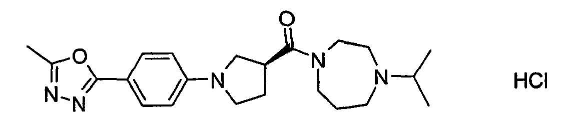

The title compound (E47) was prepared from 2-(4-bromophenyl)-5-methyl-1,3,4-oxadiazole (may be prepared as described in WO9743262) and 1-(1-methylethyl)-4-[(3S)-3-pyrrolidinylcarbonyl]hexahydro-1H-1,4-diazepine (may be prepared as described in Description 40) using an analogous process to that described in Example 26. LCMS electrospray (+ve) 398 (MH⁺). ¹H NMR (DMSO-d6) δ 1.29 (6H, m), 2.01-2.36 (4H, m), 2.52 (3H, m), 2.93-3.23 (2H, m), 3.33-3.80 (10H, m), 3.85-4.06 (2H, m), 6.67 (2H, d, J=8.8Hz), 7.76 (2H, d, J=8.8Hz), 10.20-10.41 (1H, m).

### Example 48

### 1-({(3S)-1-[4-(3-Ethyl-1,2,4-oxadiazol-5-yl)phenyl]-3-pyrrolidinyl}carbonyl)-4-(1-methylethyl)hexahydro-1H-1,4-diazepine hydrochloride (E48)

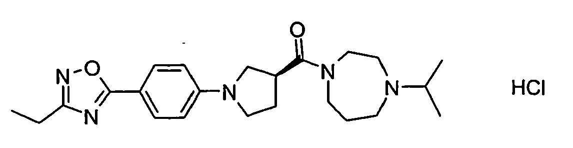

The title compound (E48) was prepared from 5-(4-bromophenyl)-3-ethyl-1,2,4-oxadiazole (may be prepared as described in Description 49) and 1-(1-methylethyl)-4-[(3S)-3-pyrrolidinylcarbonyl]hexahydro-1H-1,4-diazepine (may be prepared as described in Description 40) using an analogous process to that described in Example 26. LCMS electrospray (+ve) 412 (MH⁺). ¹H NMR (CDCl₃) δ 1.37 (3H, t, J=7.6Hz), 1.42 (6H, m), 2.14-2.36 (3H, m), 2.79 (2H, q, J=7.6), 2.81-2.95 (2H, m), 3.24 (1H, m), 3.40-3.88 (11H, m), 4.40 (1H, m), 6.59 (2H, d, J=8.8Hz), 7.96 (2H, d, J=8.8Hz), 12.41 (1H, m).

### Example 49

### 1-({(3S)-1-[4-(3-Ethyl-1,2,4-oxadiazol-5-yl)phenyl]-3-pyrrolidinyl}carbonyl)-4-(1-methylethyl)piperazine hydrochloride (E49)

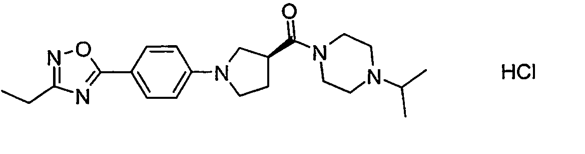

The title compound (E49) was prepared from 5-(4-bromophenyl)-3-ethyl-1,2,4-oxadiazole (may be prepared as described in Description 49) and 1-(1-methylethyl)-4-[(3S)-3-pyrrolidinylcarbonyl]piperazine (may be prepared as described in Description 6) using an analogous process to that described in Example 26. LCMS electrospray (+ve) 398 (MH⁺). ¹H NMR (DMSO-d6) δ 1.24-1.29 (9H, m), 2.13-2.23 (2H, m), 2.71 (2H, q, J=7.6), 2.93 (1H, m), 3.09 (2H, m) 3.33-3.63 (9H, m), 4.24 (1H, m), 4.53 (1H, m) 6.69 (2H, d, J=8.8Hz), 7.87 (2H, d, J=8.8Hz), 10.40 (1H, m).

### Example 50

### 1-[4-((3S)-3-{[4-(1-Methylethyl)-1-piperazinyl]carbonyl}-1-pyrrolidinyl)pheny)]-1-propanone hydrochloride (E50)

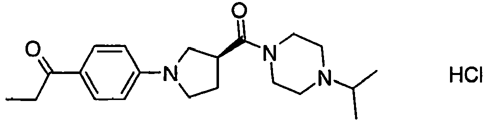

The title compound (E50) was prepared from 1-(4-bromophenyl)-1-propanone and 1-(1-methylethyl)-4-[(3*S*)-3-pyrrolidinylcarbonyl]piperazine (may be prepared as described in Description 6) using an analogous process to that described in Example 37. LCMS electrospray (+ve) 358 (MH⁺). ¹H NMR (CDCl₃) δ 1.05 (3H, t, J=7.2Hz), 1.29 (6H, m), 2.05-2.22 (2H, m), 2.88 (2H, q, J=7.2), 2.91 (1H, m), 3.01-3.22 (3H, m), 3.34-3.68 (8H, m), 4.25 (1H, m), 4,53 (1H, m), 6.58 (2H, d, J=8.8Hz), 7.81 (2H, d, J=8.8Hz), 10.85 (1H, bs).

### Example 51

### 1-Cyclobutyl-4-({(3S)-1-[6-(3-methyl-1,2,4-oxadiazol-5-yl)-3-pyridinyl]-3-pyrrolidinyl}carbonyl)piperazine hydrochloride (E51)

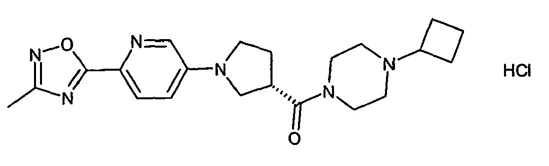

5-Bromo-2-(3-methyl-1,2,4-oxadiazol-5-yl)pyridine (may be prepared as described in Description 51) (0.305g) was added to a stirred solution of tris (dibenzylideneacetone)dipalladium (0) (0.83g) and 2-dicyclohexylphosphino-2'-(*N,N-*dimethylamino)biphenyl (0.1g) in DME (12ml) under argon at room temperature. Potassium phosphate (0.554g) and 1-cyclobutyl-4-[(3*S*)-3-pyrrolidinylcarbonyl]piperazine (may be prepared as described in Description 10) (0.3g in 4ml DME) were added sequentially and the mixture heated to 70°C for 4 hours. The mixture was filtered and then diluted with MeOH (20ml), loaded onto a 10g SCX cartridge and eluted with MeOH (20ml) followed by 2M NH₃/MeOH. The ammoniacal fractions were collected and evaporated and the residue (0.42g) was purified on a Waters Mass Directed Auto Preparative HPLC (eluent: 0.1 % formic acid in water and 0.1% formic acid acetonitrile; gradient 10-100%). The pure fractions were evaporated and treated with 1 N ethereal HCl (1 ml) in MeOH (2ml). The solvents were removed by evaporation to give the title compound (E51) as a solid (0.105g). ¹H NMR (methanol-d4) δ :1.33-2.00 (2H, m), 2.2-2.44 (5H, m), 2.46 (3H, m), 2.80-3.15 (3H, m), 3.5-3.8 (10H, m), 4.36-4.45 (1H, m), 4.65-4.72 (1H, m), 7.51 (1H, dd, J=9.2Hz), 8.12 (1H, s), 8.27 (1H, d, J=9.2Hz). LCMS electrospray (+ve) 397 (MH+).

### Example 52

### 1-Cyclobutyl-4-({(3S)-1-[3-fluoro-4-(3-methyl-1,2,4-oxadiazol-5-yl)phenyl]-3-pyrrolidinyl}carbonyl)piperazine hydrochloride (E52)

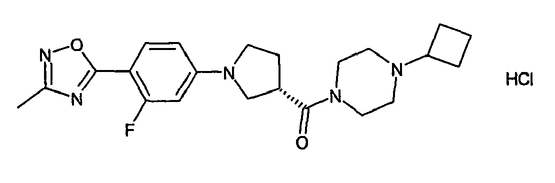

5-(2,4-Difluorophenyl)-3-methyl-1,2,4-oxadiazole (may be prepared as described in Description 53) (0.219g) and 1-cyclobutyl-4-[(3*S*)-3-pyrrolidinylcarbonyl]piperazine (0.263g) (may be prepared as described in Description 10) were dissolved in DMSO (5ml). Potassium carbonate (0.349g) was added and the mixture was stirred and heated at 160°C for 30 minutes. The reaction mixture was cooled, diluted with MeOH (20ml) and loaded onto a 10g SCX cartridge. The cartridge was washed with MeOH and eluted with 2M NH₃/MeOH. The ammoniacal fractions were combined and evaporated. The residue was partitioned between DCM (20ml) and water (20ml). The DCM layer was separated by passing the mixture through a phase separation column and was then evaporated to give an oil which was triturated (x2) with a hexane/ether mixture to give a solid. ¹H NMR (CDCl₃) δ 1.67-1.80 (2H, m), 1.83-1.98 (2H, m), 2.03-2.09 (2H, m), 2.21-2.39 (6H, m), 2.45 (3H, s), 2.75 (1H, m), 3.37-3.67 (9H, m), 6.30 (1H, dd, J=14.0 and 2.4Hz), 6.40 (1H, dd, J=8.8 and 2.4Hz), 7.89 (1H, t, J=8.8Hz).
The solid was dissolved in MeOH (2ml) and treated with 1 ml ethereal HCl. The mixture was blown down to give the title compound (E52) as a solid (0.104g). ¹H NMR (methanol-d4) δ:1.83-1.99 (2H, m), 2.17-2.38 (6H, m), 2.38 (3H,s), 2.59-3.30 (4H, m), 3.42-3.84 (8H, m), 4.10-4.60 (2H, 2 x m), 6.44 (1H, dd, J=14.4Hz), 6.54 (1H, dd,J=8.8Hz), 7.87 (1H, t, J=8.8Hz). LCMS electrospray (+ve) 414 (MH+).

### Example 53

### 1-(1-Methylethyl)-4-({(3S)-1-[6-(3-methyl-1,2,4-oxadiazol-5-yl)-3-pyridinyl]-3-pyrrolidinyl}carbonyl)hexahydro-1H-1,4-diazepine hydrochloride (E53)

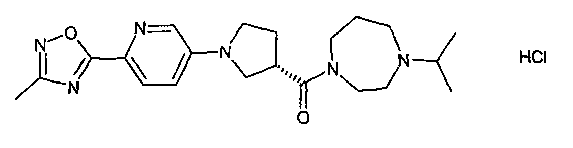

5-Bromo-2-(3-methyl-1,2,4-oxadiazol-5-yl)pyridine (may be prepared as described in Description 51) (0.2g) was added to a solution of 1-(1-methylethyl)-4-[(3S)-3-pyrrolidinylcarbonyl]hexahydro-1H-1,4-diazepine (may be prepared as described in Description 40) (0.203g) in DME (5ml). Tris(dibenzylideneacetone) dipalladium (0) (0.055g), 2-dicyclohexylphosphino-2'-(N,N-dimethylamino)-biphenyl (0.072g) and potassium phosphate (0.364g) were added and the reaction mixture was heated at 70°C with stirring for 4 hours. The reaction mixture was filtered and diluted with MeOH (20 ml) The diluted mixutre was then placed on a 10g SCX column. The column was washed with MeOH and eluted with 2NH₃ in MeOH. All ammoniacal fractions were combined and the solvent was evaporated. The residue was taken up in DMSO/MeCN and purified on MDAP. The relevant fractions from MDAP were combined and the solvent evaporated. The residue was then taken up in DCM (1ml) and treated with 1M HCl in ether (2 ml). The solvent was evaporated to afford the title compound (E53). ¹H NMR (methanol-d4) δ : 1.28-1.48 (6H, m), 2.21-2.40 (3H, m), 2.40-2.43 (1H, m), 2.48 (3H, s), 3.18-3.30 (2H, m), 3.51-4.25 (12H, m), 7.58 (1H, dd, J=9.2Hz), 8.13 (1H, s), 8.31 (1H ,d, J=9.2Hz). LCMS electrospray (+ve) 399 (MH+).

### Example 54

### 1-(1-Methylethyl)-4-({(3S)-1-[6-(3-methyl-1,2,4-oxadiazol-5-yl)-3-pyridinyl]-3-pyrrolidinyl}carbonyl)piperazine hydrochloride (E54)

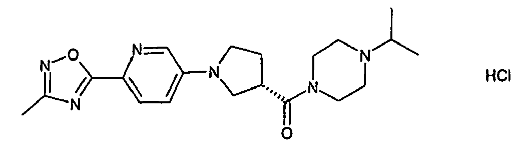

5-Bromo-2-(3-methyl-1,2,4-oxadiazol-5-yl)pyridine (may be prepared as described in Description 51) (0.3g) was dissolved in DME (5ml). Tris(dibenzylideneacetone) dipalladium (0) (0.082g), 2-dicyclohexylphosphino-2'-(N,N-dimethylamino)-biphenyl (0.1078g) and potassium phosphate (0.545g) were added, followed by the addition of 1-(1-methylethyl)-4-[(3S)-3-pyrrolidinylcarbonyl]piperazine (may be prepared as described in Description 6) (0.236g) in DME (5ml). The reaction mixture was heated at 70°C with stirring for 4 hours. After cooling, the reaction mixture was diluted with MeOH (20 ml) and filtered. The filtrate was then placed on a 10g SCX column. The column was washed with MeOH and eluted with 2NH₃ in MeOH. All ammoniacal fractions were combined and the solvent was evaporated. The residue was purified on MDAP. The relevant fractions from MDAP were combined and the solvent stripped. The residue was then taken up in MeOH (2ml) and treated with 1 M HCl in ether (1 ml). The solvent was blown down to afford the title compound (E53) (112mg). ¹H NMR (methanol-d4) δ : 1.41 (6H ,d, J=6.8Hz), 2.23-2.44 (2H,m), 2.45 (3H, s), 3.04-3.18 (3H, m), 3.55-3.85 (9H, m), 4.35-4.49 (1H, m), 4.70-4.80 (1H, m), 7.62 (1H, dd, J=9.2Hz), 8.14 (1H, s), 8.33 (1H ,d, J=9.2Hz). LCMS electrospray (+ve) 385 (MH+).

### Example 55

### 1-(1-Methylethyl)-4-({(3S)-1-[4-(4-methyl-1H-imidazol-1-yl)phenyl]-3-pyrrolidinyl}carbonyl)piperazine dihydrochloride (E55)

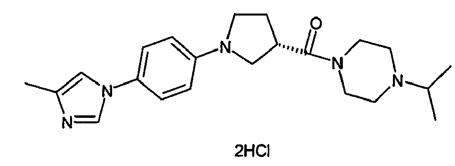

### Method A

A mixture of 4-(4-methylimidazol-1-yl)iodobenzene and 4-(4-methylimidazol-1-yl)bromobenzene (may be prepared as described in Description 54) (1.58g) was added to a solution of tris(dibenzylideneacetone) dipalladium(0) (0.152g) and 2-dicyclohexylphosphino-2'-(*N,N*-dimethylamino)-biphenyl (0.196g) in MeCN (10ml). 1-(1-Methylethyl)-4-[(3S)-3-pyrrolidinylcarbonyl]piperazine (may be prepared as described in Description 6) (1g in 10ml MeCN) was then added followed by potassium phosphate (1.88g). The mixture was heated to 80°C for 5h. After cooling, the mixture was diluted with MeOH, loaded onto 2x10g SCX cartridges and eluted with MeOH (50ml) followed by 2M NH₃/MeOH (50ml). The ammoniacal fractions were evaporated and the residue was triturated with ether (x2) and crystallised from EtOH (x2). The residue was then dissolved in a minimum volume of MeOH and 1 M HCl/diethylether (5ml) added. The solvents were evaporated and the residue re-evaporated from acetone (x2) to afford the title compound (E55) as a white solid (0.21g). LCMS electrospray (+ve) 382 (MH⁺). ¹H NMR (DMSO-d6) δ 1.29 (6H, d, J=6.4Hz), 2.07-2.12 (1H, m), 2.19-2.22 (1H, m), 2.33 (3H, s), 2.85-2.91 (1H, m), 3.00-3.12 (1H, m), 3.15-3.25 (1H, m), 3.30-3.75 (9H, m), 4.24 (1H, m), 4.51 (1H, m), 6.70 (2H, d, J=8.8Hz), 7.52 (2H, d, J=8.8Hz), 7.87 (1H, s), 9.46 (1H, s), 11.25 (1H, bs), 14.90 (1H, bs).

### Method B

1-(1-Methylethyl)-4-[(3S)-3-pyrrolidinylcarbonyl]piperazine (may be prepared as described in Description 6) (0.25g in 2.5 ml MeCN) and potassium phosphate (0.47g) were added to a stirred mixture of tris(dibenzylideneacetone) dipalladium(0) (0.076g), 2-dicyclohexylphosphino-2'-(*N,N*-dimethylamino)-biphenyl (0.098g), MeCN (2.5ml) and a mixture of 4-(4-methylimidazol-1-yl)iodobenzene and 4-(4-methylimidazol-1-yl)bromobenzene (may be prepared as described in Description 54) (0.395g). The mixture was heated to 80°C for 5h under argon. After cooling, the mixture was diluted with MeOH, loaded onto a 10g SCX cartridge and eluted with MeOH (50ml) followed by 2M NH₃/MeOH (50ml). The ammoniacal fractions were evaporated and the residue was washed with ether (x2) and crystallised from EtOH. The residue was then dissolved in a minimum volume of DCM and 0.5ml 1M HCl/diethylether was added. The solvents were removed and the residue was evaporated from acetone (x2) to afford the title compound (E55) as a white solid (0.072g). LCMS electrospray (+ve) 382 (MH⁺). ¹H NMR (DMSO-d6) δ 1.29 (6H, d, J=6.4Hz), 2.07-2.12 (1H, m), 2.19-2.22.(1H, m), 2.33 (3H, s), 2.85-2.91 (1H, m), 3.00-3.12 (1H, m), 3.15-3.25 (1H, m), 3.30-3.75 (9H, m), 4.24 (1H, m), 4.51 (1H, m), 6.70 (2H, d, J=8.8Hz), 7.52 (2H, d, J=8.8Hz), 7.87 (1H, s), 9.46 (1H, s), 11.25 (1H, bs), 14.90 (1H, bs).

### Example 55 A

### 1-(1-Methylethyl)-4-({(3S)-1-[4-(4-methyl-1H-imidazol-1-yl)phenyl]-3-pyrrolidinyl}carbonyl)piperazine dihydrochloride (E55A)

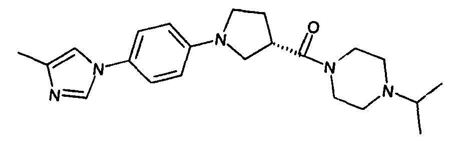

1-(1-Methylethyl)-4-({(3S)-1-[4-(4-methyl-1H-imidazol-1-yl)phenyl]-3-pyrrolidinyl}carbonyl)piperazine dihydrochloride (may be prepared as described in Example 55) (0.145g) was dissolved in MeOH and loaded onto a 10g SCX cartridge. The cartridge was eluted with MeOH (60ml) followed by 2M NH₃/MeOH (60 ml). The ammoniacal fractions were evaporated to dryness. The residue was then dissolved in DCM and washed with an aqueous solution of K₂CO₃. The DCM layer was dried (MgSO₄) and evaporated. The residue was then crystallised from ethanol to afford the title compound (E55A) as a crystalline white solid (0.04g). LCMS: 382 (MH+) NMR (CDCl3):1.06 (6H, d, J = 6.4Hz), 2.21-2.38 (5H, m), 2.50-2.57 (4H, m), 2.70-2.76 (1H, m), 3.35-3.59 (7H, m), 3.65-3.68 (2H, m), 6.58 (2H, d, J=8.8Hz), 6.89 (1H, s), 7.19 (2H, d, J=8.8Hz), 7.61 (1H,s).

### Example 56

### 1-(1-Methylethyl)-4-({(3S)-1-[4-(2-methy)-1H-imidazol-1-yl)phenyl]-3-pyrrolidinyl}carbonyl)piperazine dihydrochloride (E56)

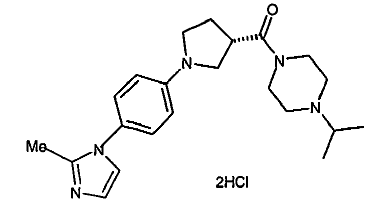

The title compound (E56) was prepared using an analogous process to that described in Example 37 from 1-(1-methylethyl)-4-[(3*S*)-3-pyrrolidinylcarbonyl]piperazine (may be prepared as described in Description 6) and 4-(2-methylimidazol-1-yl)iodobenzene (may be prepared as described in Example 8A of WO 96/11911), and the free base product was converted into the dihydrochloride salt. LCMS Electrospray (+ve) 382 (MH⁺). NMR (DMSO) 1.29 (6H, d, J=6.4Hz), 2.04-2.29 (2H, m), 2.52 (3H, s), 2.93 (1H, m), 3.16 (1H, m), 3.21 (1H, m), 3.31-3.69 (9H, m), 4.24 (1H, m), 4.51 (1H, m), 6.70 (2H, d, J=8.8Hz), 7.37 (2H, d, J=8.8Hz), 7.72-7.75 (2H, m), 11.15 (1H, bs), 14.65 (1H, bs).

### Example 57

### 4-((3S)-3-{[(3S)-3-Methyt-4-(1-methylethyl)-1-piperazinyl]carbonyl}-1-pyrrolidinyl)benzonitrile hydrochloride (E57)

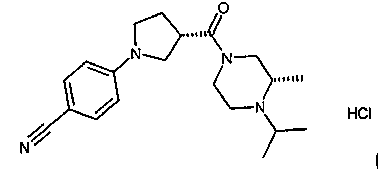

(2S)-1-(1-Methylethyl)-2-methyl-4-[(3S)-3-pyrrolidinylcarbonyl]piperazine (may be prepared as described in Description 21) (0.12g), 4-fluorobenzonitrile (0.121g) and potassium carbonate (0.138g) were heated in DMSO (1ml) at 120°C under argon for 2h. After cooling, the mixture was divided into 2 equal portions and each portion applied to a 10g SCX column. After initial elution (under gravity) with MeOH, the product was eluted with 2M NH₃ in MeOH and the combined ammoniacal eluates were evaporated to give a gum, which was further purified by chromatography on silica gel eluting with 2M methanolic NH₃/DCM (0 to 3.5% in 0.5% steps). The freebase was thereby obtained as a yellow gum (0.106g). ¹H NMR (CDCl₃) 0.89 (3H, m), 1.06-1.16 (6H, m), 2.20-2.37 (3H, m), 2.50-2.86, 2.94-3.07 and 3.20-3.81 (3xm, total 11H), 4.21-4.31 (1H, m), 6.51 (2H, d, J = 8Hz), 7.46 (2H, d, J = 8Hz). This was dissolved in DCM (3ml) and treated with 1M HCl in Et₂O (3ml). The solvents were blown off in a stream of argon and the residual material was dried *in vacuo* for 48h at 40°C to afford the title hydrochloride salt (E57) as a solid foam (0.111g). LCMS electrospray (+ve) 341 (MH⁺).

### Examples 58-59 (E58-E59)

Example 58 was prepared using an analogous process to that described in Example 51 from 5-bromo-2-(3-methyl-1,2,4-oxadiazol-5-yl)pyridine (may be prepared as described in Description 51) and 1-(cyclopropylmethyl)-4-[(3S)-pyrrolidin-3-ylcarbonyl]-hexahydro-1*H*-1,4-diazepine (may be prepared as described in Description 16). Example 59 was prepared using an analogous process to that described in Example 51 from 5-bromo-2-(3-methyl-1,2,4-oxadiazol-5-yl)pyridine (may be prepared as described in Description 51) and 1-(cyclopropylmethyl)-4-[(3S)-3-pyrrolidinylcarbonyl]piperazine (may be prepared as described in Description 28). The free base products were converted into the corresponding hydrochloride salts in DCM (1ml) with 1 N ethereal HCl (2ml) followed by evaporation of solvents. Examples displayed ¹H NMR and mass spectral data consistent with structure.

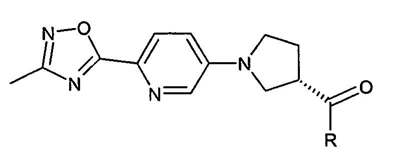

| **Example No** | **Name** | **R** | **Mass Spectrum (ES⁺)** |
|---|---|---|---|
| **E58** | 1-(Cyclopropylmethyl)-4-({(3*S*)-1-[6-(5-methyl-1,2,4-oxadiazol-3-yl)-3-pyridinyl]-3-pyrrolidinyl}carbonyl)hexahydro-1*H*-1,4-diazepine hydrochloride | 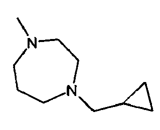 | 411 (MH⁺) |
| **E59** | 1-(Cyclopropylmethyl)-4-({(3S)-1-[6-(5-methyl-1,2,4-oxadiazol-3-yl)-3-pyridinyl]-3-pyrrolidinyl}carbonyl)piperazine hydrochloride | 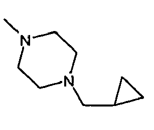 | 397 (MH⁺) |

### Example 60

### 1-Ethyl-4-({(3S)-1-[4-(3-methyl-1,2,4-oxadiazol-5-yl)phenyl]-3-pyrrolidinyl}carbonyl)piperazine hydrochloride (E60)

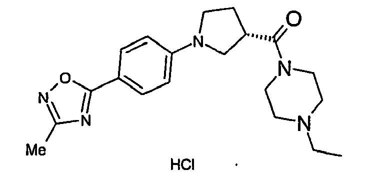

The title compound (E60) was prepared from 1-ethyl-4-[(3S)-3-pyrrolidinylcarbonyl] piperazine (may be prepared as described in Description 57) (0.052g) and 5-(4-bromophenyl)-3-methyl-1,2,4-oxadiazole (may be prepared as described in Description D23) (0.058g) using an analogous process to that described in Example 51. LCMS electrospray (+ve) 370 (MH⁺). ¹H NMR δ: (methanol d4) δ: 1.35-1.45 (3H, t), 2.18-2.34 (2H, m), 2.36 (3H, s), 2.95-3.23 (6H, m), 3.45-3.75 (7, m), 4.35-4.49 (1H,m), 4.65-4.77 (1H, m), 6.70 (2H, d, J=8.0Hz). 7.90 (2H, d, J=8.0Hz).

### Abbreviations

- 12M silica columns: pre-packed FLASH 12+M silica cartridges supplied by Biotage.
- Biotage SP1: column chromatography system supplied by Biotage.
- DBU: 1,8-diazabicyclo[5.4.0]undec-7-ene
- dioxane: 1,4-dioxane
- DCM: dichloromethane
- DMSO: dimethylsulfoxide
- DME: dimethoxyethane
- DMF: N,N-dimethylformamide
- EDC/EDCl: 1-(3-dimethylaminopropyl)-3-ethylcarbodiimide hydrochloride
- EtOAc: ethyl acetate
- Flash 75: Flash 75 chromatography system supplied by Biotage.
- FM (II): Flashmaster II automated chromatography system supplied by Argonaut.
- HOAT: 1-hydroxy-7-azabenzotriazole
- HOBT: 1-hydroxybenzotriazole
- h: hour
- KP-NH: Amino-bonded silica cartridges supplied by Biotage
- min: minutes
- NMP: 1-methyl-2-pyrrolidinone
- THF: tetrahydrofuran
- SCX: strong cation exchange
- Size E column: glass chromatography column (10cm x 40cm) capable of holding up to 1Kg of silica gel.

All publications, including but not limited to patents and patent applications, cited in this specification are herein incorporated by reference as if each individual publication were fully set forth.

### Biological Data

A membrane preparation containing histamine H3 receptors may be prepared in accordance with the following procedures:

### (i) Generation of histamine H3 cell line

DNA encoding the human histamine H3 gene (Huvar, A. et al. (1999) Mol. Pharmacol. 55(6), 1101-1107) was cloned into a holding vector, pCDNA3.1 TOPO (InVitrogen) and its cDNA was isolated from this vector by restriction digestion of plasmid DNA with the enzymes BamH1 and Not-1 and ligated into the inducible expression vector pGene (InVitrogen) digested with the same enzymes. The GeneSwitch^{™} system (a system where in transgene expression is switched off in the absence of an inducer and switched on in the presence of an inducer) was performed as described in US Patent nos: 5,364,791; 5,874,534; and 5,935,934. Ligated DNA was transformed into competent DH5α E. coli host bacterial cells and plated onto Luria Broth (LB) agar containing Zeocin^{™} (an antibiotic which allows the selection of cells expressing the sh ble gene which is present on pGene and pSwitch) at 50µg ml⁻¹. Colonies containing the re-ligated plasmid were identified by restriction analysis. DNA for transfection into mammalian cells was prepared from 250ml cultures of the host bacterium containing the pGeneH3 plasmid and isolated using a DNA preparation kit (Qiagen Midi-Prep) as per manufacturers guidelines (Qiagen).
CHO K1 cells previously transfected with the pSwitch regulatory plasmid (InVitrogen) were seeded at 2x10e6 cells per T75 flask in Complete Medium, containing Hams F12 (GIBCOBRL, Life Technologies) medium supplemented with 10% v/v dialysed foetal bovine serum, L-glutamine, and hygromycin (100µg ml⁻¹), 24 hours prior to use. Plasmid DNA was transfected into the cells using Lipofectamine plus according to the manufacturers guidelines (InVitrogen). 48 hours post transfection cells were placed into complete medium supplemented with 500µg ml⁻¹ Zeocin^{™}.
10-14 days post selection 10nM Mifepristone (InVitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1x 10e7 cells were examined for receptor expression by staining with a rabbit polyclonal antibody, 4a, raised against the N-terminal domain of the histamine H3 receptor, incubated on ice for 60 minutes, followed by two washes in sorting medium. Receptor bound antibody was detected by incubation of the cells for 60 minutes on ice with a goat anti rabbit antibody, conjugated with Alexa 488 fluorescence marker (Molecular Probes). Following two further washes with Sorting Medium, cells were filtered through a 50µm Filcon^{™} (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500µg ml⁻¹ Zeocin^{™} and allowed to expand before reanalysis for receptor expression via antibody and ligand binding studies. One clone, 3H3, was selected for membrane preparation.

### (ii) Membrane preparation from cultured cells

All steps of the protocol are carried out at 4°C and with pre-cooled reagents. The cell pellet is resuspended in 10 volumes of homogenisation buffer (50mM N-2-hydroxyethylpiperazine-N'-2-ethanesulfonic acid (HEPES), 1mM ethylenediamine tetra-acetic acid (EDTA), pH 7.4 with KOH, supplemented with 10e-6M leupeptin (acetyl-leucyl-leucyl-arginal; Sigma L2884), 25µg/ml bacitracin (Sigma B0125), , 1mM phenylmethylsulfonyl fluoride (PMSF) and 2x10e-6M pepstain A (Sigma)). The cells are then homogenised by 2 x 15 second bursts in a 1 litre glass Waring blender, followed by centrifugation at 500g for 20 minutes. The supernatant is then spun at 48,000g for 30 minutes. The pellet is resuspended in homogenisation buffer (4X the volume of the original cell pellet) by vortexing for 5 seconds, followed by homogenisation in a Dounce homogeniser (10-15 strokes). At this point the preparation is aliquoted into polypropylene tubes and stored at -80°C.

A histamine H1 cell line may be generated in accordance with the following procedure:

### (iii) Generation of histamine H1 cell line

The human H1 receptor was cloned using known procedures described in the literature [Biochem. Biophys. Res. Commun. 1994, 201(2), 894]. Chinese hamster ovary cells stably expressing the human H1 receptor were generated according to known procedures described in the literature [Br. J. Pharmacol. 1996, 117(6), 1071].

Compounds of the invention may be tested for in vitro biological activity in accordance with the following assays:

### (I) Histamine H3 functional antagonist assay (method A)

For each compound being assayed, in a solid white 384 well plate, is added:-
(a) 5µl of test compound diluted to the required concentration in 10% DMSO (or 5µl 10% DMSO as a control); and
(b) 30µl bead/membrane/GDP mix prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20mM N-2-Hydroxyethylpiperazine-N'-2-ethanesulfonic acid (HEPES) + 100mM NaCl + 10mM MgCl₂, pH7.4 NaOH) to give a final volume of 30µl which contains 5µg protein and 0.25mg bead per well, incubating at 4°C for 30 minutes on a roller and, just prior to addition to the plate, adding 10µM final concentration of guanosine 5' diphosphate (GDP) (Sigma; diluted in assay buffer).
   The plates were then incubated at room temperature for 30 minutes on a shaker followed by addition of:
(c) 15 µl 0.38nM [³⁵S]-GTPγS (Amersham; Radioactivity concentration=37MBq/ml; Specific activity=1160Ci/mmol), histamine (at a concentration that results in the final assay concentration of histamine being EC₈₀).
   After 2-6 hours, the plate is centrifuged for 5 min at 1500 rpm and counted on a Viewlux counter using a 613/55 filter for 5 min/plate. Data is analysed using a 4-parameter logistical equation. Basal activity used as minimum i.e. histamine not added to well.

### (II) Histamine H3 functional antagonist assay (method B)

For each compound being assayed, in a solid white 384 well plate, is added:-
(a) 0.5µl of test compound diluted to the required concentration in DMSO (or 0.5µl DMSO as a control);
(b) 30µl bead/membrane/GDP mix prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20mM N-2-Hydroxyethylpiperazine-N'-2-ethanesulfonic acid (HEPES) + 100mM NaCl + 10mM MgCl₂, pH7.4 NaOH) to give a final volume of 30µl which contains 5µg protein and 0.25mg bead per well, incubating at room temperature for 60 minutes on a roller and, just prior to addition to the plate, adding 10µM final concentration of guanosine 5' diphosphate (GDP) (Sigma; diluted in assay buffer);
(c) 15µl 0.38nM [³⁵S]-GTPγS (Amersham; Radioactivity concentration=37MBq/ml; Specific activity=1160Ci/mmol), histamine (at a concentration that results in the final assay concentration of histamine being EC₈₀).
After 2-6 hours, the plate is centrifuged for 5 min at 1500 rpm and counted on a Viewlux counter using a 613/55 filter for 5 min/plate. Data is analysed using a 4-parameter logistical equation. Basal activity used as minimum i.e. histamine not added to well.

### (III) Histamine H1 functional antagonist assay

The histamine H1 cell line was seeded into non-coated black-walled clear bottom 384-well tissue culture plates in alpha minimum essential medium (Gibco /Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and maintained overnight at 5% CO₂, 37°C.

Excess medium was removed from each well to leave 10µl. 30µl loading dye (250µM Brilliant Black, 2µM Fluo-4 diluted in Tyrodes buffer + probenecid (145 mM NaCl, 2.5 mM KCI, 10mM HEPES, 10mM D-glucose, 1.2 mM MgCl₂, 1.5 mM CaCl₂, 2.5 mM probenecid, pH adjusted to 7.40 with NaOH 1.0 M)) was added to each well and the plates were incubated for 60 minutes at 5% CO₂, 37°C.

10µl of test compound, diluted to the required concentration in Tyrodes buffer + probenecid (or 10µl Tyrodes buffer + probenecid as a control) was added to each well and the plate incubated for 30 min at 37°C, 5% CO₂. The plates were then placed into a FLIPR^{™} (Molecular Devices, UK) to monitor cell fluorescence (λₑₓ= 488 nm, λ_{EM}= 540 nm) in the manner described in Sullivan *et al.* (In: Lambert DG (ed.), Calcium Signaling Protocols, New Jersey: Humana Press, 1999, 125-136) before and after the addition of 10µl histamine at a concentration that results in the final assay concentration of histamine being EC₈₀.

Functional antagonism is indicated by a suppression of histamine induced increase in fluorescence, as measured by the FLIPR^{™} system (Molecular Devices). By means of concentration effect curves, functional affinities are determined using standard pharmacological mathematical analysis.

### Results

The compounds of Examples E17, E17A, E17B, E22 and E23 were tested in the histamine H3 functional antagonist assay (method A). The results are expressed as functional pKᵢ (fpkᵢ) values. A functional pKi is the negative logarithm of the antagonist equilibrium dissociation constant as determined in the H3 functional antagonist assay using membrane prepared from cultured H3 cells. The results given are averages of a number of experiments. These compounds exhibited antagonism >8.0 fpkᵢ. More particularly, the compounds of Examples E17, E17A, E17B and E23 exhibited antagonism > 9.5 fpkᵢ.

The compounds of Examples E1-3, E4-5, E6, E7-16, E17, E18, E18A, E18B, E19, E19A, E19B, E20, E20A, E20B, E21, E21A, E21B, E23-31, E32A, E33-55 and E56-59 were tested in the histamine H3 functional antagonist assay (method B). Again, the results are expressed as functional pKᵢ (fpkᵢ) values and are averages of a number of experiments. These compounds exhibited antagonism >8.0 fKᵢ. More particularly, the compounds of Examples E2, E3, E5, E6, E8-13, E17, E18, E18A, E18B, E19B, E20, E20B, E21, E21 B, E24, E25, E27-30, E32A, E33-34, E36, E40, E41, E43, E47-49, E51-55 and E58 exhibited antagonism ≥ 9.5 fpKᵢ.

The compounds of Examples E1-3, E4-5, E6, E7-17, E17A, E17B, E18, E18A, E18B, E19, E19A, E19B, E20, E20A, E20B, E21, E21A, E21B, E22-31, E32A, E33-55 and E56-59 were tested in the histamine H1 functional antagonist assay. The results are expressed as functional pKᵢ (fpkᵢ) values and are averages of a number of experiments. The functional pKi may be derived from the negative logarithm of the plC50 (concentration producing 50% inhibition) in the H1 functional antagonist assay according to the Cheng-Prusoff equation (Cheng, Y.C. and Prusoff, W. H., 1973, Biochem. Pharmacol. 22, 3099-3108.). All compounds tested exhibited antagonism < 6.5 fpKᵢ.

## Claims

1. A compound of formula (I) or a pharmaceutically acceptable salt thereof: 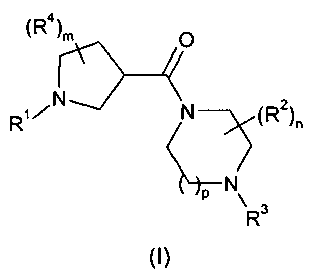 wherein:
R¹ represents aryl, heteroaryl, -aryl-X-C₃₋₇ cycloalkyl, -heteroaryl-X-C₃₋₇ cycloalkyl, -aryl-X-aryl, -aryl-X-heteroaryl, -aryl-X-heterocyclyl, -heteroaryl-X-heteroaryl, -heteroaryl-X-aryl or -heteroaryl-X-heterocyclyl;
wherein said aryl, heteroaryl and heterocyclyl groups of R¹ may be optionally substituted by one or more substituents which may be the same or different, and which are selected from the group consisting of halogen, hydroxy, cyano, nitro, oxo, haloC₁₋₆ alkyl, haloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, -COC₁₋₆ alkyl, -CO-haloC₁₋₆ alkyl, -COC₁₋₆ alkyl-cyano, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -NR¹⁵R¹⁶, -CONR¹⁵R¹⁶, -NR¹⁵COR¹⁶, -C(R¹⁵)=NOR¹⁶, -NR¹⁵SO₂R¹⁶ or -SO₂NR¹⁵R¹⁶,
wherein R¹⁵ and R¹⁶ independently represent hydrogen or C₁₋₆ alkyl or together form a heterocyclic ring;
X represents a bond, O, CO, SO₂, OCH₂ or CH₂O;
each R² and R⁴ independently represents C₁₋₄ alkyl;
R³ represents C₂₋₆ alkyl, C₃₋₆ alkenyl, C₃₋₆ alkynyl, C₃₋₆ cycloalkyl, C₅₋₆ cycloalkenyl or -C₁₋₄alkyl-C₃₋₆ cycloalkyl;
wherein said C₃₋₆ cycloalkyl groups of R³ may be optionally substituted by one or more substituents which may be the same or different, and which are selected from the group consisting of halogen, C₁₋₄ alkyl or trifluoromethyl groups;
m and n independently represent 0, 1 or 2;
p represents 1 or 2;
or a solvate thereof.

2. A compound according to claim 1, wherein the stereochemistry of the carbon atom in the pyrrolidine group that is attached to the carbonyl group has the S configuration.

3. A compound according to claim 1 or claim 2, wherein R¹ represents:
- aryl, optionally substituted by one or more -COC₁₋₆ alkyl or cyano groups;
- aryl-X-heteroaryl, optionally substituted on the aryl group by a halogen, and/or optionally substituted on the heteroaryl by a C₁₋₆ alkyl group;
- aryl-X-heterocyclyl, optionally substituted by one or more oxo groups;
- heteroaryl optionally substituted by one or more C₁₋₆ alkyl or haloC₁₋₆ alkyl groups; or
- heteroaryl-X-heteroaryl optionally substituted by one or more C₁₋₆ alkyl groups.

4. A compound according to claim 3, wherein R¹ represents
- aryl optionally substituted by one or more -COC₁₋₆ alkyl or cyano groups;
- aryl-X-heteroaryl optionally substituted by a halogen or C₁₋₆ alkyl group; or
- heteroaryl optionally substituted by one or more haloC₁₋₆ alkyl groups.

5. A compound according to any preceding claim, wherein X represents a bond.

6. A compound according to any preceding claim, wherein m represents 0.

7. A compound according to any preceding claim, wherein n represents 0 or 1.

8. A compound according to any preceding claim, wherein p represents 1 or 2.

9. A compound according to any preceding claim, wherein R³ represents C₂₋₆ alkyl, C₃₋₆ cycloalkyl or -C₁₋₄alkyl-C₃₋₆ cycloalkyl.

10. A compound according to claim 1 which is:
1-(1-Methylethyl)-4-({(3*S*)-1-[6-(trifluoromethyl)-3-pyridinyl]-3-pyrrolidinyl} carbonyl)piperazine;
1-[4-((3*S*)-3-{[4-(1-Methylethyl)-1-piperazinyl]carbonyl}-1-pyrrolidinyl) phenyl]ethanone; 1-(1-Methylethyl)-4-({(3*S*)-1-[4-(3-methyl-1,2,4-oxadiazo)-5-yl)phenyl]-3-pyrrolidinyl}carbonyl)piperazine;
(2*S*)-2-Methyl-1-(1-methylethyl)-4-({(3*S*)-1-[6-(trifluoromethyl)-3-pyridinyl]-3-pyrrolidinyl}carbonyl)piperazine;
1-[4-((3*S*)-3-{[(3*S*)-3-Methyl-4-(1-methylethyl)-1-piperazinyl]carbonyl}-1-pyrrolidinyl)phenyl]ethanone;
(2*S*)-2-Methyl-1-(1-methylethyl)-4-({(3*S*)-1-[4-(3-methyl-1,2,4-oxadiazol-5-yl)phenyl]-3-pyrrolidinyl}carbonyl)piperazine;
1-(1-Ethylpropyl)-4-({(3*S*)-1-[6-(trifluoromethyl)-3-pyridinyl]-3-pyrrolidinyl} carbonyl)piperazine;
1-[4-((3S)-3-{[4-(1-Ethylpropyl)-1-piperazinyl]carbonyl}-1-pyrrolidinyl) phenyl]ethanone;
1-(1-Ethylpropyl)-4-({(3*S*)-1-[4-(3-methyl-1,2,4-oxadiazol-5-yl)phenyl]-3-pyrrolidinyl}carbonyl)piperazine;
1-[4-((3*S*)-3-{[4-(Cyclopropylmethyl)hexahydro-1*H*-1,4-diazepin-1-yl]carbonyl}-1-pyrrolidinyl)phenyl]ethanone;
1-(Cyclopropylmethyl)-4-({(3*S*)-1-[4-(3-methyl-1,2,4-oxadiazol-5-yl)phenyl]-3-pyrrolidinyl}carbonyl)hexahydro-1*H*-1,4-diazepine;
1-Cyclobutyl-4-({(3*S*)-1-[6-(trifluoromethyl)-3-pyridinyl]-3-pyrrolidinyl}carbonyl)piperazine; 1-Cyclobutyl-4-({(3*S*)-1-[4-(3-methyl-1,2,4-oxadiazol-5-yl)phenyl]-3-pyrrolidinyllcarbonyl)piperazine;
1-(Cyclopropylmethyl)-4-({(3*S*)-1-[6-(trifluoromethyl)-3-pyridinyl]-3-pyrrolidinyl}carbonyl)piperazine;
1-[4-((3*S*)-3-{[4-(Cyclopropytmethyl)-1-piperazinyl]carbonyl}-1-pyrrolidinyl)phenyl]ethanone;
1-(Cyclopropylmethyl)-4-({(3*S*)-1-[4-(3-methyl-1,2,4-oxadiazol-5-yl)phenyl]-3-pyrrolidinyl}carbonyl)piperazine;
(R,S)-1-Cyclobutyl-4-({1-[6-(trifluoromethyl)-3-pyridinyl]-3-pyrrolidinyl}carbonyl)hexahydro-1*H*-1,4-diazepine;
1-Cyclobutyl-4-({(3*R*)-1-[6-(trifluoromethyl)-3-pyridinyl]-3-pyrrolidinyl}carbonyl)hexahydro-1H-1,4-diazepine;
1-Cyclobutyl-4-({(3*S*)-1-[6-(trifluoromethyl)-3-pyridinyl]-3-pyrrolidinyl}carbonyl) hexahydro -1*H*-1,4-diazepine;
1-Cyclobutyl-4-({(3*R*)-1-[4-(3-methyl-1,2,4-oxadiazol-5-yl)phenyl]-3-pyrrolidinyl}carbonyl)hexahydro-1*H*-1,4-diazepine;
1-Cyclobutyl-4-({(3*S*)-1-[4-(3-methyl-1,2,4-oxadiazol-5-yl)phenyl]-3-pyrrolidinyl}carbonyl) hexahydro-1*H*-1,4-diazepine;
6-{(3*R*)-3-[(4-Cyclobutylhexahydro-1*H*-1,4-diazepin-1-yl)carbonyl]-1-pyrrolidinyl}-2-methylquinoline;
6-{(3*S*)-3-[(4-Cyclobutylhexahydro-1*H*-1,4-diazepin-1-yl)carbonyl]-1-pyrrolidinyl}-2-methylquinoline;
1-Cyclobutyl-4-({(3*R*)-1-[2-fluoro-4-(3-methyl-1,2,4-oxadiazol-5-yl)phenyl]-3-pyrrolidinyl}carbonyl)hexahydro-1*H*-1,4-diazepine;
1-Cyclobutyl-4-({(3*S*)-1-[2-fluoro-4-(3-methyl-1,2,4-oxadiazol-5-yl)phenyl]-3-pyrrolidinyl}carbonyl)hexahydro-1*H*-1,4-diazepine;1-(4-{(3*R*)-3-[(4-Cyclobutylhexahydro-1*H-*1,4-diazepin-1-yl)carbonyl]-1-pyrrolidinyl}phenyl)ethanone;
1-(4-{(3*S*)-3-[(4-Cyclobutylhexahydro-1*H*-1,4-diazepin-1-yl)carbonyl]-1-pyrrolidinyl}phenyl)ethanone;
(R,S)-1-(1-Methylethyl)-4-({1-[6-(trifluoromethyl)-3-pyridinyl]-3-pyrrolidinyl}carbonyl)hexahydro-1*H*-1,4-diazepine;
(R,S)-1-Cyclobutyl-4-({1-[2-(trifluoromethyl)-4-pyridinyl]-3-pyrrolidinyl}carbonyl)hexahydro-1*H*-1,4-diazepine; 1-({(3S)-1-[3-Fluoro-4-(3-methyl-1,2,4-oxadiazol-5-yl)phenyl]-3-pyrrolidinyl}carbonyl)-4-(1-methylethyl)piperazine;
1-({(3*S*)-1-[2-Fluoro-4-(3-methyl-1,2,4-oxadiazol-5-yl)phenyl]-3-pyrrolidinyl)carbonyl)-4-(1-methylethyl)piperazine;
1-(-Methylethyl)-4-({(3*S*)-1-[6-(trifluoromethyl)-3-pyridinyl]-3-pyrrolidinyl}carbonyl)hexahydro-1*H*-1,4-diazepine;
1-[4-((3*S*)-3-([4-(1-Methylethyl)hexahydro-1*H*-1,4-diazepin-1-yl]carbonyl}-1-pyrrolidinyl)phenyl]ethanone;
1-(1-Methylethyl)-4-({(3*S*)-1-[4-(3-methyl-1,2,4-oxadiazol-5-yl)phenyl]-3-pyrrolidinyl}carbonyl)hexahydro-1*H*-1,4-diazepine;
1-({(3*S*)-1-[3-Fluoro-4-(3-methyl-1,2,4-oxadiazol-5-yl)phenyl]-3-pyrrolidinyl}carbonyl)-4-(1-methylethyl)hexahydro-1*H*-1,4-diazepine;
1-(1-Methylethyl)-4-({(3*S*)-1-[4-(5-methyl-1,3,4-oxadiazol-2-yl)phenyl]-3-pyrrolidinyl}carbonyl)piperazine;
1-(1-Methylethyl)-4-({(3*S*)-1-[3-(3-methyl-1,2,4-oxadiazol-5-yl)phenyl]-3-pyrrolidinyl}carbonyl)piperazine;
(R, S)-1-(1-Methylethyl)-4-({1-[4-(2-methyl-1,3-oxazol-4-yl)phenyl]-3-pyrrolidinyl}carbonyl)piperazine;
1-(1-Methylethyl)-4-({(3*S*)-1-[4-(2-methyl-1,3-oxazol-4-yl)phenyl]-3-pyrrolidinyl}carbonyl)piperazine;1-(1-Methylethyl)-4-({(3*S*)-1-[4-(5-methyl-1,2,4-oxadiazol-3-yl)phenyl]-3-pyrrolidinyl}carbonyl)piperazine;1-(1-Methylethyl)-4-({(3*S*)-1-[4-(2-methyl-1,3-oxazol-5-yl)phenyl]-3-pyrrolidinyl}carbonyl)piperazine;3-((3*S*)-3-{[4-(1-Methylethyl)-1-piperazinyl]carbonyl}-1-pyrrolidinyl)benzonitrile;
4-((3*S*)-3-{[4-(1-Methylethyl)-1-piperazinyl]carbonyl}-1-pyrrolidinyl)benzonitrile; 5-((3*S*)-3-{[4-(1-Methylethyl)-1-piperazinyl]carbonyl}-1-pyrrolidinyl)-2-(trifluoromethyl)pyrimidine;
1-(1-Methylethyl)-4-({(3*R*)-1-[4-(3-methyl-1,2,4-oxadiazol-5-yl)phenyl]-3-pyrrolidinyl}carbonyl)piperazine;
1-Cyclobutyl-4-({(3*R*)-1-[4-(3-methyl-1,2,4-oxadiazol-5-yl)phenyl]-3-pyrrolidinyl}carbonyl)piperazine;
1-(1-Methylethyl)-4-({(3*S*)-1-[4-(5-phenyl-1,3,4-oxadiazol-2-yl)phenyl]-3-pyrrolidinyl}carbonyl)piperazine;
1-({(3S)-1-[4-(5-Isoxazolyl)phenyl]-3-pyrrolidinyl)carbonyl-4-(1-methylethyl)piperazine;
1-(1-Methylethyl)-4-({(3*S*)-1-[4-(1H-pyrrol-1-yl)phenyl]-3-pyrrolidinyl}carbonyl)piperazine; 1-({(3S)-1-[4-(1H-Imidazol-1-yl)phenyl]-3-pyrrolidinyl}carbonyl)-4-(1-methylethyl)piperazine;
1-(1-Methylethyl)-4-({(3S)-1-[4-(1,3-oxazol-5-yl)phenyl]-3-pyrrolidinyl}carbonyl)piperazine;
1-[4-((3*S*)-3-{[4-(1-Methylethyl)-1-piperazinyl]carbonyl}-1-pyrrolidinyl)phenyl]-2-pyrrolidinone;
4-((3*S*)-3{[4-(1-Methylethyl)hexahydro-1*H*-1,4-diazepin-1-yl]carbonyl}-1-pyrrolidinyl)benzonitrile;1-(1-Methylethyl)-4-({(3*S*)-1-[4-(5-methyl-1,3,4-oxadiazol-2-yl)phenyl]-3-pyrrolidinyl}carbonyl)hexahydro-1*H*-1,4-diazepine;1-({(3*S*)-1-[4-(3-Ethyl-1,2,4-oxadiazol-5-yl)phenyl]-3-pyrrolidinyl}carbonyl)-4-(1-methylethyl)hexahydro-1*H*-1,4-diazepine;
1-({(3*S*)-1-[4-(3-Ethyl-1,2,4-oxadiazol-5-yl)phenyl]-3-pyrrolidinyl)carbonyl)-4-(1-methylethyl)piperazine;1-[4-((3*S*)-3-{[4-(1-Methylethyl)-1-piperazinyl]carbonyl}-1-pyrrolidinyl)phenyl]-1-propanone-1-Cyclobutyl-4-({(3S)-1-[6-(3-methyl-1,2,4-oxadiazol-5-yl)-3-pyridinyl]-3-pyrrolidinyl}carbonyl)piperazine;
1-Cydobutyl-4-({(3*S*)-1-[3-fluoro-4-(3-methyl-1,2,4-oxadiazol-5-yl)phenyl]-3-pyrrolidinyl}carbonyl)piperazine;
1-(1-Methylethyl)-4-({(3*S*)-1-[6-(3-methyl-1,2,4-oxad iazol-5-yl)-3-pyridinyl]-3-pyrrolidinyl}carbonyl)hexahydro-1*H*-1,4-diazepine;
1-(1-Methylethyl)-4-({(3*S*)-1-[6-(3-methyl-1,2,4-oxadiazol-5-yl)-3-pyridinyl]-3-pyrrolidinyl}carbonyl)piperazine;
1-(1-Methylethyl)-4-({(3S)-1-[4-(4-methyl-1H-imidazol-1-yl)phenyl]-3-pyrrolidinyl}carbonyl)piperazine;
1-(1-Methylethyl)-4-({(3S)-1-[4-(2-methyl-1H-imidazol-1-yl)phenyl]-3-pyrrolidinyl}carbonyl)piperazine;
4-((3*S*)-3-{[(3*S*)-3-Methyl-4-(1-methylethyl)-1-piperazinyl]carbonyl}-1-pyrrolidinyl)benzonitrile;
1-(Cyclopropylmethyl)-4-({(3*S*)-1-[6-(5-methyl-1,2,4-oxadiazol-3-yl)-3-pyridinyl]-3-pyrrolidinyl}carbonyl)hexahydro-1*H*-1,4-diazepine;
1-(Cyclopropylmethyl)-4-({(3*S*)-1-[6-(5-methyl-1,2,4-oxadiazol-3-yl)-3-pyridinyl]-3-pyrrolidinyl}carbonyl)piperazine; or
1-Ethyl-4-({(3S)-1-[4-(3-methyl-1,2,4-oxadiazol-5-yl)phenyl]-3-pyrrolidinyl}carbonyl)piperazine;or a pharmaceutically acceptable salt or solvate thereof.

11. A pharmaceutical composition which comprises the compound of formula (I) as defined in any one of claims 1 to 10 or a pharmaceutically acceptable salt or solvate thereof and a pharmaceutically acceptable carrier or excipient.

12. A compound as defined in any one of claims 1 to 10 for use in therapy.

13. A compound as defined in any one of claims 1 to 10 for use in the treatment of neurological diseases.

14. Use of a compound as defined in any one of claims 1 to 10 in the manufacture of a medicament for the treatment of neurological diseases.

15. A process for the preparation of a compound of formula (I) or a pharmaceutically acceptable salt or solvate thereof, which process comprises:
(a) reacting a compound of formula (II) 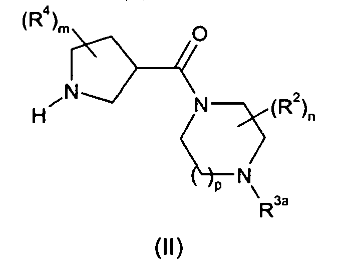 or an optionally activated or protected derivative thereof, wherein R², R⁴, m, n and p are as defined in claim 1 and R^{3a} is as defined for R³ in claim 1 or a group convertible to R³, with a compound of formula R¹-L¹, wherein R¹ is as defined in claim 1 and L¹ represents a suitable leaving group, such as a halogen atom or triflate group, followed by a deprotection reaction as necessary; or
(b) reacting a compound of formula (III) 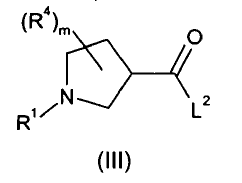 wherein R¹, R⁴ and m are as defined in claim 1 and L² represents OH or a suitable leaving group, such as a halogen atom, with a compound of formula (IV) 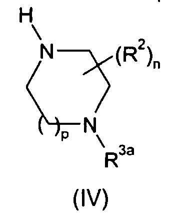 wherein R², n and p are as defined in claim 1, R^{3a} is as defined for R³ in claim 1 or a group convertible to R³; or
(c) deprotecting a compound of formula (I) or converting groups which are protected; or
(d) interconversion from one compound of formula (I) to another.

16. A process for the preparation of a single enantiomer of a compound of formula (I) or a pharmaceutically acceptable salt or solvate thereof, which process comprises reacting a compound of formula (XV) or (XV)^{a} wherein R⁴ and m are as defined in claim 1, wherein L⁷ represents a leaving group and wherein P³ represents a protecting group or R¹, as defined in claim 1: 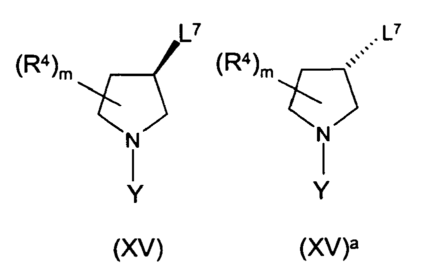 with a carbon nucleophile that can be converted to a carboxylic acid, to produce a compound of formula (XVI) or (XVI)^{a}, wherein cNu a carbon nucleophile that can be converted to a carboxylic acid, wherein the compound of formula (XVI) or (XVI)^{a} is used in the preparation of the single enantiomer of the compound of formula (I).

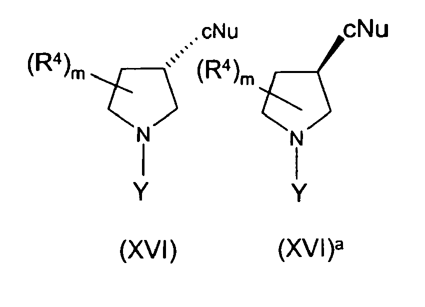

## Patentansprüche

1. Verbindung der Formel (I) oder ein pharmazeutisch verträgliches Salz davon: 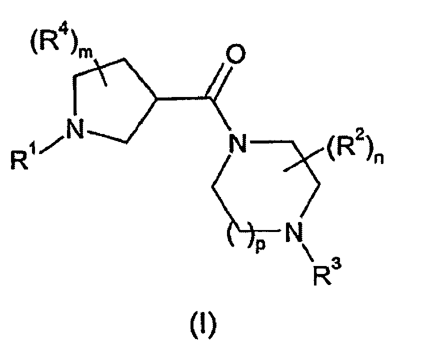 wobei:
R¹ Aryl, Heteroaryl, -Aryl-X-C₃₋₇-cycloalkyl, -Heteroaryl-X-C₃₋₇-cycloalkyl, -Aryl-X-aryl, -Aryl-X-heteroaryl, -Aryl-X-heterocyclyl, -Heteroaryl-X-heteroaryl, -Heteroaryl-X-aryl oder -Heteroaryl-X-heterocyclyl darstellt;
wobei die Aryl-, Heteroaryl- und Heterocyclylreste von R¹ gegebenenfalls mit einem oder mehreren Substituenten substituiert sein können, welche gleich oder verschieden sein können und welche ausgewählt sind aus Halogen, Hydroxy, Cyano, Nitro, Oxo, Halogen-C₁₋₆-alkyl, Halogen-C₁₋₆-alkoxy, C₁₋₆-Alkyl, C₁₋₆-Alkoxy, C₁₋₆-Alkylthio, C₁₋₆-Alkoxy-C₁₋₆-alkyl, C₃₋₇-Cycloalkyl-C₁₋₆-alkoxy, -COC₁₋₆-Alkyl, -CO-Halogen-C₁₋₆-alkyl, -COC₁₋₆-Alkylcyano, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, C₁₋₆-Alkylsulfonamido-C₁₋₆-alkyl, C₁₋₆-Alkylamido-C₁₋₆-alkyl, Aryl, Arylsulfonyl, Arylsulfonyloxy, Aryloxy, Arylsulfonamido, Arylcarboxamido, Aroyl, oder einem Rest -NR¹⁵R¹⁶, -CONR¹⁵R¹⁶, -NR¹⁵COR¹⁶, -C(R¹⁵)=NOR¹⁶, -NR¹⁵SO₂R¹⁶ oder -SO₂NR¹⁵R¹⁶, wobei R¹⁵ und R¹⁶ unabhängig Wasserstoff oder C₁₋₆-Alkyl darstellen oder zusammen einen heterocyclischen Ring bilden;
X eine Bindung, O, CO, SO₂, OCH₂ oder CH₂O darstellt;
R² und R⁴ jeweils unabhängig C₁₋₄-Alkyl darstellen;
R³ C₂₋₆-Alkyl, C₃₋₆-Alkenyl, C₃₋₆-Alkinyl, C₃₋₆-Cycloalkyl, C₅₋₆-Cycloalkenyl oder -C₁₋₄-Alkyl-C₃₋₆-cycloalkyl darstellt;
wobei die C₃₋₆-Cycloalkylreste von R³ gegebenenfalls mit einem oder mehreren Substituenten substituiert sein können, welche gleich oder verschieden sein können und welche ausgewählt sind aus Halogenatomen, C₁₋₄-Alkyl- oder Trifluormethylresten;
m und n unabhängig 0, 1 oder 2 darstellen;
p 1 oder 2 darstellt;
oder ein Solvat davon.

2. Verbindung gemäß Anspruch 1, wobei die Stereochemie des Kohlenstoffatoms im Pyrrolidinrest, welcher an die Carbonylgruppe gebunden ist, die S-Konfiguration aufweist.

3. Verbindung gemäß Anspruch 1 oder Anspruch 2, wobei R¹
- Aryl, gegebenenfalls substituiert mit einem oder mehreren -COC₁₋₆-Alkyl- oder Cyanoresten;
- Aryl-X-heteroaryl, gegebenenfalls mit einem Halogenatom am Arylrest substituiert und/oder gegebenenfalls mit einem C₁₋₆-Alkylrest an dem Heteroarylrest substituiert;
- Aryl-X-heterocyclyl, gegebenenfalls substituiert mit einem oder mehreren Oxo-Resten;
- Heteroaryl, gegebenenfalls substituiert mit einem oder mehreren C₁₋₆-Alkyl- oder Halogen-C₁₋₆-alkylresten; oder
- Heteroaryl-X-heteroaryl, gegebenenfalls substituiert mit einem oder mehreren C₁₋₆-Alkylresten
darstellt.

4. Verbindung gemäß Anspruch 3, wobei R¹
- Aryl, gegebenenfalls substituiert mit einem oder mehreren -COC₁₋₆-Alkyl- oder Cyanoresten;
- Aryl-X-heteroaryl, gegebenenfalls mit einem Halogenatom oder einem C₁₋₆-Alkylrest substituiert; oder
- Heteroaryl, gegebenenfalls substituiert mit einem oder mehreren Halogen-C₁₋₆-alkylresten darstellt.

5. Verbindung gemäß einem der vorstehenden Ansprüche, wobei X eine Bindung darstellt.

6. Verbindung gemäß einem der vorstehenden Ansprüche, wobei m die Bedeutung 0 hat.

7. Verbindung gemäß einem der vorstehenden Ansprüche, wobei n die Bedeutung 0 oder 1 hat.

8. Verbindung gemäß einem der vorstehenden Ansprüche, wobei p die Bedeutung 1 oder 2 hat.

9. Verbindung gemäß einem der vorstehenden Ansprüche, wobei R³ C₂₋₆-Alkyl, C₃₋₆-Cycloalkyl oder -C₁₋₄-Alkyl-C₃₋₆-cycloalkyl darstellt.

10. Verbindung gemäß Anspruch 1, nämlich:
1-(1-Methylethyl)-4-({(3S)-1-[6-(trifluormethyl)-3-pyridinyl]-3-pyrrolidinyl}carbonyl)-piperazin;
1-[4-((3S)-3-{[4-(1-Methylethyl)-1-piperazinyl]carbonyl}-1-pyrrolidinyl)phenyl]ethanon; 1-(1-Methylethyl)-4-({(3S)-1-[4-(3-methyl-1,2,4-oxadiazol-5-yl)phenyl]-3-pyrrolidinyl}carbonyl)piperazin;
(2S)-2-Methyl-1-(1-methylethyl)-4-({(3S)-1-[6-(trifluormethyl)-3-pyridinyl]-3-pyrrolidinyl}carbonyl)piperazin;
1-[4-((3S)-3-{[(3S)-3-Methyl-4-(1-methylethyl)-1-piperazinyl]carbonyl}-1-pyrrolidinyl)-phenyl]ethanon;
(2S)-2-Methyl-1-(1-methylethyl)-4-({(3S)-1-[4-(3-methyl-1,2,4-oxadiazol-5-yl)phenyl]-3-pyrrolidinyl carbonyl)piperazin;
1-(1-Ethylpropyl)-4-({(3S)-1-[6-(trifluormethyl)-3-pyridinyl]-3-pyrrolidinyl}carbonyl)-piperazin;
1-[4-((3S)-3-{[4-(1-Ethylpropyl)-1-piperazinyl]carbonyl}-1-pyrrolidinyl)phenyl]ethanon; 1-(1-Ethylpropyl)-4-({(3S)-1-[4-(3-methyl-1,2,4-oxadiazol-5-yl)phenyl]-3-pyrrolidinyl}carbonyl)piperazin;
1-[4-((3S)-3-1[4-(Cyclopropylmethyl)hexahydro-1H-1,4-diazepin-1-yl]carbonyl}-1-pyrrolidinyl)phenyl]ethanon;
1-(Cyclopropylmethyl)-4-({(3S)-1-[4-(3-methyl-1,2,4-oxadiazol-5-yl)phenyl]-3-pyrrolidinyl}carbonyl)hexahydro-1H-1,4-diazepin;
1-Cyclobutyl-4-({(3S)-1-[6-(trifluormethyl)-3-pyridinyl]-3-pyrrolidinyl}carbonyl)-piperazin;
1-Cyclobutyl-4-({(3S)-1-[4-(3-methyl-1,2,4-oxadiazol-5-yl)phenyl]-3-pyrrolidinyl)-carbonyl)piperazin;
1-(Cyclopropylmethyl)-4-({(3S)-1-[6-(trifluormethyl)-3-pyridinyl]-3-pyrrolidinyl}carbonyl)piperazin;
1-[4-((3S)-3-{[4-(Cyclopropylmethyl)-1-piperazinyl]carbonyl}-1-pyrrolidinyl)phenyl]ethanon;
1-(Cyclopropylmethyl)-4-({(3S)-1-[4-(3-methyl-1,2,4-oxadiazol-5-yl)phenyl]-3-pyrrolidinyl}carbonyl)piperazin;
(R,S)-1-Cyclobutyl-4-({1-[6-(trifluormethyl)-3-pyridinyl]-3-pyrrolidinyl}carbonyl)-hexahydro-1H-1,4-diazepin;
1-Cyclobutyl-4-({(3R)-1-[6-(trifluormethyl)-3-pyridinyl]-3-pyrrolidinyl}carbonyl)-hexahydro-1H-1,4-diazepin;
1-Cyclobutyl-4-({(3S)-1-[6-(trifluormethyl)-3-pyridinyl]-3-pyrrolidinyl}carbonyl)-hexahydro-1H-1,4-diazepin;
1-Cyclobutyl-4-({(3R)-1-[4-(3-methyl-1,2,4-oxadiazol-5-yl)phenyl]-3-pyrrolidinyl}carbonyl)hexahydro-1H-1,4-diazepin;
1-Cyclobutyl-4-({(3S)-1-[4-(3-methyl-1,2,4-oxadiazol-5-yl)phenyl]-3-pyrrolidinyl}carbonyl)hexahydro-1H-1,4-diazepin;
6-{(3R)-3-[(4-Cyclobutylhexahydro-1H-1,4-diazepin-1-yl)carbonyl]-1-pyrrolidinyl}-2-methylchinolin;
6-{(3S)-3-[(4-Cyclobutylhexahydro-1H-1,4-diazepin-1-yl)carbonyl]-1-pyrrolidinyl}-2-methylchinolin;
1-Cyclobutyl-4-({(3R)-1-[2-fluor-4-(3-methyl-1,2,4-oxadiazol-5-yl)phenyl]-3-pyrrolidinyl}carbonyl)hexahydro-1H-1,4-diazepin;
1-Cyclobutyl-4-({(3S)-1-[2-fluor-4-(3-methyl-1,2,4-oxadiazol-5-yl)phenyl]-3-pyrrolidinyl}carbonyl)hexahydro-1H-1,4-diazepin;
1-(4-{(3R)-3-[(4-Cyclobutylhexahydro-1H-1,4-diazepin-1-yl)carbonyl]-1-pyrrolidinyl }phenyl)ethanon;
1-(4-{(3S)-3-[(4-cyclobutylhexahydro-1H-1,4-diazepin-1-yl)carbonyl]-1-pyrrolidinyl}phenyl)ethanon;
(R,S)-1-(1-Methylethyl)-4-({1-[6-(trifluormethyl)-3-pyridinyl]-3-pyrrolidinyl}carbonyl)-hexahydro-1H-1,4-diazepin;
(R,S)-1-Cyclobutyl-4-({1-[2-(trifluormethyl)-4-pyridinyl]-3-pyrrolidinyl}carbonyl)-hexahydro-1H-1,4-diazepin;
1-({(3S)-1-[3-Fluor-4-(3-methyl-1,2,4-oxadiazol-5-yl)phenyl]-3-pyrrolidinyl}carbonyl)-4-(1-methylethyl)piperazin;
1-({(3S)-1-[2-Fluor-4-(3-methyl-1,2,4-oxadiazol-5-yl)phenyl]-3-pyrrolidinyl}carbonyl)-4-(1-methylethyl)piperazin;
1-(1-Methylethyl)-4-({(3S)-1-[6-(trifluormethyl)-3-pyridinyl]-3-pyrrolidinyl}carbonyl)-hexahydro-1H-1,4-diazepin;
1-(4-((3S)-3-{[4-(1-Methylethyl)hexahydro-1H-1,4-diazepin-1-yl]carbonyl}-1-pyrrolidinyl)phenyl]ethanon;
1-(1-Methylethyl)-4-({(3S)-1-[4-(3-methyl-1,2,4-oxadiazol-5-yl)phenyl]-3-pyrrolidinyl}carbonyl)hexahydro-1H-1,4-diazepin;
1-({(3S)-1-[3-Fluor-4-(3-methyl-1,2,4-oxadiazol-5-yl)phenyl]-3-pyrrolidinyl}carbonyl)-4-(1-methylethyl)hexahydro-1H-1,4-diazepin;
1-(1-Methylethyl)-4-({(3S)-1-[4-(5-methyl-1,3,4-oxadiazol-2-yl)phenyl]-3-pyrrolidinyl}carbonyl)piperazin;
1-(1-Methylethyl)-4-({(3S)-1-[3-(3-methyl-1,2,4-oxadiazol-5-yl)phenyl]-3-pyrrolidinyl}carbonyl)piperazin;
(R,S)-1-(1-Methylethyl)-4-({1-[4-(2-methyl-1,3-oxazol-4-yl)phenyl]-3-pyrrolidinyl}carbonyl)piperazin;
1-(1-Methylethyl)-4-({(3S)-1-[4-(2-methyl-1,3-oxazol-4-yl)phenyl]-3-pyrrolidinyl}carbonyl)piperazin;
1-(1-Methylethyl)-4-({(3S)-1-[4-(5-methyl-1,2,4-oxadiazol-3-yl)phenyl]-3-pyrrolidinyl } carbonyl)piperazin;
1-(1-Methylethyl)-4-({(3S)-1-[4-(2-methyl-1,3-oxazol-5-yl)phenyl]-3-pyrrolidinyl}carbonyl)piperazin;
3-((3S)-3-{[4-(1-Methylethyl)-1-piperazinyl]carbonyl}-1-pyrrolidinyl)benzonitril,
4-((3 S)-3-{[4-(1-Methylethyl)-1-piperazinyl]carbonyl}-1-pyrrolidinyl)benzonitril;
5-((3S)-3-{[4-(1-Methylethyl)-1-piperazinyl]cabonyl}-1-pyrrolidinyl)-2-(trifluormethyl)-pyrimidin;
1-(1-Methylethyl)-4-(f ({(3R)-1-[4-(3-methyl-1,2,4-oxadiazol-5-yl)phenyl]-3-pyrrolidinyl}carbonyl)piperazin;
1-Cyclobutyl-4-({(3R)-1-[4-(3-methyl-1,2,4-oxadiazol-5-yl)phenyl]-3-pyrrolidinyl}carbonyl)piperazin;
1-(1-Methylethyl)-4-({(3S)-1-[4-(5-phenyl-1,3,4-oxadiazol-2-yl)phenyl]-3-pyrrolidinyl}carbonyl)piperazin;
1-({(3S)-1-[4-(5-Isoxazolyl)phenyl]-3-pyrrolidinyl}carbonyl)-4-(1-methylethyl)piperazin;
1-(1-Methylethyl)-4-({(3S)-1-[4-(1H-pyrrol-1-yl)phenyl]3-pyrrolidinyl}carbonyl)-piperazin;
1({(3S)-1-[4-(1H-Imidazol-1-yl)phenyl]-3-pyrrolidinyl}carbonyl)-4-(1-methylethyl)-piperazin;
1-(1-Methylethyl)-4-({(3S)-1-[4-(1,3-oxazol-5-yl)phenyl]-3-pyrrolidinyl}carbonyl)-piperazin;
1-[4-((3S)-3-{[4-(1-Methylethyl)-1-piperazinyl]carbonyl)-1-pyrrolidinyl)phenyl]-2-pyrrolidinon;
4-((3S)-3-{[4-(1-Methylethyl)hexahydro-1H-1,4-diazepin-1-yl]carbonyl)-1-pyrrolidinyl)benzonitril;
1-(1-Methylethyl)-4-({(3S)-1-[4-(5-methyl-1,3,4-oxadiazol-2-yl)phenyl]-3-pyrrolidinyl }carbonyl)hexahydro-1H-1,4-diazepin;
1-({(3S)-1-[4-(3-Ethyl-1,2,4-oxadiazol-5-yl)phenyl]-3-pyrrolidinyl)carbonyl)-4-(1-methylethyl)hexahydro-1H-1,4-diazepin;
1-({(3S)-1-[ 4-(3-Ethyl-1,2,4-oxadiazol-5-yl)phenyl]-3-pyrrolidinyl carbonyl)-4-(1-methylethyl)piperazin;
1-[4-((3S)-3-{[4-(1-Methylethyl)-1-piperazinyl]carbonyl}-1-pyrrolidinyl)phenyl]-1-propanon;
1-Cyclobutyl-4-({(3S)-1-[6-(3-methyl-1,2,4-oxadiazol-5-yl)-3-pyridinyl]-3-pyrrolidinyl}carbonyl)piperazin;
1-Cyclobutyl-4-({(3S)-1-[3-Fluor-4-(3-methyl-1,2,4-oxadiazol-5-yl)phenyl]-3-pyrrolidinyl}carbonyl)piperazin;
1-(1-Methylethyl)-4-({(3S)-1-[6-(3-methyl-1,2,4-oxadiazol-5-yl)-3-pyridinyl]-3-pyrrolidinyl }carbonyl)hexahydro-1H-1,4-diazepin;
1-(1-Methylethyl)-4-({(3S)-1-[6-(3-methyl-1,2,4-oxadiazol-5-yl)-3-pyridinyl]-3-pyrrolidinyl }carbonyl)piperazin;
1-(1-Methylethyl)-4-(1{3S)-1-[4-(4-methyl-1H-imidazol-1-yl)phenyl]-3-pyrrolidinyl}- carbonyl)piperazin;
1-(1-Methylethyl)-4-({(3S)-1-[4-(2-methyl-1H-imidazol-1-yl)phenyl]-3-pyrrolidinyl}carbonyl)piperazin;
4-((3S)-3-{[(3S)-3-Methyl-4-(1-methylethyl)-1-piperazinyl]carbonyl}-1-pyrrolidinyl)-benzonitril;
1-(Cyclopropylmethyl)-4-({(3S)-1-[6-(5-methyl-1,2,4-oxadiazol-3-yl)-3-pyridinyl]-3-pyrrolidinyl}carbonyl)hexahydro-1H-1,4-diazepin;
1-(Cyclopropylmethyl)-4-({(3S)-1-[6-(5-methyl-1,2,4-oxadiazol-3-yl)-3-pyridinyl]-3-pyrrolidinyl }carbonyl)piperazin; oder
1-Ethyl-4-({(3S)-1-[4-(3-methyl-1,2,4-oxadiazol-5-yl)phenyl]-3-pyrrolidinyl}carbonyl)piperazin;
oder ein pharmazeutisch verträgliches Salz oder Solvat davon.

11. Arzneimittel, welches die Verbindung der Formel (I) wie in einem der Ansprüche 1 bis 10 definiert, oder ein pharmazeutisch verträgliches Salz oder Solvat davon, und einen pharmazeutisch verträglichen Träger oder Exzipienten umfasst.

12. Verbindung wie in einem der Ansprüche 1 bis 10 definiert, zur Verwendung in der Therapie.

13. Verbindung wie in einem der Ansprüche 1 bis 10 definiert, zur Verwendung in der Behandlung von neurologischen Erkrankungen.

14. Verwendung einer Verbindung wie in einem der Ansprüche 1 bis 10 definiert zur Herstellung eines Medikaments zur Behandlung von neurologischen Erkrankungen.

15. Verfahren zur Herstellung einer Verbindung der Formel (I), oder eines pharmazeutisch verträglichen Salzes oder Solvats davon, wobei das Verfahren umfasst:
(a) Umsetzen einer Verbindung der Formel (II) 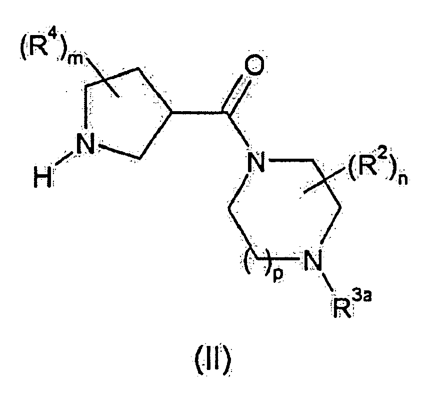 oder eines gegebenenfalls aktivierten oder geschützten Derivats davon, wobei R², R⁴, m, n und p wie in Anspruch 1 definiert sind und R^{3a} wie für R³ in Anspruch 1 definiert ist, oder ein Rest ist, der in R³ umwandelbar ist,
mit einer Verbindung der Formel R¹-L¹, wobei R¹ wie in Anspruch 1 definiert ist und L¹ eine geeignete Abgangsgruppe, wie ein Halogenatom oder ein Triflatrest, darstellt, gefolgt von einer Entschützungsreaktion, wenn nötig; oder
(b) Umsetzen einer Verbindung der Formel (III) 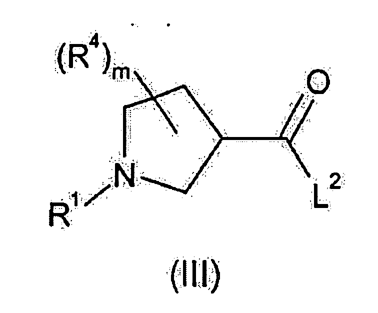 wobei R¹, R⁴ und m wie in Anspruch 1 definiert sind und L² OH oder eine geeignete Abgangsgruppe, wie ein Halogenatom, darstellt, mit einer Verbindung der Formel (IV) 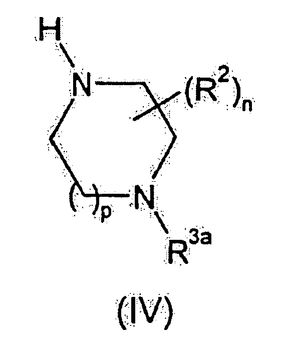 wobei R², n und p wie in Anspruch 1 definiert sind, R^{3a} wie für R³ in Anspruch 1 definiert ist; oder ein Rest ist, der in R³ umwandelbar ist, oder
c) Entschützen einer Verbindung der Formel (I) oder Umwandeln von Resten, welche geschützt sind; oder
d) Untereinander Umwandeln einer Verbindung der Formel (I) in eine andere.

16. Verfahren zur Herstellung eines einzelnen Enantiomers einer Verbindung der Formel (I), oder eines pharmazeutisch verträglichen Salzes oder Solvats davon, wobei das Verfahren das Umsetzen einer Verbindung der Formel (XV) oder (XV)^{a} umfasst, wobei R⁴ und m wie in Anspruch 1 definiert sind, wobei L⁷ eine Abgangsgruppe darstellt, und wobei P³ eine Schutzgruppe oder R¹ wie in Anspruch 1 definiert, darstellt: 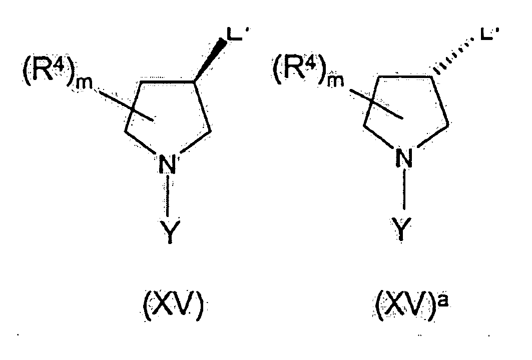 mit einem Kohlenstoffnukleophil, welches in eine Carbonsäure umgewandelt werden kann, um eine Verbindung der Formel (XVI) oder (XVI)^{a} herzustellen, wobei cNu ein Kohlenstoffnukleophil darstellt, welches in eine Carbonsäure umgewandelt werden kann, wobei die Verbindung der Formel (XVI) oder (XVI)^{a} zur Herstellung des einzelnen Enantiomers der Verbindung der Formel (I) verwendet wird.

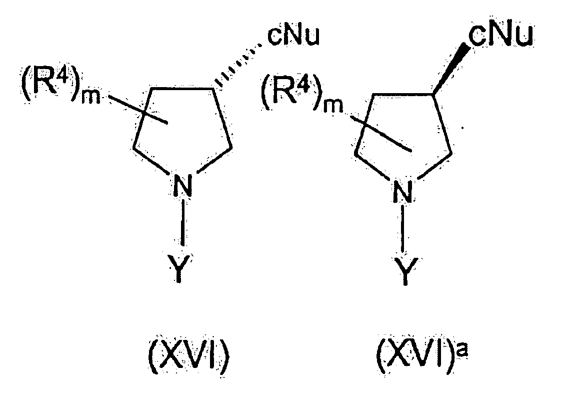

## Revendications

1. Composé de formule (I) ou un de ses sels pharmaceutiquement acceptables 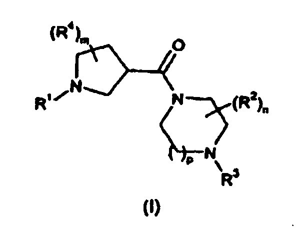 formule dans laquelle
R¹ représente un groupe aryle, hétéroaryle, -aryl-X-(cycloalkyle en C₃ à C₇), -hétéroaryl-X-(cycloalkyle en C₃ à C₇), -aryl-X-aryle, -aryl-X-hétéroaryle, -aryl-X-hétérocyclyle, -hétéroaryl-X-hétéroaryle, -hétéroaryl-X-aryle ou -hétéroaryl-X-hétérocyclyle ;
lesdits groupes aryle, hétéroaryle et hétérocyclyle de R¹ pouvant être facultativement substitués avec un ou plusieurs substituants qui peuvent être identiques ou différents et qui sont choisis dans le groupe consistant en des substituants halogéno, hydroxy, cyano, nitro, oxo, halogénalkyle en C₁ à C₆, halogénalkoxy en C₁ à C₆, alkyle en C₁ à C₆, alkoxy en C₁ à C₆, alkylthio en C₁ à C₆, (alkoxy en C₁ à C₆) (alkyle en C₁ à C₆), (cycloalkyle en C₃ à C₇) (alkoxy en C₁ à C₆), -CO(alkyle en C₁ à C₆), -CO-halogéno(alkyle en C₁ à C₆), -CO(alkyle en C₁ à C₆)cyano, (alkoxy en C₁ à C₆) carbonyle, alkylsulfonyle en C₁ à C₆, alkylsulfinyle en C₁ à C₆, alkylsulfonyloxy en C₁ à C₆, (alkyle en C₁ à C₆)sulfonyl(alkyle en C₁ à C₆), (alkyle en C₁ à C₆) sulfonamido (alkyle en C₁ à C₆), (alkyle en C₁ à C₆) amido(alkyle en C₁ à C₆), aryle, arylsulfonyle, arylsulfonyloxy, aryloxy, arylsulfonamido, arylcarboxamido, aroyle ou un groupe -NR¹⁵R¹⁶, -CONR¹⁵R¹⁶, -NR¹⁵COR¹⁶, -C(R¹⁵)=NOR¹⁶, -NR¹⁵SO₂R¹⁶ ou -SO₂NR¹⁵R¹⁶, dans lequel R¹⁵ et R¹⁶ représentent indépendamment un atome d'hydrogène ou un groupe alkyle en C₁ à C₆, ou bien forment conjointement un noyau hétérocyclique ;
X représente une liaison, O, un groupe CO, SO₂, OCH₂ ou CH₂O ;
chacun de R² et R⁴ représente indépendamment un groupe alkyle en C₁ à C₄ ;
R³ représente un groupe alkyle en C₂ à C₆, alcényle en C₃ à C₆, alcynyle en C₃ à C₆, cycloalkyle en C₃ à C₆, cycloalcényle en C₅ ou C₆ ou -(alkyle en C₁ à C₄) (cycloalkyle en C₃ à C₆) ;
lesdits groupes cycloalkyle en C₃ à C₆ de R³ pouvant être facultativement substitués avec un ou plusieurs substituants qui peuvent être identiques ou différents et qui sont choisis dans le groupe consistant en des groupes halogéno, alkyle en C₁ à C₉ et trifluorométhyle ;
m et n sont égaux indépendamment à 0, 1 ou 2 ;
p est égal à 1 ou 2 ;
ou un de ses produits de solvatation.

2. Composé suivant la revendication 1, dans lequel la stéréochimie de l'atome de carbone dans le groupe pyrrolidine qui est fixé au groupe carbonyle a la configuration S.

3. Composé suivant la revendication 1 ou la revendication 2, dans lequel R¹ représente un groupe :
- aryle, facultativement substitué avec un ou plusieurs groupes -CO (alkyle en C₁ à C₆) ou cyano ;
- aryl-X-hétéroaryle, facultativement substitué sur le groupe aryle avec un substituant halogéno et/ou facultativement substitué sur le groupe hétéroaryle avec un groupe alkyle en C₁ à C₆ ;
- aryl-X-hétérocyclyle, facultativement substitué avec un ou plusieurs groupes oxo ;
- hétéroaryle facultativement substitué avec un ou plusieurs groupes alkyle en C₁ à C₆ ou halogénalkyle en C₁ à C₆ ; ou
- hétéroaryl-X-hétéroaryle facultativement substitué avec un ou plusieurs groupes alkyle en C₁ à C₆.

4. Composé suivant la revendication 3, dans lequel R¹ représente un groupe
- aryle facultativement substitué avec un ou plusieurs groupes -CO-(alkyle en C₁ à C₆) ou cyano ;
- aryl-X-hétéroaryle facultativement substitué avec un groupe halogéno ou alkyle en C₁ à C₆ ; ou
- hétéroaryle facultativement substitué avec un ou plusieurs groupes halogénalkyle en C₁ à C₆.

5. Composé suivant l'une quelconque des revendications précédentes, dans lequel X représente une liaison.

6. Composé suivant l'une quelconque des revendications précédentes, dans lequel m est égal à 0.

7. Composé suivant l'une quelconque des revendications précédentes, dans lequel n est égal à 0 ou 1.

8. Composé suivant l'une quelconque des revendications précédentes, dans lequel p est égal à 1 ou 2.

9. Composé suivant l'une quelconque des revendications précédentes, dans lequel R³ représente un groupe alkyle en C₂ à C₆, cycloalkyle en C₃ à C₆ ou -(alkyle en C₁ à C₄)-(cycloalkyle en C₃ à C₆).

10. Composé suivant la revendication 1, qui est :
la 1-(1-méthyléthyl)-4-(((3*S*)-1-[6-(trifluorométhyl)-3-pyridinyl]-3-pyrrolidinyl}carbonyl)pipérazine ;
la 1-[4-((3*S*)-3-([4-(1-méthyléthyl)-1-pipérazinyl]-carbonyl}-1-pyrrolidinyl)phényl]éthanone ;
la 1-(1-méthyléthyl)-[4-(((3*S*)-1-[4-(3-méthyl-1,2,4-oxadiazole-5-yl)phényl]-3-pyrrolidinyl}carbonyl)pipérazine ;
la (2*S*)-2-méthyl-1-(1-méthyléthyl)-4-({(3*S*)-1-[6-(trifluorométhyl)-3-pyridinyl]-3-pyrrolidinyl}-3-carbonyl)pipérazine ;
la 1-[4-((3*S*)-3-{[(3*S*)-3-méthyl-4-(1-méthyléthyl)-1-pipérazinyl]carbonyl}-1-pyrrolidinyl)phényl]éthanone ;
la (2*S*)-2-méthyl-1-(1-méthyléthyl)-4-({(3*S*)-1-[4(3-méthyl-1,2,4-oxadiazole-5-yl)phényl]-3-pyrrolidinyl}carbonyl)pipérazine ;
la 1-(1-éthylpropyl)-4-({(3*S*)-1-[6-(trifluorométhyl)-3-pyridinyl]-3-pyrrolidinyl}carbonyl)pipérazine ;
la 1-[4-((3*S*)-3-([4-(1-méthylpropyl)-1-pipérazinyl]carbonyl}-1-pyrrolidinyl)phényl]éthanone ;
la 1-(1-éthylpropyl)-4-({(3S)-1-[4-(3-méthyl-1,2,4-oxadiazole-5-yl)phényl]-3-pyrrolidinyl}carbonyl)pipérazine ;
la 1-[4-((3*S*)-3-{[4-(cyclopropylméthyl)hexahydro-1*H*-1,4-diazépine-1-yl]carbonyl}-1-pyrrolidinyl)phényl]éthanone ;
la 1-(cyclopropylméthyl)-4-({(3*S*)-1-[4-(3-méthyl-1,2,4-oxadiazole-5-yl)phényl]-3-pyrrolidinyl}carbonyl)hexahydro-1*H*-1,4-diazépine ;
la 1-cyclobutyl-4-({(3*S*)-1-[6-(trifluorométhyl)-3-pyridinyl]-3-pyrrolidinyl}carbonyl)pipérazine ;
la 1-cyclobutyl-4-({(3*S*)-1-[4-(3-méthyl-1,2,4-oxadiazole-5-yl)phényl]-3-pyrrolidinyl}carbonyl)pipérazine ;
la 1-(cyclopropylméthyl)-4-({(3*S*)-1-[6-(trifluorométhyl]-3-pyridinyl]-3-pyrrolidinyl}carbonyl)pipérazine ;
la 1-[4-((3*S*)-3-{[4-(cyclopropylméthyl)-1-pipérazinyl]-carbonyl}-1-pyrrolidinyl)phényl]éthanone ;
la 1-(cyclopropylméthyl)-4-({(3*S*)-1-[4-(3-méthyl-1,2,4-oxadiazole-5-yl)phényl]-3-pyrrolidinyl}carbonyl)pipérazine ;
la (R,S)-1-cyclobutyl-4-({1-[6-(trifluorométhyl)-3-pyridinyl]-3-pyrrolidinyl)carbonyl)hexahydro-1*H*-1,4-diazépine ;
la 1-cyclobut yl-4-({(3R)-1-[6-(trifluorométhyl)-3-pyridinyl]-3-pyrrolidinyl}carbonyl)hexahydro-1*H*-1,4-diazépine ;
la 1-cyclobutyl-4-({(3S)-1-[6-(trifluorométhyl)-3-pyridinyl]-3-pyrrolidinyl}carbonyl)hexahydro-1*H*-1,4-diazépine ;
la 1-cyclobutyl-4-({(3*R*)-1-[4-(3-méthyl-1,2,4-oxadiazole-5-yl)phényl]-3-pyrrolidinyl}carbonyl)-hexahydro-1*H*-1,4-diazépine;
la 1-cyclobutyl-4-(((3*S*)-1-[4-(3-méthyl-1,2,4-oxadiazole-5-yl)phényl]-3-pyrrolidinyl)carbonyl)-hexahydro-1*H*-1,4-diazépine;
la 6-{(3R)-3-[(4-cyclobutylhexahydro-1*H*-1,4-diazépine-1-yl)carbonyl]-1-pyrrolidinyl}-2-méthylquinoléine ;
la 6-{(3*S*)-3-[(4-cyclobutylhexahydro-1*H*-1,4-diazépine-1-yl)carbonyl]-1-pyrrolidinyl}-2-méthylquinoléine ;
la 1-cyclobutyl-4-({(3*R*)-1-[2-fluoro-4-(3-méthyl-1,2,4-oxadiazole-5-yl)phényl]-3-pyrrolidinyl)carbonyl)-hexahydro-1*H*-1,4-diazépine ;
la 1-cyclobutyl-4-(((3*S*)-1-[2-fluoro-4-(3-méthyl-1,2,4-oxadiazole-5-yl)phényl]-3-pyrrolidinyl)carbonyl)-hexahydro-1*H*-1,4-diazépine ;
la 1-(4-{(3*R*)-3-[(4-cyclobutylhexahydro-1*H*-1,4-diazépine-1-yl)carbonyl]-1-pyrrolidinyl}phényl)éthanone ;
la 1-(4-{(3*S*)-3-[(4-cyclobutylhexahydro-1*H*-1,4-diazépine-1-yl)carbonyl)-1-pyrrolidinyl}phényl)éthanone ;
la (R,S)-1-(1-méthyléthyl)-4-({1-[6-(trifluorométhyl)-3-pyridinyl]-3-pyrrolidinyl}carbonyl)hexahydro-1*H*-1,4-diazépine ;
la (R,S)-1-cyclobutyl-4-({1-[2-(trifluorométhyl)-4-pyridinyl]-3-pyrrolidinyl}carbonyl)hexahydro-1*H*-1,4-diazépine ;
la 1-({(3*S*)-[3-fluoro-4-(3-méthyl-1,2,4-oxadiazole-5-yl)-phényl]-3-pyrrolidinyl}carbonyl)-4-(1-méthyléthyl)pipérazine ;
la 1-({(3*S*)-1-[2-fluoro-4-(3-méthyl-1,2,4-oxadiazole-5-yl)-phényl]-3-pyrrolidinyl}carbonyl)-4-(1-méthyléthyl)pipérazine ;
la 1-(1-méthyléthyl)-4-({(3*S*)-1-[6-(trifluorométhyl)-3-pyridinyl]-3-pyrrolidinyl}carbonyl)hexahydro-1*H*-1,4-diazépine ;
la 1-[4-((3*S*)-3-{[4-(1-méthyléthyl) hexahydro-1*H*-1,4-diazépine-1-yl]carbonyl}-1-pyrrolidinyl)phényl]éthanone ;
la 1-(1-méthyléthyl)-4-({(3*S*)-1-[4-(3-méthyl-1,2,4-oxadiazole-5-yl)phényl]-3-pyrrolidinyl}carbonyl)hexahydro-1*H*-1,4-diazépine ;
la 1-({(3*S*)-[3-fluoro-4-(3-méthyl-1,2,4-oxadiazole-5-yl)-phényl]-3-pyrrolidinyl}carbonyl)-4-(1-méthyléthyl)hexahydro-1*H*-1,4-diazépine ;
la 1-(1-méthyléthyl)-4-({(3*S*)-1-[4-(5-méthyl-1,3,4-oxadiazole-2-yl)phényl]-3-pyrrolidinyl}carbonyl)pipérazine ;
la 1-(1-méthyléthyl)-4-({(3*S*)-1-[3-(3-méthyl-1,2,4-oxadiazole-5-yl)phényl]-3-pyrrolidinyl}carbonyl)pipérazine ;
la (R,S)-1-(1-méthyléthyl)-4-({1-[4-(2-méthyl-1,3-oxazole-4-yl)phényl]-3-pyrrolidinyl}carbonyl)pipérazine ;
la 1-(1-méthyléthyl)-4-({(3*S*)-1-[4-(2-méthyl-1,3-oxazole-4-yl)phényl]-3-pyrrolidinyl}carbonyl)pipérazine ;
la 1-(1-méthyléthyl)-4-({(3*S*)-1-[4-(5-méthyl-1,2,4-oxadiazole-3-yl)phényl]-3-pyrrolidinyl}carbonyl)pipérazine ;
la 1-(1-méthyléthyl)-4-({(3*S*)-1-[4-(2-méthyl-1,3-oxazole-5-yl)phényl]-3-pyrrolidinyl}carbonyl)pipérazine ;
le 3-((3*S*)-1-{[4-(1-méthyléthyl)-1-pipérazinyl]carbonyl}-1-pyrrolidinyl)benzonitrile ;
le 4-((3*S*)-3-{[4-(1-méthyléthyl)-1-pipérazinyl]carbonyl}-1-pyrrolidinyl)benzonitrile ;
la 5-((3*S*)-3-{[4-(1-méthyléthyl)-1-pipérazinyl]carbonyl}-1-pyrrolidinyl)-2-(trifluorométhyl)pyrimidine ;
la 1-(1-méthyléthyl)-4-({(3*R*)-1-[4-(3-méthyl-1,2,4-oxadiazole-5-yl)phényl]-3-pyrrolidinyl}carbonyl)pipérazine ;
la 1-cyclobutyl-4-({(3*R*)-1-[4-(3-méthyl-1,2,4-oxadiazole-5-yl)phényl]-3-pyrrolidinyl}carbonyl)pipérazine ;
la 1-(1-méthyléthyl)-4-({(3*S*)-1-[4-(5-phényl-1,3,4-oxadiazole-2-yl)phényl]-3-pyrrolidinyl}carbonyl)pipérazine ;
la 1-({(3*S*)-1-[4-(5-isoxazolyl)phényl]-3-pyrrolidinyl}carbonyl)-4-(1-méthyléthyl)pipérazine ;
la 1-(1-méthyléthyl)-4-({(3S)-1-[4-(1H-pyrrole-1-yl)phényl]-3-pyrrolidinyl}carbonyl)pipérazine ;
la 1-({(3*S*)-1-[4-(1H-imidazole-1-yl)phényl]-3-pyrrolidinyl)-carbonyl)-4-(1-méthyléthyl)pipérazine ;
la 1-(1-méthyléthyl)-4-({(3S)-1-[4-(1,3-oxazole-5-yl)phényl]-3-pyrrolidinyl}carbonyl)pipérazine ;
la 1-[4-((3*S*)-3-{[4-(1-méthyléthyl)-1-pipérazinyl]carbonyl}-1-pyrrolidinyl)phényl]-2-pyrrolidone;
le 4-((3*S*)-3-{[4-(1-méthyléthyl)hexahydro-1*H*-1,4-diazépine-1-yl]carbonyl}-1-pyrrolidinyl)benzonitrile ;
la 1-(1-méthyléthyl)-[4-({(3*S*)-1-[4-(5-méthyl-1,3,4-oxadiazole-2-yl)phényl]-3-pyrrolidinyl}carbonyl)hexahydro-1*H*-1,4-diazépine ;
la 1-({(3*S*)-1-[4-(3-éthyl-1, 2, 4-oxadiazole-5-yl)phényl]-3-pyrrolidinyl}carbonyl)-4-(1-méthyléthyl)hexahydro-1*H*-1,4-diazépine ;
la 1-({(3*S*)-1-[4-(3-éthyl-1,2,4-oxadiazole-5-yl)phényl]-3-pyrrolidinyl}carbonyl)-4-(1-méthyléthyl)pipérazine ;
la 1-[4-((3*S*)-3-{[4-(1-méthyléthyl)-1-pipérazinyl]carbonyl}-1-pyrrolidinyl)phényl]-1-propanone ;
la 1-cyclobutyl-4-({(3*S*)-1-[6-(3-méthyl-1,2,4-oxadiazole-5-yl)-3-pyridinyl]-3-pyrrolidinyl}carbonyl)pipérazine ;
la 1-cyclobutyl-4-({(3*S*)-1-[3-fluoro-4-(3-méthyl-1,2,4-oxadiazole-5-yl)phényl]-3-pyrrolidinyl}carbonyl)pipérazine ;
la 1-(1-méthyléthyl)-4-({(3*S*)-1-[6-(3-méthyl-1,2,4-oxadiazole-5-yl)-3-pyridinyl]-3-pyrrolidinyl}carbonyl)hexahydro-1*H*-1,4-diazépine ;
la 1-(1-méthyléthyl)-4-({(3*S*)-1-[6-(3-méthyl-1,2,4-oxadiazole-5-yl)-3-pyridinyl]-3-pyrrolidinyl}carbonyl)pipérazine ;
la 1-(1-méthyléthyl)-4-({(3S)-1-[4-(4-méthyl-1H-imidazole-1-yl)phényl]-3-pyrrolidinyl}carbonyl)pipérazine ;
la 1-(1-méthyléthyl)-4-({(3S)-1-[4-(2-méthyl-1H-imidazole-1-yl)phényl]-3-pyrrolidinyl}carbonyl)pipérazine ;
le 4-((3*S*)-3-{[(3*S*)-3-méthyl-4-(1-méthyléthyl)-1-pipérazinyl]-carbonyl}-1-pyrrolidinyl)benzonitrile ;
la 1-(cyclopropylméthyl)-4-({(3*S*)-1-[6-(5-méthyl-1,2,4-oxadiazole-3-yl)-3-pyridinyl]-3-pyrrolidinyl}carbonyl)hexahydro-1H-1,4-diazépine ;
la 1-(cyclopropylméthyl)-4-(((3*S*)-1-[6-(5-méthyl-1,2,4-oxadiazole-3-yl)-3-pyridinyl]-3-pyrrolidinyl}carbonyl)pipérazine ; ou
la 1-éthyl-4-({(3S)-1-[4-(3-méthyl-1,2,4-oxadiazole-5-yl)phényl]-3-pyrrolidinyl}carbonyl)pipérazine ; ou un de ses sels ou produits de solvatation pharmaceutiquement acceptables.

11. Composition pharmaceutique qui comprend le composé de formule (I) tel que défini dans l'une quelconque des revendications 1 à 10, ou un de ses sels ou produits de solvatation pharmaceutiquement acceptables, et un support ou excipient pharmaceutiquement acceptable.

12. Composé suivant l'une quelconque des revendications 1 à 10, destiné à être utilisé en thérapie.

13. Composé suivant l'une quelconque des revendications 1 à 10, destiné à être utilisé dans le traitement de maladies neurologiques.

14. Utilisation d'un composé suivant l'une quelconque des revendications 1 à 10, dans la production d'un médicament destiné au traitement de maladies neurologiques.

15. Procédé pour la préparation d'un composé de formule (I) ou d'un de ses sels ou produits de solvatation pharmaceutiquement acceptables, procédé qui comprend :
(a) la réaction de formule (II) 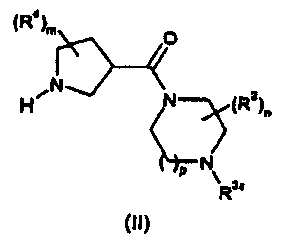 ou d'un de ses dérivés facultativement activés ou protégés, formule dans laquelle R², R⁴, m, n et p répondent aux définitions de la revendication 1 et R^{3a} est tel que défini pour R³ dans la revendication 1 ou représente un groupe pouvant être converti en R³ avec un composé de formule R¹-L¹, dans laquelle R¹ est tel que défini dans la revendication 1 et L¹ représente un groupe partant convenable tel qu'un atome d'halogène ou un groupe triflate, avec ensuite une réaction de suppression de protection telle que nécessaire ; ou
(b) la réaction d'un composé de formule (III) 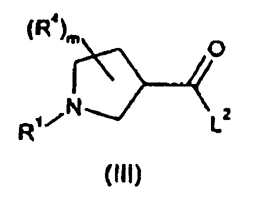 dans laquelle R¹, R⁴ et m sont tels que définis dans la revendication 1 et L² représente un groupe OH ou un groupe partant convenable tel qu'un atome d'halogène, avec un composé de formule (IV) 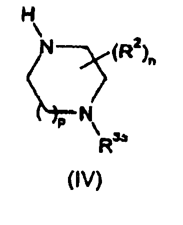 dans laquelle R², n et p sont tels que définis dans la revendication 1, R^{3a} est tel que défini pour R³ dans la revendication 1 ou représente un groupe pouvant être converti en R³ ; ou
(c) la suppression de protection d'un composé de formule (I) ou la conversion de groupes qui sont protégés ; ou
(d) l'interconversion d'un composé de formule (I) en un autre.

16. Procédé pour la préparation d'un énantiomère unique d'un composé de formule (I) ou d'un de ses sels ou produits de solvatation pharmaceutiquement acceptables, procédé qui comprend la réaction d'un composé de formule (XV) ou (XV)^{a} dans laquelle R⁴ et m sont tels que définis dans la revendication 1, où L⁷ représente un groupe partant et où P³ représente un groupe protecteur ou un groupe R¹, comme défini dans la revendication 1 : 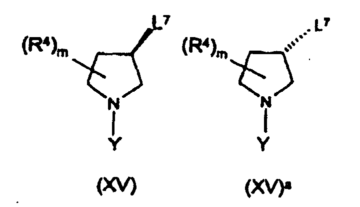 avec un agent nucléophile carboné qui peut être converti en un acide carboxylique, pour produire un composé de formule (XVI) ou (XVI)^{a}, dans laquelle cNu représente un agent nucléophile carboné qui peut être converti en un acide carboxylique, le composé de formule (XVI) ou (XVI)^{a} étant utilisé dans la préparation de l'énantiomère unique du composé de formule (I).
